# EUROPEAN PATENT APPLICATION

(11) **EP 1 726 656 A2**
(43) Date of publication of application: **29.11.2006**
(21) Application number: 06011788.4
(22) Date of filing: 21.01.2000
(51) Int. Cl.: C12N 15/57, C12N 9/64, C07K 16/40, C12N 1/21, C12N 5/10

(54) **Metalloproteinase-disintegrin family members: SVPH DNAs and polypeptides**

(30) Priority: 21.01.1999 US 116670 P; 14.06.1999 US 138682 P; 27.09.1999 US 155798 P
(62) Divisional of application: 00903352.3
(71) Applicant: IMMUNEX CORPORATION, Seattle Washington 98101 (US)
(72) Inventor: The designation of the inventor has not yet been filed
(74) Representative: Cornish, Kristina Victoria Joy

(57) **Abstract**

The invention is directed to purified and isolated novel SVPH polypeptides, the nucleic acids encoding such polypeptides, processes for production of recombinant forms of such polypeptides, and the uses of the above.

SEQUENCE LISTING

<110> Immunex Corporation

<120> NEW METALLOPROTEINASE-DISINTEGRIN FAMILY MEMBERS: SVPH
      DNAS AND POLYPEPTIDES

<130> 03260.0093-00304

<140>
<141>

<150> 60/116,670
<151> 1999-01-21

<150> 60/138,682
<151> 1999-06-14

<150> 60/155,798
<151> 1999-09-27

<160> 33

<170> PatentIn Ver. 2.1

<210> 1
<211> 129
<212> DNA
<213> Homo sapiens

<220>
<223> "n" at various positions throughout the sequence
      may be any nucleotide

<400> 1
atttttgata ccacagtgac caacacggtc acctaaggtg ttcaattctt tgtagcaagt 60
ctcacttgca gtatttgcgc ctgcaccaaa aatcctccta cactgttcan ttgcggtcat 120
gacangctc                                                          129


<210> 2
<211> 469
<212> DNA
<213> Homo sapiens

<400> 2
tttttgagta agaataggtc atgttttagt aaaacttcca aaagaacaaa acagattctt 60
caacccagga ggacatgtga gtcacaatac cctttaatcc acaggttggc tccttggttt 120
ctggaacttt ctgcctcctg taaacgatgt gcgggtggta ccctccctca accagtggat 180
gcttcttcac gggttcaatg aaaaagtctc catgtggtag ttggaaaaat ccagtcagtc 240
catggcaggc actgagggct gccgtcccaa ctctggtgcc ctgctgtaga accgtgccac 300
tgagatggca gaggggggca gaggaagcca tcatcttaac atgggagagg ttcccatatc 360
tcttctccat gatgtagcta ttggaaagaa atccttcatt gaccgtcaag ttaaaaaaca 420
ggtccttctc ctcgtgagaa attctgtagt acacccagtc ctctgagcc             469


<210> 3
<211> 1500

<212> DNA
<213> Homo sapiens

<400> 3
cacgaggatt tatatcttca aagaaaatat aatgatgctc ttgcatggtc gtttggaaaa 60
gtgtgttctc tagaatatgc tggatcagtg agtactttac tagatacaaa tatccttgcc 120
cctgctacct ggtctgctca tgagctgggt catgctgtag gaatgtcaca tgatgaacaa 180
tactgccaat gtaggggtag gcctaattgc atcatgggct caggacgcac tgggtttagc 240
aattgcagtt atatctcttt ttttaaacat atctcttcgg gagcaacatg tctaaataat 300
atcccaggac taggttatgt gcttaagaga tgtggaaaca aaattgtgga ggacaatgag 360
gaatgtgatt gtggttccac agaggagtgt cagaaagatc ggtgttgcca atcaaattgt 420
aagttgcaac caggtgccaa ctgtagcatt ggactttgct gtcatgattg tcggtttcgt 480
ccatctggat acgtgtgtag gcaggaagga aatgaatgtg accttgcaga gtactgcgac 540
gggaattcaa gttcctgccc aaatgacgtt tataagcagg atggaacccc ttgcaagtat 600
gaaggccgtt gtttcaggaa ggggtgcaga tccagatata tgcagtgcca aagcattttt 660
ggacctgatg ccatggaggc tcctagtgag tgctatgatg cagttaactt aataggtgat 720
caatttggta actgtgagat tacaggaatt cgaaatttta aaaagtgtga aagtgcaaat 780
tcaatatgtg gcaggctaca gtgtataaat gttgaaacca tccctgattt gccagagcat 840
acgactataa tttctactca tttacaggca gaaaatctca tgtgctgggg cacaggctat 900
catctatcca tgaaacccat gggaatacct gacctaggta tgataaatga tggcacctcc 960
tgtggagaag gccgggtatg ttttaaaaaa aattgcgtca atagctcagt cctgcagttt 1020
gactgtttgc ctgagaaatg caatacccgg ggtgtttgca acaacagaaa aaactgccac 1080
tgcatgtatg ggtgggcacc tccattctgt gaggaagtgg ggtatggagg aagcattgac 1140
agtgggcctc caggactgct cagaggggcg attccctcgt caatttgggt tgtgtccatc 1200
ataatgtttc gccttatttt attaatcctt tcagtggttt ttgtgttttt ccggcaagtg 1260
ataggaaacc acttaaaacc caaacaggaa aaaatgccac tatccaaagc aaaaactgaa 1320
caggaagaat ctaaaacaaa aactgtacag gaagaatcta aaacaaaaac tggacaggaa 1380
gaatctgaag caaaaactgg acaggaagaa tctaaagcaa aaactggaca ggaagaatct 1440
aaagcaaaca ttgaaagtaa acgacccaaa gcaaagagtg tcaagaaaca aaaaaagtaa 1500

<210> 4
<211> 40
<212> PRT
<213> Homo sapiens

<220>
<223> "Xaa" at various positions throughout the sequence
      may be any amino acid

<400> 4
Met Thr Ala Xaa Glu Gln Cys Arg Arg Ile Phe Gly Ala Gly Ala Asn
 1               5                  10                  15

Thr Ala Ser Glu Thr Cys Tyr Lys Glu Leu Asn Thr Leu Gly Asp Arg
            20                  25                  30

Val Gly His Cys Gly Ile Lys Asn
        35                  40

<210> 5
<211> 123
<212> PRT
<213> Homo sapiens

<400> 5
Glu Asp Trp Val Tyr Tyr Arg Ile Ser His Glu Glu Lys Asp Leu Phe
1               5                   10                  15

Phe Asn Leu Thr Val Asn Glu Gly Phe Leu Ser Asn Ser Tyr Ile Met
            20                  25                  30

Glu Lys Arg Tyr Gly Asn Leu Ser His Val Lys Met Met Ala Ser Ser
        35                  40                  45

Ala Pro Leu Cys His Leu Ser Gly Thr Val Leu Gln Gln Gly Thr Arg
    50                  55                  60

Val Gly Thr Ala Ala Leu Ser Ala Cys His Gly Leu Thr Gly Phe Phe
65                  70                  75                  80

Gln Leu Pro His Gly Asp Phe Phe Ile Glu Pro Val Lys Lys His Pro
                85                  90                  95

Leu Val Glu Gly Gly Tyr His Pro His Ile Val Tyr Arg Arg Gln Lys
            100                 105                 110

Val Pro Glu Thr Lys Glu Pro Thr Cys Gly Leu
        115                 120


<210> 6
<211> 499
<212> PRT
<213> Homo sapiens

<400> 6
His Glu Asp Leu Tyr Leu Gln Arg Lys Tyr Asn Asp Ala Leu Ala Trp
1               5                   10                  15

Ser Phe Gly Lys Val Cys Ser Leu Glu Tyr Ala Gly Ser Val Ser Thr
            20                  25                  30

Leu Leu Asp Thr Asn Ile Leu Ala Pro Ala Thr Trp Ser Ala His Glu
        35                  40                  45

Leu Gly His Ala Val Gly Met Ser His Asp Glu Gln Tyr Cys Gln Cys
    50                  55                  60

Arg Gly Arg Pro Asn Cys Ile Met Gly Ser Gly Arg Thr Gly Phe Ser
65                  70                  75                  80

Asn Cys Ser Tyr Ile Ser Phe Phe Lys His Ile Ser Ser Gly Ala Thr
                85                  90                  95

Cys Leu Asn Asn Ile Pro Gly Leu Gly Tyr Val Leu Lys Arg Cys Gly
            100                 105                 110

Asn Lys Ile Val Glu Asp Asn Glu Glu Cys Asp Cys Gly Ser Thr Glu
        115                 120                 125

Glu Cys Gln Lys Asp Arg Cys Cys Gln Ser Asn Cys Lys Leu Gln Pro
    130                 135                 140

Gly Ala Asn Cys Ser Ile Gly Leu Cys Cys His Asp Cys Arg Phe Arg
145                 150                 155                 160

Pro Ser Gly Tyr Val Cys Arg Gln Glu Gly Asn Glu Cys Asp Leu Ala
                165                 170                 175

Glu Tyr Cys Asp Gly Asn Ser Ser Ser Cys Pro Asn Asp Val Tyr Lys
            180                 185                 190

Gln Asp Gly Thr Pro Cys Lys Tyr Glu Gly Arg Cys Phe Arg Lys Gly
        195                 200                 205

Cys Arg Ser Arg Tyr Met Gln Cys Gln Ser Ile Phe Gly Pro Asp Ala
    210                 215                 220

Met Glu Ala Pro Ser Glu Cys Tyr Asp Ala Val Asn Leu Ile Gly Asp
225                 230                 235                 240

Gln Phe Gly Asn Cys Glu Ile Thr Gly Ile Arg Asn Phe Lys Lys Cys
                245                 250                 255

Glu Ser Ala Asn Ser Ile Cys Gly Arg Leu Gln Cys Ile Asn Val Glu
            260                 265                 270

Thr Ile Pro Asp Leu Pro Glu His Thr Thr Ile Ile Ser Thr His Leu
        275                 280                 285

Gln Ala Glu Asn Leu Met Cys Trp Gly Thr Gly Tyr His Leu Ser Met
    290                 295                 300

Lys Pro Met Gly Ile Pro Asp Leu Gly Met Ile Asn Asp Gly Thr Ser
305                 310                 315                 320

Cys Gly Glu Gly Arg Val Cys Phe Lys Lys Asn Cys Val Asn Ser Ser
                325                 330                 335

Val Leu Gln Phe Asp Cys Leu Pro Glu Lys Cys Asn Thr Arg Gly Val
            340                 345                 350

Cys Asn Asn Arg Lys Asn Cys His Cys Met Tyr Gly Trp Ala Pro Pro
        355                 360                 365

Phe Cys Glu Glu Val Gly Tyr Gly Gly Ser Ile Asp Ser Gly Pro Pro
    370                 375                 380

Gly Leu Leu Arg Gly Ala Ile Pro Ser Ser Ile Trp Val Val Ser Ile
385                 390                 395                 400

Ile Met Phe Arg Leu Ile Leu Leu Ile Leu Ser Val Val Phe Val Phe
                405                 410                 415

Phe Arg Gln Val Ile Gly Asn His Leu Lys Pro Lys Gln Glu Lys Met
            420                 425                 430

Pro Leu Ser Lys Ala Lys Thr Glu Gln Glu Glu Ser Lys Thr Lys Thr
        435                 440                 445

Val Gln Glu Glu Ser Lys Thr Lys Thr Gly Gln Glu Glu Ser Glu Ala
    450                 455                 460

Lys Thr Gly Gln Glu Glu Ser Lys Ala Lys Thr Gly Gln Glu Glu Ser
465                 470                 475                 480

Lys Ala Asn Ile Glu Ser Lys Arg Pro Lys Ala Lys Ser Val Lys Lys
                485                 490                 495

Gln Lys Lys

<210> 7
<211> 2301
<212> DNA
<213> Homo sapiens

<400> 7
atgaagatgt tactcctgct gcattgcctt ggggtgtttc tgtcctgttc tggacacatc 60
caggatgagc acccccaata tcacagccct ccggatgtgg tgattcctgt gaggataact 120
ggcaccacca gaggcatgac acctccaggc tggctctcct atatcctgcc ctttggaggc 180
cagaaacaca ttatccacat aaaggtcaag aagcttttgt tttccaaaca cctccctgtg 240
ttcacctaca cagaccaggg tgctatcctt gaggaccagc catttgtcca gaataactgc 300
tactatcatg gttatgtgga aggggaccca gaatccctgg tttccctcag tacctgtttt 360
gggggtttcc aaggaatatt acagataaat gactttgctt atgaaatcaa gcccctagca 420
ttttctacca cgtttgaaca tctggtatac aagatggaca gtgaggagaa acaattttca 480
accatgagat ccggatttat gcaaaatgaa ataacatgcc gaatggaatt tgaagaaatt 540
gataattcca ctcagaagca aagttcttat gtgggctggt ggatccattt taggattgtt 600
gaaattgtag tcgtcattga taattatctg tacattcgtt atgaaaggaa cgactcaaag 660
ttgctggagg atctatatgt tattgttaat atagtggatt ccattttgga tgtcattggt 720
gttaaggtgt tattatttgg tttggagatc tggaccaata aaaacctcat tgtagtagat 780
gatgtaagga aatctgtgca cctgtattgc aagtggaagt cggagaacat tacgccccgg 840
atgcaacatg acacctcaca tcttttcaca actctaggat taagagggtt aagtggcata 900
ggagctttta gaggaatgtg tacaccacac cgtagttgtg caattgttac tttcatgaac 960
aaaactttgg gcactttttc aattgcagtg gctcatcatc taggtcataa tttgggcatg 1020
aaccatgatg aggatacatg tcgttgttca caacctagat gcataatgca tgaaggcaac 1080
ccaccaataa ctaaatttag caattgtagt tatggtgatt tttgggaata tactgtagag 1140
aggacaaagt gtttgcttga aacagtacac acaaaggaca tctttaatgt gaagcgctgt 1200
gggaatggtg ttgttgaaga aggagaagag tgtgactgtg gacctttaaa gcattgtgca 1260
aaagatccct gctgtctgtc aaattgcact ctgactgatg gttctacttg tgcttttggg 1320
ctttgttgca aagactgcaa gttcctacca tcagggaaag tgtgtagaaa ggaggtcaat 1380
gaatgtgatc ttccagagtg gtgcaatggt acttcccata agtgcccaga tgacttttat 1440
gtggaagatg gaattccctg taaggagagg ggctactgct atgaaaagag ctgtcatgac 1500
cgcaatgaac agtgtaggag gatttttggt gcaggcgcaa atactgcaag tgagacttgc 1560
tacaaagaat tgaacacctt aggtgaccgt gttggtcact gtggtatcaa aaatgctaca 1620
tatataaagt gtaatatctc agatgtccag tgtggaagaa ttcagtgtga gaatgtgaca 1680
gaaattccca atatgagtga tcatactact gtgcattggg ctcgcttcaa tgacataatg 1740
tgctggagta ctgattacca tttggggatg aagggacctg atattggtga agtgaaagat 1800
ggaacagagt gtgggataga tcatatatgc atccacaggc actgtgtcca tataaccatc 1860
ttgaatagta attgctcacc tgcattttgt aacaagaggg gcatctgcaa caataaacat 1920
cactgccatt gcaattatct gtgggacccc cccaactgcc tgataaaagg ctatggaggt 1980
agtgttgaca gtggcccacc ccctaagaga aagaagaaaa agaagttctg ttatctgtgt 2040
atattgttgc ttattgtttt gtttatttta ttatgttgtc tttatcgact ttgtaaaaaa 2100
agtaaaccaa taaaaaagca gcaagatgtt caaactccat ctgcaaaaga agaggaaaaa 2160
attcagcgtc gacctcatga gttacctccc cagagtcaac cttgggtgat gccttcccag 2220
agtcaacctc ctgtgacacc ctcccagagg caacctcagt tgatgccttc ccagagtcaa 2280
cctcctgtga cgccctccta g 2301

<210> 8
<211> 2364
<212> DNA
<213> Homo sapiens

<400> 8
atgaagatgt tactcctgct gcattgcctt ggggtgtttc tgtcctgttc tggacacatc 60
caggatgagc acccccaata tcacagccct ccggatgtgg tgattcctgt gaggataact 120
ggcaccacca gaggcatgac acctccaggc tggctctcct atatcctgcc ctttggaggc 180
cagaaacaca ttatccacat aaaggtcaag aagcttttgt tttccaaaca cctccctgtg 240
ttcacctaca cagaccaggg tgctatcctt gaggaccagc catttgtcca gaataactgc 300
tactatcatg gttatgtgga aggggaccca gaatccctgg tttccctcag tacctgtttt 360
gggggttttc aaggaatatt acagataaat gactttgctt atgaaatcaa gcccctagca 420
ttttctacca cgtttgaaca tctggtatac aagatggaca gtgaggagaa acaattttca 480
accatgagat ccggatttat gcaaaatgaa ataacatgcc gaatggaatt tgaagaaatt 540
gataattcca ctcagaagca aagttcttat gtgggctggt ggatccattt taggattgtt 600
gaaattgtag tcgtcattga taattatctg tacattcgtt atgaaaggaa cgactcaaag 660
ttgctggagg atctatatgt tattgttaat atagtggatt ccatttggga tgtcattggt 720
gttaaggtgt tattatttgg tttggagatc tggaccaata aaaacctcat tgtagtagat 780
gatgtaagga aatctgtgca cctgtattgc aagtggaagt cggagaacat tacgccccgg 840
atgcaacatg acacctcaca tcttttcaca actctaggat taagagggtt aagtggcata 900
ggagctttta gaggaatgtg tacaccacac cgtagttgtg caattgttac tttcatgaac 960
aaaactttgg gcactttttc aattgcagtg gctcatcatc taggtcataa tttgggcatg 1020
aaccatgatg aggatacatg tcgttgttca caacctagat gcataatgca tgaaggcaac 1080
ccaccaataa ctaaatttag caattgtagt tatggtgatt tttgggaata tactgtagag 1140
aggacaaagt gtttgcttga aacagtacac acaaaggaca tctttaatgt gaagcgctgt 1200
gggaatggtg ttgttgaaga aggagaagag tgtgactgtg gacctttaaa gcattgtgca 1260
aaagatccct gctgtctgtc aaattgcact ctgactgatg gttctacttg tgcttttggg 1320
ctttgttgca aagactgcaa gttcctacca tcagggaaag tgtgtagaaa ggaggtcaat 1380
gaatgtgatc ttccagagtg gtgcaatggt acttcccata agtgcccaga tgacttttat 1440
gtggaagatg gaattccctg taaggagagg ggctactgct atgaaaagag ctgtcatgac 1500
cgcaatgaac agtgtaggag gatttttggt gcaggcgcaa atactgcaag tgagacttgc 1560
tacaaagaat tgaacacctt aggtgaccgt gttggtcact gtggtatcaa aaatgctaca 1620
tatataaagt gtaatatctc agatgtccag tgtggaagaa ttcagtgtga gaatgtgaca 1680
gaaattccca atatgagtga tcatactact gtgcattggg ctcgcttcaa tgacataatg 1740
tgctggagta ctgattacca tttggggatg aagggacctg atattggtga agtgaaagat 1800
ggaacagagt gtgggataga tcatatatgc atccacaggc actgtgtcca tataaccatc 1860
ttgaatagta attgctcacc tgcattttgt aacaagaggg gcatctgcaa caataaacat 1920
cactgccatt gcaattatct gtgggacccc cccaactgcc tgataaaagg ctatggaggt 1980
agtgttgaca gtggtccacc ccctaagaga aagaagaaaa agaagttctg ttatctgtgt 2040
atattgttgc ttattgtttt gtttatttta ttatgttgtc tttatcgact ttgtaaaaaa 2100
agtaaaccaa taaaaaagca gcaagatgtt caaactccat ctgcaaaaga agaggaaaaa 2160
attcagcgtc gacctcatga gttacctccc cagagtcaac cttgggtgat gccttcccag 2220
agtcaacctc ctgtgacgcc ttcccagagt catcctcagg tgatgccttc ccagagtcaa 2280
cctcctcaaa atttattcct gttcagcttc tcaatcagtg actgtgtgct aaattttagg 2340
ctactgtatc ttcaggccac ctga 2364

<210> 9
<211> 2463
<212> DNA
<213> Homo sapiens

<400> 9
atgaagatgt tactcctgct gcattgcctt ggggtgtttc tgtcctgttc tggacacatc 60
caggatgagc acccccaata tcacagccct ccggatgtgg tgattcctgt gaggataact 120
ggcaccacca gaggcatgac acctccaggc tggctctcct atatcctgcc ctttggaggc 180
cagaaacaca ttatccacat aaaggtcaag aagcttttgt tttccaaaca cctccctgtg 240

ttcacctaca cagaccaggg tgctatcctt gaggaccagc catttgtcca gaataactgc 300
tactatcatg gttatgtgga aggggaccca gaatccctgg tttccctcag tacctgtttt 360
gggggttttc aaggaatatt acagataaat gactttgctt atgaaatcaa gcccctagca 420
ttttctacca cgtttgaaca tctggtatac aagatggaca gtgaggagaa acaattttca 480
accatgagat ccggatttat gcaaaatgaa ataacatgcc gaatggaatt tgaagaaatt 540
gataattcca ctcagaagca aagttcttat gtgggctggt ggatccattt taggattgtt 600
gaaattgtag tcgtcattga taattatctg tacattcgtt atgaaaggaa cgactcaaag 660
ttgctggagg atctatatgt tattgttaat atagtggatt ccattttgga tgtcattggt 720
gttaaggtgt tattatttgg tttggagatc tggaccaata aaaacctcat tgtagtagat 780
gatgtaagga aatctgtgca cctgtattgc aagtggaagt cggagaacat tacgccccgg 840
atgcaacatg acacctcaca tcttttcaca actctaggat taagagggtt aagtggcata 900
ggagctttta gaggaatgtg tacaccacac cgtagttgtg caattgttac tttcatgaac 960
aaaactttgg gcactttttc aattgcagtg gctcatcatc taggtcataa tttgggcatg 1020
aaccatgatg aggatacatg tcgttgttca caacctagat gcataatgca tgaaggcaac 1080
ccaccaataa ctaaatttag caattgtagt tatggtgatt tttgggaata tactgtagag 1140
aggacaaagt gtttgcttga aacagtacac acaaaggaca tctttaatgt gaagcgctgt 1200
gggaatggtg ttgttgaaga aggagaagag tgtgactgtg gacctttaaa gcattgtgca 1260
aaagatccct gctgtctgtc aaattgcact ctgactgatg gttctacttg tgcttttggg 1320
ctttgttgca aagactgcaa gttcctacca tcagggaaag tgtgtagaaa ggaggtcaat 1380
gaatgtgatc ttccagagtg gtgcaatggt acttcccata agtgcccaga tgacttttat 1440
gtggaagatg gaattccctg taaggagagg ggctactgct atgaaaagag ctgtcatgac 1500
cgcaatgaac agtgtaggag gatttttggt gcaggcgcaa atactgcaag tgagacttgc 1560
tacaaagaat tgaacacctt aggtgaccgt gttggtcact gtggtatcaa aaatgctaca 1620
tatataaagt gtaatatctc agatgtccag tgtggaagaa ttcagtgtga gaatgtgaca 1680
gaaattccca atatgagtga tcatactact gtgcattggg ctcgcttcaa tgacataatg 1740
tgctggagta ctgattacca tttggggatg aagggacctg atattggtga agtgaaagat 1800
ggaacagagt gtgggataga tcatatatgc atccacaggc actgtgtcca tataaccatc 1860
ttgaatagta attgctcacc tgcattttgt aacaagaggg gcatctgcaa caataaacat 1920
cactgccatt gcaattatct gtgggaccct cccaactgcc tgataaaagg ctatggaggt 1980
agtgttgaca gtggcccacc ccctaagaga aagaagaaaa agaagttctg ttatctgtgt 2040
atattgttgc ttattgtttt gtttatttta ttatgttgtc tttatcgact ttgtaaaaaa 2100
agtaaaccaa taaaaaagca gcaagatgtt caaactccat ctgcaaaaga agaggaaaaa 2160
attcagcgtc gacctcatga gttacctccc cagagtcaac cttgggtgat gccttcccag 2220
agtcaacctc ctgtgacgcc ttcccagagt catcctcggg tgatgccttc tcagagtcaa 2280
cctcctgtga tgccttccca gagtcatcct cagttgacgc cttcccagag tcaacctcct 2340
gtgatgcctt cccagagtca tcctcagttg acgccttccc agagtcaacc tcctgtgaca 2400
ccctcccaga ggcaacctca gttgatgcct tcccagagtc aacctcctgt gacgccctcc 2460
tag 2463

<210> 10
<211> 2373
<212> DNA
<213> Homo sapiens

<400> 10
atgaggtcag tgcagatctt cctctcccaa tgccgtttgc tccttctact agttccgaca 60
atgctcctta agtctcttgg cgaagatgta atttttcacc ctgaagggga gtttgactcg 120
tatgaagtca ccattcctga gaagctgagc ttccggggag aggtgcaggg tgtggtcagt 180
cccgtgtcct acctactgca gttaaaaggc aagaagcacg tcctccattt gtggcccaag 240
agacttctgt tgccccgaca tctgcgcgtt ttctccttca cagaacatgg ggaactgctg 300
gaggatcatc cttacatacc aaaggactgc aactacatgg gctccgtgaa agagtctctg 360
gactctaaag ctactataag cacatgcatg gggggtctcc gaggtgtatt taacattgat 420
gccaaacatt accaaattga gcccctcaag gcctctccca gttttgaaca tgtcgtctat 480
ctcctgaaga aagagcagtt tgggaatcag gtttgtggct taagtgatga tgaaatagaa 540
tggcagatgg ccccttatga gaataaggcg aggctaaggg actttcctgg atcctataaa 600
cacccaaagt acttggaatt gatcctactc tttgatcaaa gtaggtatag gtttgtgaac 660
aacaatcttt ctcaagtcat acatgatgcc attcttttga ctgggattat ggacacctac 720

tttcaagatg ttcgtatgag gatacactta aaggctcttg aagtatggac agattttaac 780
aaaatacgcg ttggatatcc agagttagct gaagttttag gcagatttgt aatatataaa 840
aaaagtgtat taaatgctcg cctgtcatca gattgggcac atttatatct tcaaagaaaa 900
tataatgatg ctcttgcatg gtcgtttgga aaagtgtgtt ctctagaata tgctggatca 960
gtgagtactt tactagatac aaatatcctt gcccctgcta cctggtctgc tcatgagctg 1020
ggtcatgctg taggaatgtc acatgatgaa caatactgcc aatgtagggg taggcctaat 1080
tgcatcatgg gctcaggacg cactgggttt agcaattgca gttatatctc tttttttaaa 1140
catatctctt cgggagcaac atgtctaaat aatatcccag gactaggtta tgtgcttaag 1200
agatgtggaa acaaaattgt ggaggacaat gaggaatgtg attgtggttc cacagaggag 1260
tgtcagaaag atcggtgttg ccaatcaaat tgtaagttgc aaccaggtgc caactgtagc 1320
attggacttt gctgtcatga ttgtcggttt cgtccatctg gatacgtgtg taggcaggaa 1380
ggaaatgaat gtgaccttgc agagtactgc gacgggaatt caagttcctg cccaaatgac 1440
gtttataagc aggatggaac cccttgcaag tatgaaggcc gttgtttcag gaaggggtgc 1500
agatccagat atatgcagtg ccaaagcatt tttggacctg atgccatgga ggctcctagt 1560
gagtgctatg atgcagttaa cttaataggt gatcaatttg gtaactgtga gattacagga 1620
attcgaaatt ttaaaaagtg tgaaagtgca aattcaatat gtggcaggct acagtgtata 1680
aatgttgaaa ccatccctga tttgccagag catacgacta taatttctac tcatttacag 1740
gcagaaaatc tcatgtgctg gggcacaggc tatcatctat ccatgaaacc catgggaata 1800
cctgacctag gtatgataaa tgatggcacc tcctgtggag aaggccgggt atgttttaaa 1860
aaaaattgcg tcaatagctc agtcctgcag tttgactgtt tgcctgagaa atgcaatacc 1920
cggggtgttt gcaacaacag aaaaaactgc cactgcatgt atgggtgggc acctccattc 1980
tgtgaggaag tggggtatgg aggaagcatt gacagtgggc ctccaggact gctcagaggg 2040
gcgattccct cgtcaatttg ggttgtgtcc atcataatgt ttcgccttat tttattaatc 2100
ctttcagtgg tttttgtgtt tttccggcaa gtgataggaa accacttaaa acccaaacag 2160
gaaaaaatgc cactatccaa agcaaaaact gaacaggaag aatctaaaac aaaaactgta 2220
caggaagaat ctaaaacaaa aactggacag gaagaatctg aagcaaaaac tggacaggaa 2280
gaatctaaag caaaaactgg acaggaagaa tctaaagcaa acattgaaag taaacgaccc 2340
aaagcaaaga gtgtcaagaa acaaaaaaag taa 2373

<210> 11
<211> 2346
<212> DNA
<213> Homo sapiens

<400> 11
atgaggtcag tgcagatctt cctctcccaa tgccgtttgc tccttctact agttccgaca 60
atgctcctta agtctcttgg cgaagatgta attttcaccc ctgaagggga gtttgactcg 120
tatgaagtca ccattcctga gaagctgagc ttccggggag aggtgcaggg tgtggtcagt 180
cccgtgtcct acctactgca gttaaaaggc aagaagcacg tcctccattt gtggcccaag 240
agacttctgt tgccccgaca tctgcgcgtt ttctccttca cagaacatgg ggaactgctg 300
gaggatcatc cttacatacc aaaggactgc aactacatgg gctccgtgaa agagtctctg 360
gactctaaag ctactataag cacatgcatg gggggtctcc gaggtgtatt taacattgat 420
gccaaacatt accaaattga gcccctcaag gcctctccca gttttgaaca tgtcgtctat 480
ctcctgaaga aagagcagtt tgggaatcag gtttgtggct taagtgatga tgaaatagaa 540
tggcagatgg ccccttatga gaataaggcg aggctaaggg actttcctgg atcctataaa 600
cacccaaagt acttggaatt gatcctactc tttgatcaaa gtaggtatag gtttgtgaac 660
aacaatcttt ctcaagtcat acatgatgcc attcttttga ctgggattat ggacacctac 720
tttcaagatg ttcgtatgag gatacactta aaggctcttg aagtatggac agattttaac 780
aaaatacgcg ttggatatcc agagttagct gaagttttag gcagatttgt aatatataaa 840
aaaagtgtat taaatgctcg cctgtcatca gattgggcac atttatatct tcaaagaaaa 900
tataatgatg ctcttgcatg gtcgtttgga aaagtgtgtt ctctagaata tgctggatca 960
gtgagtactt tactagatac aaatatcctt gcccctgcta cctggcctgc tcatgagctg 1020
ggtcatgctg taggaatgtc acatgatgaa caatactgcc aatgtagggg taggcttaat 1080
tgcatcatgg gctcaggacg cactgggttt agcaattgca gttatatctc tttttttaaa 1140
catatctctt cgggagcaac atgtctaaat aatatcccag gactaggtta tgtgcttaag 1200
agatgtggaa acaaaattgt ggaggacaat gaggaatgtg actgtggttc cacagaggag 1260
tgtcagaaag atcggtgttg ccaatcaaat tgtaagttgc aaccaggtgc caactgtagc 1320

attggacttt gctgtcatga ttgtcggttt cgtccatctg gatacgtgtg taggcaggaa 1380
ggaaatgaat gtgaccttgc agagtactgc gacgggaatt caagttcctg cccaaatgac 1440
gtttataagc aggatggaac cccttgcaag tatgaaggcc gttgtttcag gaaggggtgc 1500
agatccagat atatgcagtg ccaaagcatt tttggacctg atgccatgga ggctcctagt 1560
gagtgctatg atgcagttaa cttaataggt gatcaatttg gtaactgtga gattacagga 1620
attcgaaatt ttaaaaagtg tgaaagtgca aattcaatat gtggcaggct acagtgtata 1680
aatgttgaaa ccatccctga tttgccagag catacgacta taatttctac tcatttacag 1740
gcagaaaatc tcatgtgctg gggcacaggc tatcatctat ccatgaaacc catgggaata 1800
cctgacctag gtatgataaa tgatggcacc tcctgtggag aaggccgggt atgttttaaa 1860
aaaaattgcg tcaatagctc agtcctgcag tttgactgtt tgcctgagaa atgcaatacc 1920
cggggtgttt gcaacaacag aaaaaactgc cactgcatgt atgggtgggc acctccattc 1980
tgtgaggaag tggggtatgg aggaagcatt gacagtgggc ctccaggact gctcagaggg 2040
gcgattccct cgtcaatttg ggttgtgtcc atcataatgt ttcgccttat tttattaatc 2100
ctttcagtgg tttttgtgtt tttccggcaa gtgataggaa accacttaaa acccaaacag 2160
gaaaaaatgc cactatccaa agcaaaaact gaacaggaag aatctaaaac aaaaactgta 2220
caggaagaat ctaaaacaaa aactggacag gaagaatctg aagcaaaaac tggacaggaa 2280
gaatctaaag caaacattga aagtaaacga cccaaagcaa agagtgtcaa gaaacaaaaa 2340
aagtaa 2346

<210> 12
<211> 766
<212> PRT
<213> Homo sapiens

<400> 12
Met Lys Met Leu Leu Leu Leu His Cys Leu Gly Val Phe Leu Ser Cys
1               5                   10                  15

Ser Gly His Ile Gln Asp Glu His Pro Gln Tyr His Ser Pro Pro Asp
            20                  25                  30

Val Val Ile Pro Val Arg Ile Thr Gly Thr Thr Arg Gly Met Thr Pro
        35                  40                  45

Pro Gly Trp Leu Ser Tyr Ile Leu Pro Phe Gly Gly Gln Lys His Ile
    50                  55                  60

Ile His Ile Lys Val Lys Lys Leu Leu Phe Ser Lys His Leu Pro Val
65                  70                  75                  80

Phe Thr Tyr Thr Asp Gln Gly Ala Ile Leu Glu Asp Gln Pro Phe Val
                85                  90                  95

Gln Asn Asn Cys Tyr Tyr His Gly Tyr Val Glu Gly Asp Pro Glu Ser
            100                 105                 110

Leu Val Ser Leu Ser Thr Cys Phe Gly Gly Phe Gln Gly Ile Leu Gln
        115                 120                 125

Ile Asn Asp Phe Ala Tyr Glu Ile Lys Pro Leu Ala Phe Ser Thr Thr
    130                 135                 140

Phe Glu His Leu Val Tyr Lys Met Asp Ser Glu Glu Lys Gln Phe Ser
145                 150                 155                 160

Thr Met Arg Ser Gly Phe Met Gln Asn Glu Ile Thr Cys Arg Met Glu
                165                 170                 175

Phe Glu Glu Ile Asp Asn Ser Thr Gln Lys Gln Ser Ser Tyr Val Gly
            180                 185                 190

Trp Trp Ile His Phe Arg Ile Val Glu Ile Val Val Val Ile Asp Asn
        195                 200                 205

Tyr Leu Tyr Ile Arg Tyr Glu Arg Asn Asp Ser Lys Leu Leu Glu Asp
    210                 215                 220

Leu Tyr Val Ile Val Asn Ile Val Asp Ser Ile Leu Asp Val Ile Gly
225                 230                 235                 240

Val Lys Val Leu Leu Phe Gly Leu Glu Ile Trp Thr Asn Lys Asn Leu
                245                 250                 255

Ile Val Val Asp Asp Val Arg Lys Ser Val His Leu Tyr Cys Lys Trp
            260                 265                 270

Lys Ser Glu Asn Ile Thr Pro Arg Met Gln His Asp Thr Ser His Leu
        275                 280                 285

Phe Thr Thr Leu Gly Leu Arg Gly Leu Ser Gly Ile Gly Ala Phe Arg
    290                 295                 300

Gly Met Cys Thr Pro His Arg Ser Cys Ala Ile Val Thr Phe Met Asn
305                 310                 315                 320

Lys Thr Leu Gly Thr Phe Ser Ile Ala Val Ala His His Leu Gly His
                325                 330                 335

Asn Leu Gly Met Asn His Asp Glu Asp Thr Cys Arg Cys Ser Gln Pro
            340                 345                 350

Arg Cys Ile Met His Glu Gly Asn Pro Pro Ile Thr Lys Phe Ser Asn
        355                 360                 365

Cys Ser Tyr Gly Asp Phe Trp Glu Tyr Thr Val Glu Arg Thr Lys Cys
    370                 375                 380

Leu Leu Glu Thr Val His Thr Lys Asp Ile Phe Asn Val Lys Arg Cys
385                 390                 395                 400

Gly Asn Gly Val Val Glu Glu Gly Glu Glu Cys Asp Cys Gly Pro Leu
                405                 410                 415

Lys His Cys Ala Lys Asp Pro Cys Cys Leu Ser Asn Cys Thr Leu Thr
            420                 425                 430

Asp Gly Ser Thr Cys Ala Phe Gly Leu Cys Cys Lys Asp Cys Lys Phe
        435                 440                 445

Leu Pro Ser Gly Lys Val Cys Arg Lys Glu Val Asn Glu Cys Asp Leu
    450                 455                 460

Pro Glu Trp Cys Asn Gly Thr Ser His Lys Cys Pro Asp Asp Phe Tyr
465                 470                 475                 480

Val Glu Asp Gly Ile Pro Cys Lys Glu Arg Gly Tyr Cys Tyr Glu Lys
                485                 490                 495

Ser Cys His Asp Arg Asn Glu Gln Cys Arg Arg Ile Phe Gly Ala Gly
            500                 505                 510

Ala Asn Thr Ala Ser Glu Thr Cys Tyr Lys Glu Leu Asn Thr Leu Gly
        515                 520                 525

Asp Arg Val Gly His Cys Gly Ile Lys Asn Ala Thr Tyr Ile Lys Cys
    530                 535                 540

Asn Ile Ser Asp Val Gln Cys Gly Arg Ile Gln Cys Glu Asn Val Thr
545                 550                 555                 560

Glu Ile Pro Asn Met Ser Asp His Thr Thr Val His Trp Ala Arg Phe
                565                 570                 575

Asn Asp Ile Met Cys Trp Ser Thr Asp Tyr His Leu Gly Met Lys Gly
            580                 585                 590

Pro Asp Ile Gly Glu Val Lys Asp Gly Thr Glu Cys Gly Ile Asp His
        595                 600                 605

Ile Cys Ile His Arg His Cys Val His Ile Thr Ile Leu Asn Ser Asn
    610                 615                 620

Cys Ser Pro Ala Phe Cys Asn Lys Arg Gly Ile Cys Asn Asn Lys His
625                 630                 635                 640

His Cys His Cys Asn Tyr Leu Trp Asp Pro Pro Asn Cys Leu Ile Lys
                645                 650                 655

Gly Tyr Gly Gly Ser Val Asp Ser Gly Pro Pro Pro Lys Arg Lys Lys
            660                 665                 670

Lys Lys Lys Phe Cys Tyr Leu Cys Ile Leu Leu Leu Ile Val Leu Phe
        675                 680                 685

Ile Leu Leu Cys Cys Leu Tyr Arg Leu Cys Lys Lys Ser Lys Pro Ile
    690                 695                 700

Lys Lys Gln Gln Asp Val Gln Thr Pro Ser Ala Lys Glu Glu Glu Lys
705                 710                 715                 720

Ile Gln Arg Arg Pro His Glu Leu Pro Pro Gln Ser Gln Pro Trp Val
                725                 730                 735

Met Pro Ser Gln Ser Gln Pro Pro Val Thr Pro Ser Gln Arg Gln Pro
            740                 745                 750

Gln Leu Met Pro Ser Gln Ser Gln Pro Pro Val Thr Pro Ser
        755                 760                 765

<210> 13
<211> 787
<212> PRT

<213> Homo sapiens

<400> 13
Met Lys Met Leu Leu Leu Leu His Cys Leu Gly Val Phe Leu Ser Cys
1               5                   10                  15

Ser Gly His Ile Gln Asp Glu His Pro Gln Tyr His Ser Pro Pro Asp
            20                  25                  30

Val Val Ile Pro Val Arg Ile Thr Gly Thr Thr Arg Gly Met Thr Pro
        35                  40                  45

Pro Gly Trp Leu Ser Tyr Ile Leu Pro Phe Gly Gly Gln Lys His Ile
    50                  55                  60

Ile His Ile Lys Val Lys Lys Leu Leu Phe Ser Lys His Leu Pro Val
65                  70                  75                  80

Phe Thr Tyr Thr Asp Gln Gly Ala Ile Leu Glu Asp Gln Pro Phe Val
                85                  90                  95

Gln Asn Asn Cys Tyr Tyr His Gly Tyr Val Glu Gly Asp Pro Glu Ser
            100                 105                 110

Leu Val Ser Leu Ser Thr Cys Phe Gly Gly Phe Gln Gly Ile Leu Gln
        115                 120                 125

Ile Asn Asp Phe Ala Tyr Glu Ile Lys Pro Leu Ala Phe Ser Thr Thr
    130                 135                 140

Phe Glu His Leu Val Tyr Lys Met Asp Ser Glu Glu Lys Gln Phe Ser
145                 150                 155                 160

Thr Met Arg Ser Gly Phe Met Gln Asn Glu Ile Thr Cys Arg Met Glu
                165                 170                 175

Phe Glu Glu Ile Asp Asn Ser Thr Gln Lys Gln Ser Ser Tyr Val Gly
            180                 185                 190

Trp Trp Ile His Phe Arg Ile Val Glu Ile Val Val Val Ile Asp Asn
        195                 200                 205

Tyr Leu Tyr Ile Arg Tyr Glu Arg Asn Asp Ser Lys Leu Leu Glu Asp
    210                 215                 220

Leu Tyr Val Ile Val Asn Ile Val Asp Ser Ile Leu Asp Val Ile Gly
225                 230                 235                 240

Val Lys Val Leu Leu Phe Gly Leu Glu Ile Trp Thr Asn Lys Asn Leu
                245                 250                 255

Ile Val Val Asp Asp Val Arg Lys Ser Val His Leu Tyr Cys Lys Trp
            260                 265                 270

Lys Ser Glu Asn Ile Thr Pro Arg Met Gln His Asp Thr Ser His Leu
        275                 280                 285

Phe Thr Thr Leu Gly Leu Arg Gly Leu Ser Gly Ile Gly Ala Phe Arg
    290                 295                 300

Gly Met Cys Thr Pro His Arg Ser Cys Ala Ile Val Thr Phe Met Asn
305                 310                 315                 320

Lys Thr Leu Gly Thr Phe Ser Ile Ala Val Ala His His Leu Gly His
                325                 330                 335

Asn Leu Gly Met Asn His Asp Glu Asp Thr Cys Arg Cys Ser Gln Pro
            340                 345                 350

Arg Cys Ile Met His Glu Gly Asn Pro Pro Ile Thr Lys Phe Ser Asn
        355                 360                 365

Cys Ser Tyr Gly Asp Phe Trp Glu Tyr Thr Val Glu Arg Thr Lys Cys
    370                 375                 380

Leu Leu Glu Thr Val His Thr Lys Asp Ile Phe Asn Val Lys Arg Cys
385                 390                 395                 400

Gly Asn Gly Val Val Glu Glu Gly Glu Glu Cys Asp Cys Gly Pro Leu
                405                 410                 415

Lys His Cys Ala Lys Asp Pro Cys Cys Leu Ser Asn Cys Thr Leu Thr
            420                 425                 430

Asp Gly Ser Thr Cys Ala Phe Gly Leu Cys Cys Lys Asp Cys Lys Phe
        435                 440                 445

Leu Pro Ser Gly Lys Val Cys Arg Lys Glu Val Asn Glu Cys Asp Leu
    450                 455                 460

Pro Glu Trp Cys Asn Gly Thr Ser His Lys Cys Pro Asp Asp Phe Tyr
465                 470                 475                 480

Val Glu Asp Gly Ile Pro Cys Lys Glu Arg Gly Tyr Cys Tyr Glu Lys
                485                 490                 495

Ser Cys His Asp Arg Asn Glu Gln Cys Arg Arg Ile Phe Gly Ala Gly
            500                 505                 510

Ala Asn Thr Ala Ser Glu Thr Cys Tyr Lys Glu Leu Asn Thr Leu Gly
        515                 520                 525

Asp Arg Val Gly His Cys Gly Ile Lys Asn Ala Thr Tyr Ile Lys Cys
    530                 535                 540

Asn Ile Ser Asp Val Gln Cys Gly Arg Ile Gln Cys Glu Asn Val Thr
545                 550                 555                 560

Glu Ile Pro Asn Met Ser Asp His Thr Thr Val His Trp Ala Arg Phe
                565                 570                 575

Asn Asp Ile Met Cys Trp Ser Thr Asp Tyr His Leu Gly Met Lys Gly
            580                 585                 590

Pro Asp Ile Gly Glu Val Lys Asp Gly Thr Glu Cys Gly Ile Asp His
        595                 600                 605

Ile Cys Ile His Arg His Cys Val His Ile Thr Ile Leu Asn Ser Asn
    610                 615                 620

Cys Ser Pro Ala Phe Cys Asn Lys Arg Gly Ile Cys Asn Asn Lys His
625                 630                 635                 640

His Cys His Cys Asn Tyr Leu Trp Asp Pro Pro Asn Cys Leu Ile Lys
                645                 650                 655

Gly Tyr Gly Gly Ser Val Asp Ser Gly Pro Pro Pro Lys Arg Lys Lys
            660                 665                 670

Lys Lys Lys Phe Cys Tyr Leu Cys Ile Leu Leu Leu Ile Val Leu Phe
        675                 680                 685

Ile Leu Leu Cys Cys Leu Tyr Arg Leu Cys Lys Lys Ser Lys Pro Ile
    690                 695                 700

Lys Lys Gln Gln Asp Val Gln Thr Pro Ser Ala Lys Glu Glu Glu Lys
705                 710                 715                 720

Ile Gln Arg Arg Pro His Glu Leu Pro Pro Gln Ser Gln Pro Trp Val
                725                 730                 735

Met Pro Ser Gln Ser Gln Pro Pro Val Thr Pro Ser Gln Ser His Pro
            740                 745                 750

Gln Val Met Pro Ser Gln Ser Gln Pro Pro Gln Asn Leu Phe Leu Phe
        755                 760                 765

Ser Phe Ser Ile Ser Asp Cys Val Leu Asn Phe Arg Leu Leu Tyr Leu
    770                 775                 780

Gln Ala Thr
785

<210> 14
<211> 820
<212> PRT
<213> Homo sapiens

<400> 14
Met Lys Met Leu Leu Leu Leu His Cys Leu Gly Val Phe Leu Ser Cys
1               5                   10                  15

Ser Gly His Ile Gln Asp Glu His Pro Gln Tyr His Ser Pro Pro Asp
            20                  25                  30

Val Val Ile Pro Val Arg Ile Thr Gly Thr Thr Arg Gly Met Thr Pro
        35                  40                  45

Pro Gly Trp Leu Ser Tyr Ile Leu Pro Phe Gly Gly Gln Lys His Ile
    50                  55                  60

Ile His Ile Lys Val Lys Lys Leu Leu Phe Ser Lys His Leu Pro Val
65                  70                  75                  80

Phe Thr Tyr Thr Asp Gln Gly Ala Ile Leu Glu Asp Gln Pro Phe Val
                85                  90                  95

Gln Asn Asn Cys Tyr Tyr His Gly Tyr Val Glu Gly Asp Pro Glu Ser
            100                 105                 110

Leu Val Ser Leu Ser Thr Cys Phe Gly Gly Phe Gln Gly Ile Leu Gln
        115                 120                 125

Ile Asn Asp Phe Ala Tyr Glu Ile Lys Pro Leu Ala Phe Ser Thr Thr
    130                 135                 140

Phe Glu His Leu Val Tyr Lys Met Asp Ser Glu Glu Lys Gln Phe Ser
145                 150                 155                 160

Thr Met Arg Ser Gly Phe Met Gln Asn Glu Ile Thr Cys Arg Met Glu
                165                 170                 175

Phe Glu Glu Ile Asp Asn Ser Thr Gln Lys Gln Ser Ser Tyr Val Gly
            180                 185                 190

Trp Trp Ile His Phe Arg Ile Val Glu Ile Val Val Val Ile Asp Asn
        195                 200                 205

Tyr Leu Tyr Ile Arg Tyr Glu Arg Asn Asp Ser Lys Leu Leu Glu Asp
    210                 215                 220

Leu Tyr Val Ile Val Asn Ile Val Asp Ser Ile Leu Asp Val Ile Gly
225                 230                 235                 240

Val Lys Val Leu Leu Phe Gly Leu Glu Ile Trp Thr Asn Lys Asn Leu
                245                 250                 255

Ile Val Val Asp Asp Val Arg Lys Ser Val His Leu Tyr Cys Lys Trp
            260                 265                 270

Lys Ser Glu Asn Ile Thr Pro Arg Met Gln His Asp Thr Ser His Leu
        275                 280                 285

Phe Thr Thr Leu Gly Leu Arg Gly Leu Ser Gly Ile Gly Ala Phe Arg
    290                 295                 300

Gly Met Cys Thr Pro His Arg Ser Cys Ala Ile Val Thr Phe Met Asn
305                 310                 315                 320

Lys Thr Leu Gly Thr Phe Ser Ile Ala Val Ala His His Leu Gly His
                325                 330                 335

Asn Leu Gly Met Asn His Asp Glu Asp Thr Cys Arg Cys Ser Gln Pro
            340                 345                 350

Arg Cys Ile Met His Glu Gly Asn Pro Pro Ile Thr Lys Phe Ser Asn
        355                 360                 365

Cys Ser Tyr Gly Asp Phe Trp Glu Tyr Thr Val Glu Arg Thr Lys Cys
    370                 375                 380

Leu Leu Glu Thr Val His Thr Lys Asp Ile Phe Asn Val Lys Arg Cys
385                 390                 395                 400

Gly Asn Gly Val Val Glu Glu Gly Glu Glu Cys Asp Cys Gly Pro Leu
                405                 410                 415

Lys His Cys Ala Lys Asp Pro Cys Cys Leu Ser Asn Cys Thr Leu Thr
            420                 425                 430

Asp Gly Ser Thr Cys Ala Phe Gly Leu Cys Cys Lys Asp Cys Lys Phe
        435                 440                 445

Leu Pro Ser Gly Lys Val Cys Arg Lys Glu Val Asn Glu Cys Asp Leu
    450                 455                 460

Pro Glu Trp Cys Asn Gly Thr Ser His Lys Cys Pro Asp Asp Phe Tyr
465                 470                 475                 480

Val Glu Asp Gly Ile Pro Cys Lys Glu Arg Gly Tyr Cys Tyr Glu Lys
                485                 490                 495

Ser Cys His Asp Arg Asn Glu Gln Cys Arg Arg Ile Phe Gly Ala Gly
            500                 505                 510

Ala Asn Thr Ala Ser Glu Thr Cys Tyr Lys Glu Leu Asn Thr Leu Gly
        515                 520                 525

Asp Arg Val Gly His Cys Gly Ile Lys Asn Ala Thr Tyr Ile Lys Cys
    530                 535                 540

Asn Ile Ser Asp Val Gln Cys Gly Arg Ile Gln Cys Glu Asn Val Thr
545                 550                 555                 560

Glu Ile Pro Asn Met Ser Asp His Thr Thr Val His Trp Ala Arg Phe
                565                 570                 575

Asn Asp Ile Met Cys Trp Ser Thr Asp Tyr His Leu Gly Met Lys Gly
            580                 585                 590

Pro Asp Ile Gly Glu Val Lys Asp Gly Thr Glu Cys Gly Ile Asp His
        595                 600                 605

Ile Cys Ile His Arg His Cys Val His Ile Thr Ile Leu Asn Ser Asn
    610                 615                 620

Cys Ser Pro Ala Phe Cys Asn Lys Arg Gly Ile Cys Asn Asn Lys His
625                 630                 635                 640

His Cys His Cys Asn Tyr Leu Trp Asp Pro Pro Asn Cys Leu Ile Lys
                645                 650                 655

Gly Tyr Gly Gly Ser Val Asp Ser Gly Pro Pro Pro Lys Arg Lys Lys
            660                 665                 670

Lys Lys Lys Phe Cys Tyr Leu Cys Ile Leu Leu Leu Ile Val Leu Phe
        675                 680                 685

Ile Leu Leu Cys Cys Leu Tyr Arg Leu Cys Lys Lys Ser Lys Pro Ile
    690                 695                 700

Lys Lys Gln Gln Asp Val Gln Thr Pro Ser Ala Lys Glu Glu Glu Lys
705                 710                 715                 720

Ile Gln Arg Arg Pro His Glu Leu Pro Pro Gln Ser Gln Pro Trp Val
                725                 730                 735

Met Pro Ser Gln Ser Gln Pro Pro Val Thr Pro Ser Gln Ser His Pro
            740                 745                 750

Arg Val Met Pro Ser Gln Ser Gln Pro Pro Val Met Pro Ser Gln Ser
        755                 760                 765

His Pro Gln Leu Thr Pro Ser Gln Ser Gln Pro Pro Val Met Pro Ser
    770                 775                 780

Gln Ser His Pro Gln Leu Thr Pro Ser Gln Ser Gln Pro Pro Val Thr
785                 790                 795                 800

Pro Ser Gln Arg Gln Pro Gln Leu Met Pro Ser Gln Ser Gln Pro Pro
                805                 810                 815

Val Thr Pro Ser
            820


<210> 15
<211> 790
<212> PRT
<213> Homo sapiens

<400> 15

<210> 16
<211> 781
<212> PRT
<213> Homo sapiens

<400> 16
   Met Arg Ser Val Gln Ile Phe Leu Ser Gln Cys Arg Leu Leu Leu Leu
   1 5 10 15
   Leu Val Pro Thr Met Leu Leu Lys Ser Leu Gly Glu Asp Val Ile Phe
   20 25 30
   His Pro Glu Gly Glu Phe Asp Ser Tyr Glu Val Thr Ile Pro Glu Lys
   35 40 45
   Leu Ser Phe Arg Gly Glu Val Gln Gly Val Val Ser Pro Val Ser Tyr
   50 55 60
   Leu Leu Gln Leu Lys Gly Lys Lys His Val Leu His Leu Trp Pro Lys
   65 70 75 80
   Arg Leu Leu Leu Pro Arg His Leu Arg Val Phe Ser Phe Thr Glu His
   85 90 95
   Gly Glu Leu Leu Glu Asp His Pro Tyr Ile Pro Lys Asp Cys Asn Tyr
   100 105 110
   Met Gly Ser Val Lys Glu Ser Leu Asp Ser Lys Ala Thr Ile Ser Thr
   115 120 125
   Cys Met Gly Gly Leu Arg Gly Val Phe Asn Ile Asp Ala Lys His Tyr
   130 135 140
   Gln Ile Glu Pro Leu Lys Ala Ser Pro Ser Phe Glu His Val Val Tyr
   145 150 155 160
   Leu Leu Lys Lys Glu Gln Phe Gly Asn Gln Val Cys Gly Leu Ser Asp
   165 170 175
   Asp Glu Ile Glu Trp Gln Met Ala Pro Tyr Glu Asn Lys Ala Arg Leu
   180 185 190
   Arg Asp Phe Pro Gly Ser Tyr Lys His Pro Lys Tyr Leu Glu Leu Ile
           195                 200                 205
   Leu Leu Phe Asp Gln Ser Arg Tyr Arg Phe Val Asn Asn Asn Leu Ser
       210                 215                 220
   Gln Val Ile His Asp Ala Ile Leu Leu Thr Gly Ile Met Asp Thr Tyr
   225                 230                 235                 240
   Phe Gln Asp Val Arg Met Arg Ile His Leu Lys Ala Leu Glu Val Trp
                   245                 250                 255
   Thr Asp Phe Asn Lys Ile Arg Val Gly Tyr Pro Glu Leu Ala Glu Val
               260                 265                 270
   Leu Gly Arg Phe Val Ile Tyr Lys Lys Ser Val Leu Asn Ala Arg Leu
           275                 280                 285
   Ser Ser Asp Trp Ala His Leu Tyr Leu Gln Arg Lys Tyr Asn Asp Ala
       290                 295                 300
   Leu Ala Trp Ser Phe Gly Lys Val Cys Ser Leu Glu Tyr Ala Gly Ser
   305                 310                 315                 320
   Val Ser Thr Leu Leu Asp Thr Asn Ile Leu Ala Pro Ala Thr Trp Pro
                   325                 330                 335
   Ala His Glu Leu Gly His Ala Val Gly Met Ser His Asp Glu Gln Tyr
               340                 345                 350
   Cys Gln Cys Arg Gly Arg Leu Asn Cys Ile Met Gly Ser Gly Arg Thr
           355                 360                 365
   Gly Phe Ser Asn Cys Ser Tyr Ile Ser Phe Phe Lys His Ile Ser Ser
       370                 375                 380
   Gly Ala Thr Cys Leu Asn Asn Ile Pro Gly Leu Gly Tyr Val Leu Lys
   385                 390                 395                 400
   Arg Cys Gly Asn Lys Ile Val Glu Asp Asn Glu Glu Cys Asp Cys Gly
                   405                 410                 415
   Ser Thr Glu Glu Cys Gln Lys Asp Arg Cys Cys Gln Ser Asn Cys Lys
               420                 425                 430
   Leu Gln Pro Gly Ala Asn Cys Ser Ile Gly Leu Cys Cys His Asp Cys
           435                 440                 445
   Arg Phe Arg Pro Ser Gly Tyr Val Cys Arg Gln Glu Gly Asn Glu Cys
       450                 455                 460
   Asp Leu Ala Glu Tyr Cys Asp Gly Asn Ser Ser Ser Cys Pro Asn Asp
   465                 470                 475                 480
   Val Tyr Lys Gln Asp Gly Thr Pro Cys Lys Tyr Glu Gly Arg Cys Phe
                   485                 490                 495
   Arg Lys Gly Cys Arg Ser Arg Tyr Met Gln Cys Gln Ser Ile Phe Gly
               500                 505                 510
   Pro Asp Ala Met Glu Ala Pro Ser Glu Cys Tyr Asp Ala Val Asn Leu
           515                 520                 525
   Ile Gly Asp Gln Phe Gly Asn Cys Glu Ile Thr Gly Ile Arg Asn Phe
       530                 535                 540
   Lys Lys Cys Glu Ser Ala Asn Ser Ile Cys Gly Arg Leu Gln Cys Ile
   545                 550                 555                 560
   Asn Val Glu Thr Ile Pro Asp Leu Pro Glu His Thr Thr Ile Ile Ser
                   565                 570                 575
   Thr His Leu Gln Ala Glu Asn Leu Met Cys Trp Gly Thr Gly Tyr His
               580                 585                 590
   Leu Ser Met Lys Pro Met Gly Ile Pro Asp Leu Gly Met Ile Asn Asp
           595                 600                 605
   Gly Thr Ser Cys Gly Glu Gly Arg Val Cys Phe Lys Lys Asn Cys Val
       610                 615                 620
   Asn Ser Ser Val Leu Gln Phe Asp Cys Leu Pro Glu Lys Cys Asn Thr
   625                 630                 635                 640
   Arg Gly Val Cys Asn Asn Arg Lys Asn Cys His Cys Met Tyr Gly Trp
                   645                 650                 655
   Ala Pro Pro Phe Cys Glu Glu Val Gly Tyr Gly Gly Ser Ile Asp Ser
               660                 665                 670
   Gly Pro Pro Gly Leu Leu Arg Gly Ala Ile Pro Ser Ser Ile Trp Val
           675                 680                 685
   Val Ser Ile Ile Met Phe Arg Leu Ile Leu Leu Ile Leu Ser Val Val
       690                 695                 700
   Phe Val Phe Phe Arg Gln Val Ile Gly Asn His Leu Lys Pro Lys Gln
   705                 710                 715                 720
   Glu Lys Met Pro Leu Ser Lys Ala Lys Thr Glu Gln Glu Glu Ser Lys
                   725                 730                 735
   Thr Lys Thr Val Gln Glu Glu Ser Lys Thr Lys Thr Gly Gln Glu Glu
               740                 745                 750
   Ser Glu Ala Lys Thr Gly Gln Glu Glu Ser Lys Ala Asn Ile Glu Ser
           755                 760                 765
   Lys Arg Pro Lys Ala Lys Ser Val Lys Lys Gln Lys Lys
       770                 775                 780

<210> 17
<211> 23
<212> DNA

<213> Artificial Sequence

<220>
<223> Description of Artificial Sequence:
      oligonucleotide

<400> 17
cacctaaggt gttcaattct ttg                                              23


<210> 18
<211> 23
<212> DNA
<213> Artificial Sequence

<220>
<223> Description of Artificial Sequence:
      oligonucleotide

<400> 18
caaatactgc aagtgagact tgc                                              23


<210> 19
<211> 24
<212> DNA
<213> Artificial Sequence

<220>
<223> Description of Artificial Sequence:
      oligonucleotide

<400> 19
tgcacaacta cgtgtggtgt accc                                             24


<210> 20
<211> 26
<212> DNA
<213> Artificial Sequence

<220>
<223> Description of Artificial Sequence:
      oligonucleotide

<400> 20
gagccactgc aattgaaaaa gtgccc                                           26


<210> 21
<211> 21
<212> DNA
<213> Artificial Sequence

<220>
<223> Description of Artificial Sequence:
      oligonucleotide

<400> 21
aatgatgctc ttgcatggtc g                                                21

<210> 22
<211> 26
<212> DNA
<213> Artificial Sequence

<220>
<223> Description of Artificial Sequence:
      oligonucleotide

<400> 22
ctttcacgga gcccatgtag ttgcag                                           26

<210> 23
<211> 26
<212> DNA
<213> Artificial Sequence

<220>
<223> Description of Artificial Sequence:
      oligonucleotide

<400> 23
tgaaggagaa aacgcgcaga tgtcgg                                           26

<210> 24
<211> 26
<212> DNA
<213> Artificial Sequence

<220>
<223> Description of Artificial Sequence: primer

<400> 24
tcgataatgc atgaaggcaa cccacc                                           26

<210> 25
<211> 26
<212> DNA
<213> Artificial Sequence

<220>
<223> Description of Artificial Sequence: primer

<400> 25
caagtctcac ttgcagtatt tgcgcc                                           26

<210> 26
<211> 19
<212> DNA
<213> Artificial Sequence

<220>
<223> Description of Artificial Sequence: primer

<400> 26
gccactgcat gtatgggtg                                                    19


<210> 27
<211> 21
<212> DNA
<213> Artificial Sequence

<220>
<223> Description of Artificial Sequence: primer

<400> 27
gacactcttt gctttgggtc g                                                 21


<210> 28
<211> 8
<212> PRT
<213> Artificial Sequence

<220>
<223> Description of Artificial Sequence: peptide
      fragment

<400> 28
Asp Tyr Lys Asp Asp Asp Asp Lys
  1               5


<210> 29
<211> 27
<212> PRT
<213> Artificial Sequence

<220>
<223> Description of Artificial Sequence: peptide
      fragment

<400> 29
Pro Asp Val Ala Ser Leu Arg Gln Gln Val Glu Ala Leu Gln Gly Gln
  1               5                  10                  15

Val Gln His Leu Gln Ala Ala Phe Ser Gln Tyr
             20                  25


<210> 30
<211> 33
<212> PRT
<213> Artificial Sequence

<220>
<223> Description of Artificial Sequence: peptide
      fragment

<400> 30
Arg Met Lys Gln Ile Glu Asp Lys Ile Glu Glu Ile Leu Ser Lys Ile
  1               5                  10                  15

Tyr His Ile Glu Asn Glu Ile Ala Arg Ile Lys Lys Leu Ile Gly Glu
             20                  25                  30

Arg


<210> 31
<211> 12
<212> PRT
<213> Artificial Sequence

<220>
<223> Description of Artificial Sequence: peptide
      fragment

<220>
<223> "Xaa" at various positions throughout the sequence
      may be any amino acid

<400> 31
His Glu Xaa Xaa His Xaa Xaa Gly Xaa Xaa His Asp
  1               5                  10


<210> 32
<211> 9
<212> PRT
<213> Artificial Sequence

<220>
<223> Description of Artificial Sequence: peptide
      fragment

<400> 32
Ser Gln Ser Gln Pro Pro Leu Met Pro
  1               5


<210> 33
<211> 9
<212> PRT
<213> Artificial Sequence

<220>
<223> Description of Artificial Sequence: peptide
      fragment

<220>
<223> "Xaa" at position 6 may be Thr or Ala

<400> 33
Gln Glu Glu Ser Lys Xaa Lys Thr Gly
1               5

## Description

### CROSS REFERENCE TO RELATED APPLICATIONS

This application hereby claims the benefit of United States provisional applications S.N. 60/116,670; S.N. 60/138,682; and S.N. 60/155,798; filed January 21, 1999; June 14, 1999; and September 27, 1999, respectively. The entire disclosures of these applications are relied upon and incorporated by reference herein.

### BACKGROUND OF THE INVENTION

### Field of the Invention

The invention is directed to purified and isolated, novel SVPH polypeptides (SVPH-1, SVPH-1a, SVPH-1b, SVPH-1c; SVPH-3; and SVPH-4, SVPH-4a, and SVPH-4b) and fragments thereof, the nucleic acids encoding such polypeptides, processes for production of recombinant forms of such polypeptides, antibodies generated against these polypeptides, fragmented peptides derived from these polypeptides, and uses thereof.

### Description of Related Art

Metalloproteinases are a group of proteinases characterized by the presence of a metal prosthetic group. Despite this basic similarity, the group, which' includes proteinases from snake venom, numerous microbial proteinases, and vertebrate and bacterial collagenases, would seem to present proteinases of seemingly widely varying activities. For example, snake venom proteases are metalloproteinases that affect cellmatrix interactions. Snake venom also includes "disintegrins," a class of low molecular weight, Arg-Gly-Asp (RGD)-containing, cysteine-rich peptides which bind to integrins (a family of molecules involved in cell-to-cell adhesion, cell-to-matrix adhesion, and inflammatory responses) expressed on cells.

Also included are the membrane-anchored ADAMs (**A D**isintegrin **A**nd **M**etalloproteinase), which are multimeric molecules consisting of metalloproteinase, disintegrin-like, cysteine rich, and epidermal growth factor domains. *See,* Black et al., "ADAMs: focus on the protease domain," Curr. Opin. Cell Biol. 10:654-659 (1998); Wolfsberg, T.G. et al., "ADAMs in fertilization and development," Dev. Bio. 180:389-401 (1996), all of which are herein incorporated by reference. The metalloproteinase-disintegrins or ADAMs have a unique domain structure composed of a signal sequence, pro-domain with a Cys switch, catalytic domain with a zinc-binding motif, disintegrin domain, cysteine-rich domain, a transmembrane domain, and a cytoplasmic domain (Black et al., "ADAMs: focus on the protease domain," Curr. Opin. Cell Biol. 10:654-659 (1998) ; Blobel, C. P., Cell, 90:589-592 (1997)). Thus, ADAMs are type 1 transmembrane proteins expressed on the cell surface. ADAMs have been isolated from mammalian species, *Caenorhabditis, Xenopus,* and *Drosophila.* Approximately half of the ADAMs do not contain the zinc-binding motif **HE**XX**H**XX**G**XX**HD** (SEQ ID NO:31), which is though to be required for enzymatic activity. However, all ADAMs contain the disintegrin domain, which is approximately 80 amino acids in length with 15 highly conserved Cys residues. In some members this region has been found to bind integrins (Almeida, E.A. et al., Cell 81:1095-1104 (1995); Zhang, X. P. et al., J. Biol. Chem. 273:7345-7350 (1998); Nath, D. et al., J. Cell Sci. 112:579-587 (1999)), although the role of this domain for the majority of the family members is unknown.

Over two dozen ADAMs have been identified but only a few have had their biological roles elucidated. Tumor necrosis factor-α converting enzyme (TACE/ADAM17) was isolated as the proteinase required for the shedding of TNF-α from the plasma membrane. *See,* Blobel, C.P, Cell, 90:589-592 (1997); Moss, M. et al., Nature 385:733-736 (1997); Black, R.A. et al., Nature 385:729-733 (1997). More recently TACE/ADAM17 has been found to be required for the ectodomain shedding of other cell surface proteins including L-selectin, TGF-α, p80 TNFR, p60TNFR, L-selectin, type II IL-1R, and β-amyloid precursor protein (Peschon, J. J. et al., Science 282:1281-1284 (1998)). Fertilin-α/ADAM1 and fertilin-β/ADAM2 are required for sperm-egg fusion (Myles, D. G. et al., Proc. Nat'l. Acad. Sci., USA 91:4195-4198 (1994)) while meltrin-α/ADAM12 has a role in muscle cell fusion (Yagami-Hiromasa, T. et al., Nature 377:652-656 (1995)). In addition MDC/ADAM11 is a candidate tumor suppressor gene (Emi, M. et al., Nat. Genet. 5:151-157 (1993)) and *Kuz* /ADAM10 plays an important role in neurogenesis (Pan, D. et al., Cell 90: 271-280 (1997); Rooke, J. et al., Science 273:1227-1231 (1996)).

Some ADAMs are ubiquitously expressed such as ADAM9, ADAM10, ADAM15, and ADAM 17 and may have pleiotropic effects, as has been found for ADAM15 and ADAM17. Many of the other ADAMs, however, show tissue-specific expression: ADAM12 and ADAM19 in muscle (Yagami-Hiromasa, T. et al., Nature 377:652-656 (1995)), ADAM22 in brain, and ADAM23 in brain and heart (Sagane, K. et al., J. Biochem. 334:93-98 (1998)). The largest group of ADAMs (Bjarnason, J. B. et al., Methods Enzymol. 248: 345-368 (1995); Jia, L. G. et al., Toxicon 34:1269-1276 (1996); Stocker, W. et al., Protein Sci. 4:823-840 (1995); Black, R. A. et al., Curr. Opin. Cell Biol. 10:654-659 (1998); Blobel, C. P. , Cell 90:589-592 (1997); Almeida, E. A. et al., Cell 81:1095-1104 (1995); Zhang, X. P. et al., J. Biol. Chem. 273:7345-7350 (1998); Wolfsberg, T. G. et al., Dev. Biol. 180:389-401 (1996); Zhu, G. Z. et al., Gene 234:227-237 (1999); Blobel, C. P. et al., Nature 356:248-252 (1992); Walter, M. A. et al., Nat. Genet. 7:22-28 (1994); Gribskov, M. et al., Nucleic Acids Res. 14:6745-6763 (1986); Bode, W. et al., FEBS Lett. 331:134-140 (1993); and Cerretti, D. P. et al., Cytokine 11:541-551 (1999)) is predominately expressed in testis and is thought to be involved in spermatogenesis and fertilization (Wolfsberg, T. G. et al., Dev. Biol. 180:389-401 (1996); Hooft van Huijsduijnen, R., Gene 206:273-282 (1998); Zhu, G. Z. et al., Gene 234:227-237 (1999)). Indeed, the first mammalian ADAMs discovered, ADAM1 and ADAM2, were found to be required for sperm-egg fusion (Zhu, G. Z. et al., Gene 234:227-237 (1999)).

The ADAMs family of metalloproteinase-disintegrins also share homology with the snake venom protease family (SVPH). In some snake venom protease members, the disintegrin domain prevents platelet aggregation and thus acts as an anti-coagulant.

Given the significant function ofmetalloproteinases in membrane and cell-cell fusion, cellular adhesion, shedding of membrane proteins, and anti-coagulation, there is a need in the art for additional metalloproteinases of the ADAMs family and/or the SVPH family, including the discovery, identification, and roles of new proteins within these families.

In another aspect, the identification of the primary structure, or sequence, of an unknown protein is the culmination of an arduous process of experimentation. In order to identify an unknown protein, the investigator can rely upon a comparison of the unknown protein to known peptides using a variety of techniques known to those skilled in the art. For instance, proteins are routinely analyzed using techniques such as electrophoresis, sedimentation, chromatography, sequencing and mass spectrometry

In particular, comparison of an unknown protein to polypeptides of known molecular weight allows a determination of the apparent molecular weight of the unknown protein (Brock, T. D. et al., Biology of Microorganisms 76-77 (1991)). Protein molecular weight standards are commercially available to assist in the estimation of molecular weights of unknown protein (New England Biolabs Inc. Catalog:130-131 (1995); J. L. Hartley, U.S. Patent No. 5,449,758). However, the molecular weight standards may not correspond closely enough in size to the unknown protein to allow an accurate estimation of apparent molecular weight. The difficulty in estimation of molecular weight is compounded in the case of proteins that are subjected to fragmentation by chemical or enzymatic means, modified by post-translational modification or processing, and/or associated with,other proteins in non-covalent complexes.

In addition, the unique nature of the composition of a protein with regard to its specific amino acid constituents results in unique positioning of cleavage sites within the protein Specific fragmentation of a protein by chemical or enzymatic cleavage results in a unique "peptide fingerprint" (Cleveland, D. W. et al., J. Biol. Chem. 252:1102-1106 (1977); Brown, M. et al., J. Gen. Virol. 50:309-316 (1980)). Consequently, cleavage at specific sites results in reproducible fragmentation of a given protein into peptides of precise molecular weights. Furthermore, these peptides possess unique charge characteristics that determine the isoelectric pH of the peptide. These unique characteristics can be exploited using a variety of electrophoretic and other techniques (Brock, T. D. et al., Biology of Microorganisms 76-77 (Prentice Hall, 6th ed. 1991)).

Fragmentation of proteins is further employed for amino acid composition analysis and protein sequencing (Matsudiara, P., J. Biol. Chem. 262:10035-10038 (1987); Eckerskorn, C. et al., Electrophoresis 1988, 9:830-838 (1988)), particularly the production of fragments from proteins with a "blocked" N-terminus. In addition, fragmented proteins can be used for immunization, for affinity selection (R. A. Brown, U.S. Patent No. 5,151,412), for determination of modification sites (*e.g.* phosphorylation), for generation of active biological compounds (Brock, T. D. et al., Biology of Microorganisms 300-301 (Prentice Hall, 6th ed. 1991)), and for differentiation of homologous proteins (Brown, M. et al., J. Gen. Virol. 50:309-316 (1980)).

In addition, when a peptide fingerprint of an unknown protein is obtained, it can be compared to a database of known proteins to assist in the identification of the unknown protein using mass spectrometry (Henzel, W. J. et al., Proc. Natl. Acad. Sci. USA 90:5011-5015 (1993); Fenyo, D. et al., Electrophoresis 19:998-1005 (1998)). A variety of computer software programs to facilitate these comparisons are accessible via the Internet, such as Protein Prospector (Internet site: prospector.uscf.edu), Multildent (Internet site: www.expasy.ch/sprot/multiident.html), PeptideSearch (Internet site: www.mann.embl-heiedelberg.de...deSearch/FR_PeptideSearch Form.html), and ProFound (Internet site: www.chait-sgi.rockefeller.edu/cgi-bin/protid-frag.html). These programs allow the user to specify the cleavage agent and the molecular weights of the fragmented peptides within a designated tolerance. The programs compare these molecular weights to protein molecular weight information stored in databases to assist in determining the identity of the unknown protein. Accurate information concerning the number of fragmented peptides and the precise molecular weight of those peptides is required for accurate identification. Therefore, increasing the accuracy in determining the number of fragmented peptides and their molecular weight should result in enhanced likelihood of success in the identification of unknown proteins.

In addition, peptide digests of unknown proteins can be sequenced using tandem mass spectrometry (MS/MS) and the resulting sequence searched against databases (Eng, J. K. et al., J. Am. Soc. Mass Spec. 5:976-989 (1994); Mann, M. et al., Anal. Chem. 66:4390-4399 (1994); Taylor, J.A. et al., Rapid Comm. Mass Spec. 11:1067-1075 (1997)). Searching programs that can be used in this process exist on the Internet, such as Lutefisk 97 (Internet site: www.lsbc.com:70/Lutefisk97.html), and the Protein Prospector, Peptide Search and ProFound programs described above. Therefore, adding the sequence of a gene and its predicted protein sequence and peptide fragments to a sequence database can aid in the identification of unknown proteins using tandem mass spectrometry.

Thus, there also exists a need in the art for polypeptides suitable for use in peptide fragmentation studies, for use in molecular weight measurements, and for use in protein sequencing using tandem mass spectrometry.

### SUMMARY OF THE INVENTION

The invention aids in fulfilling these various needs in the art by providing isolated, novel SVPH nucleic acids and polypeptides encoded by these nucleic acids. Particular embodiments of the invention are directed to an isolated SVPH nucleic acid molecule comprising the DNA sequence of SEQ ID NOs:1-3 and isolated SVPH nucleic acid molecules encoding the amino acid sequence of SEQ ID NOs:4-6, as well as nucleic acid molecules complementary to these sequences. Further studies have revealed the full-length nucleotide sequences of three alternatively spliced SVPH-1 clones (SEQ ID NOs:7-9) and two alternatively spliced SVPH 4 clones (SEQ ID NOs:10-11). Thus, further embodiments of the invention are directed to an isolated SVPH nucleic acid molecule comprising the DNA sequence of SEQ ID NOs:7-11 and isolated SVPH nucleic acid molecules encoding the amino acid sequence of SEQ ID NOs:12-16, as well as nucleic acid molecules complementary to these sequences. Both single-stranded and double-stranded RNA and DNA nucleic acid molecules are encompassed by the invention, as well as nucleic acid molecules that hybridize to a denatured, double-stranded DNA comprising all or a portion of SEQ ID NOs:1-3 and 7-11. Also encompassed are isolated nucleic acid molecules that are derived by *in vitro* mutagenesis of nucleic acid molecules comprising sequences of SEQ ID NOs:1-3 and 7-11, that are degenerate from nucleic acid molecules comprising sequences of SEQ ID NOs: 1-3 and 7-11, and that are allelic variants of DNA of the invention. The invention also encompasses recombinant vectors that direct the expression of these nucleic acid molecules and host cells stably or transiently transformed or transfected with these vectors.

In addition, the invention encompasses methods of using the nucleic acids noted above to identify nucleic acids encoding proteins having metalloproteinase-disintegrin activities; to identify human chromosome number 1 or 4; to map genes on human chromosome number 1 or 4; to identify genes associated with certain diseases, syndromes, or other human conditions associated with human chromosome number 1 or 4; and to study proteinases and their activities on cell/cell interactions as well as proteinase activity on the immune system.

The invention also encompasses the use of sense or antisense oligonucleotides from the nucleic acid of SEQ ID NOs:1-3 and 7-11 to inhibit the expression of the polynucleotides encoded by the SVPH-1, SVPH-3, or SVPH-4 genes.

The invention also encompasses isolated polypeptides and fragments thereof encoded by these nucleic acid molecules including soluble polypeptide portions of SEQ ID Nos:4-6 and 12-16. The invention further encompasses methods for the production of these polypeptides, including culturing a host cell under conditions promoting expression and recovering the polypeptide from the culture medium Especially, the expression of these polypeptides in bacteria, yeast, plant, insect, and animal cells is encompassed by the invention.

In general, the polypeptides of the invention can be used to study the cell/cell and cell/matrix interactions involved in cellular processes (including cell fusion as in sperm/egg interactions, cell recognition and binding) as well as those involved in the immune system. In addition, these polypeptides can be used to identify other proteins associated with SVPH family members, ADAMs family members, and other metalloproteinases

In addition, the invention includes assays utilizing these polypeptides to screen for potential inhibitors of activity associated with polypeptide counter-structure molecules, and methods of using these polypeptides as therapeutic agents for the treatment of diseases mediated by SVPH polypeptide counter-structure molecules. Further, methods of using these polypeptides in the design of inhibitors thereof are also an aspect of the invention.

The invention further provides a method for using these polypeptides as molecular weight markers that allow the estimation of the molecular weight of a protein or a fragmented protein, as well as a method for the visualization of the molecular weight markers of the invention thereof using electrophoresis. The invention further encompasses methods for using the polypeptides of the invention as markers for determining the isoelectric point of an unknown protein, as well as controls for establishing the extent of fragmentation of a protein. Further encompassed by this invention are kits to aid in these determinations.

Isolated polyclonal or monoclonal antibodies that bind to these polypeptides are also encompassed by the invention, in addition the use of these antibodies to aid in purifying the SVPH polypeptide.

Further encompassed by this invention is the use of the SVPH nucleic acid sequences, predicted amino acid sequences of the polypeptide or fragments thereof, or a combination of the predicted amino acid sequences of the polypeptide and fragments thereof for use in searching an electronic database to aid in the identification of sample nucleic acids and/or proteins.

### BRIEF DESCRIPTION OF THE FIGURES

Figure 1 depicts a Northern blot hybridization showing the tissue specificity of SVPH-1 and SVPH-4.
Figure 2 depicts a phylogenetic tree of metalloproteinase-disintegrins.
Branches marked with heavy lines indicate ADAM family members with a consensus zinc-binding motif (**HE**XX**H**XX**G**XX**HD)** (SEQ ID NO:31). The arrow indicates the probable zinc-binding motif containing common ancestor. Lineages in which the zinc-binding site was subsequently lost are denoted with an 'X'. Species abbreviations: Mm, *Mus musculus;* Rn, *Rattus norvegicus;* Hs, *Homo sapiens;* Mf, *Macaca fascicularis;* Oc, *Oryctolagus cuniculus* ; Cc, *Cavia cobaya,* Cp, *Cavia porcellus;* So, *Saguinus oedipus;* Pp, *Pongo pygmaeus;* Bt, Bos *taurus.*

### DETAILED DESCRIPTION OF THE INVENTION

The nucleic acid molecules encompassed in the invention include the following nucleotide sequences:
**Name: SVPH-1** 
1 ATTTTTGATA CCACAGTGAC CAACACGGTC ACCTAAGGTG TTCAATTCTT
51 TGTAGCAAGT CTCACTTGCA GTATTTGCGC CTGCACCAAA AATCCTCCTA
101 CACTGTTCAN TTGCGGTCAT GACANGCTC (SEQ ID NO:1)
**Name: SVPH-3** 
1 TTTTTGAGTA AGAATAGGTC ATGTTTTAGT AAAACTTCCA AAAGAACAAA
51 ACAGATTCTT CAACCCAGGA GGACATGTGA GTCACAATAC CCTTTAATCC
101 ACAGGTTGGC TCCTTGGTTT CTGGAACTTT CTGCCTCCTG TAAACGATGT
151 GCGGGTGGTA CCCTCCCTCA ACCAGTGGAT GCTTCTTCAC GGGTTCAATG
201 AAAAAGTCTC CATGTGGTAG TTGGAAAAAT CCAGTCAGTC CATGGCAGGC
251 ACTGAGGGCT GCCGTCCCAA CTCTGGTGCC CTGCTGTAGA ACCGTGCCAC
301 TGAGATGGCA GAGGGGGGCA GAGGAAGCCA TCATCTTAAC ATGGGAGAGG
351 TTCCCATATC TCTTCTCCAT GATGTAGCTA TTGGAAAGAA ATCCTTCATT
401 GACCGTCAAG TTAAAAAACA GGTCCTTCTC CTCGTGAGAA ATTCTGTAGT
451 ACACCCAGTC CTCTGAGCC (SEQ ID NO:2)
**Name: SVPH-4** 
1 CACGAGGATT TATATCTTCA AAGAAAATAT AATGATGCTC TTGCATGGTC
51 GTTTGGAAAA GTGTGTTCTC TAGAATATGC TGGATCAGTG AGTACTTTAC
101 TAGATACAAA TATCCTTGCC CCTGCTACCT GGTCTGCTCA TGAGCTGGGT
151 CATGCTGTAG GAATGTCACA TGATGAACAA TACTGCCAAT GTAGGGGTAG
201 GCCTAATTGC ATCATGGGCT CAGGACGCAC TGGGTTTAGC AATTGCAGTT
251 ATATCTCTTT TTTTAAACAT ATCTCTTCGG GAGCAACATG TCTAAATAAT
301 ATCCCAGGAC TAGGTTATGT GCTTAAGAGA TGTGGAAACA AAATTGTGGA
351 GGACAATGAG GAATGTGATT GTGGTTCCAC AGAGGAGTGT CAGAAAGATC
401 GGTGTTGCCA ATCAAATTGT AAGTTGCAAC CAGGTGCCAA CTGTAGCATT
451 GGACTTTGCT GTCATGATTG TCGGTTTCGT CCATCTGGAT ACGTGTGTAG
501 GCAGGAAGGA AATGAATGTG ACCTTGCAGA GTACTGCGAC GGGAATTCAA
551 GTTCCTGCCC AAATGACGTT TATAAGCAGG ATGGAACCCC TTGCAAGTAT
601 GAAGGCCGTT GTTTCAGGAA GGGGTGCAGA TCCAGATATA TGCAGTGCCA
651 AAGCATTTTT GGACCTGATG CCATGGAGGC TCCTAGTGAG TGCTATGATG
701 CAGTTAACTT AATAGGTGAT CAATTTGGTA ACTGTGAGAT TACAGGAATT
751 CGAAATTTTA AAAAGTGTGA AAGTGCAAAT TCAATATGTG GCAGGCTACA
801 GTGTATAAAT GTTGAAACCA TCCCTGATTT GCCAGAGCAT ACGACTATAA
851 TTTCTACTCA TTTACAGGCA GAAAATCTCA TGTGCTGGGG CACAGGCTAT
901 CATCTATCCA TGAAACCCAT GGGAATACCT GACCTAGGTA TGATAAATGA
951 TGGCACCTCC TGTGGAGAAG GCCGGGTATG TTTTAAAAAA AATTGCGTCA
1001 ATAGCTCAGT CCTGCAGTTT GACTGTTTGC CTGAGAAATG CAATACCCGG
1051 GGTGTTTGCA ACAACAGAAA AAACTGCCAC TGCATGTATG GGTGGGCACC
1101 TCCATTCTGT GAGGAAGTGG GGTATGGAGG AAGCATTGAC AGTGGGCCTC
1151 CAGGACTGCT CAGAGGGGCG ATTCCCTCGT CAATTTGGGT TGTGTCCATC
1201 ATAATGTTTC GCCTTATTTT ATTAATCCTT TCAGTGGTTT TTGTGTTTTT
1251 CCGGCAAGTG ATAGGAAACC ACTTAAAACC CAAACAGGAA AAAATGCCAC
1301 TATCCAAAGC AAAAACTGAA CAGGAAGAAT CTAAAACAAA AACTGTACAG
1351 GAAGAATCTA AAACAAAAAC TGGACAGGAA GAATCTGAAG CAAAAACTGG
1401 ACAGGAAGAA TCTAAAGCAA AAACTGGACA GGAAGAATCT AAAGCAAACA
1451 TTGAAAGTAA ACGACCCAAA GCAAAGAGTG TCAAGAAACA AAAAAAGTAA
(SEQ ID NO: 3)
**Name: SVPH-1a** 
1 ATGAAGATGT TACTCCTGCT GCATTGCCTT GGGGTGTTTC TGTCCTGTTC
51 TGGACACATC CAGGATGAGC ACCCCCAATA TCACAGCCCT CCGGATGTGG
101 TGATTCCTGT GAGGATAACT GGCACCACCA GAGGCATGAC ACCTCCAGGC
151 TGGCTCTCCT ATATCCTGCC CTTTGGAGGC CAGAAACACA TTATCCACAT
201 AAAGGTCAAG AAGCTTTTGT TTTCCAAACA CCTCCCTGTG TTCACCTACA
251 CAGACCAGGG TGCTATCCTT GAGGACCAGC CATTTGTCCA GAATAACTGC
301 TACTATCATG GTTATGTGGA AGGGGACCCA GAATCCCTGG TTTCCCTCAG
351 TACCTGTTTT GGGGGTTTTC AAGGAATATT ACAGATAAAT GACTTTGCTT
401 ATGAAATCAA GCCCCTAGCA TTTTCTACCA CGTTTGAACA TCTGGTATAC
451 AAGATGGACA GTGAGGAGAA ACAATTTTCA ACCATGAGAT CCGGATTTAT
501 GCAAAATGAA ATAACATGCC GAATGGAATT TGAAGAAATT GATAATTCCA
551 CTCAGAAGCA AAGTTCTTAT GTGGGCTGGT GGATCCATTT TAGGATTGTT
601 GAAATTGTAG TCGTCATTGA TAATTATCTG TACATTCGTT ATGAAAGGAA
651 CGACTCAAAG TTGCTGGAGG ATCTATATGT TATTGTTAAT ATAGTGGATT
701 CCATTTTGGA TGTCATTGGT GTTAAGGTGT TATTATTTGG TTTGGAGATC
751 TGGACCAATA AAAACCTCAT TGTAGTAGAT GATGTAAGGA AATCTGTGCA
801 CCTGTATTGC AAGTGGAAGT CGGAGAACAT TACGCCCCGG ATGCAACATG
851 ACACCTCACA TCTTTTCACA ACTCTAGGAT TAAGAGGGTT AAGTGGCATA
901 GGAGCTTTTA GAGGAATGTG TACACCACAC CGTAGTTGTG CAATTGTTAC
951 TTTCATGAAC AAAACTTTGG GCACTTTTTC AATTGCAGTG GCTCATCATC
1001 TAGGTCATAA TTTGGGCATG AACCATGATG AGGATACATG TCGTTGTTCA
1051 CAACCTAGAT GCATAATGCA TGAAGGCAAC CCACCAATAA CTAAATTTAG
1101 CAATTGTAGT TATGGTGATT TTTGGGAATA TACTGTAGAG AGGACAAAGT
1151 GTTTGCTTGA AACAGTACAC ACAAAGGACA TCTTTAATGT GAAGCGCTGT
1201 GGGAATGGTG TTGTTGAAGA AGGAGAAGAG TGTGACTGTG GACCTTTAAA
1251 GCATTGTGCA AAAGATCCCT GCTGTCTGTC AAATTGCACT CTGACTGATG
1301 GTTCTACTTG TGCTTTTGGG CTTTGTTGCA AAGACTGCAA GTTCCTACCA
1351 TCAGGGAAAG TGTGTAGAAA GGAGGTCAAT GAATGTGATC TTCCAGAGTG
1401 GTGCAATGGT ACTTCCCATA AGTGCCCAGA TGACTTTTAT GTGGAAGATG
1451 GAATTCCCTG TAAGGAGAGG GGCTACTGCT ATGAAAAGAG CTGTCATGAC
1501 CGCAATGAAC AGTGTAGGAG GATTTTTGGT GCAGGCGCAA ATACTGCAAG
1551 TGAGACTTGC TACAAAGAAT TGAACACCTT AGGTGACCGT GTTGGTCACT
1601 GTGGTATCAA AAATGCTACA TATATAAAGT GTAATATCTC AGATGTCCAG
1651 TGTGGAAGAA TTCAGTGTGA GAATGTGACA GAAATTCCCA ATATGAGTGA
1701 TCATACTACT GTGCATTGGG CTCGCTTCAA TGACATAATG TGCTGGAGTA
1751 CTGATTACCA TTTGGGGATG AAGGGACCTG ATATTGGTGA AGTGAAAGAT
1801 GGAACAGAGT GTGGGATAGA TCATATATGC ATCCACAGGC ACTGTGTCCA
1851 TATAACCATC TTGAATAGTA ATTGCTCACC TGCATTTTGT AACAAGAGGG
1901 GCATCTGCAA CAATAAACAT CACTGCCATT GCAATTATCT GTGGGACCCT
1951 CCCAACTGCC TGATAAAAGG CTATGGAGGT AGTGTTGACA GTGGCCCACC
2001 CCCTAAGAGA AAGAAGAAAA AGAAGTTCTG TTATCTGTGT ATATTGTTGC
2051 TTATTGTTTT GTTTATTTTA TTATGTTGTC TTTATCGACT TTGTAAAAAA
2101 AGTAAACCAA TAAAAAAGCA GCAAGATGTT CAAACTCCAT CTGCAAAAGA
2151 AGAGGAAAAA ATTCAGCGTC GACCTCATGA GTTACCTCCC CAGAGTCAAC
2201 CTTGGGTGAT GCCTTCCCAG AGTCAACCTC CTGTGACACC CTCCCAGAGG
2251 CAACCTCAGT TGATGCCTTC CCAGAGTCAA CCTCCTGTGA CGCCCTCCTA
2301 G (SEQ ID NO:7)
**Name: SVPH-1b** 
1 ATGAAGATGT TACTCCTGCT GCATTGCCTT GGGGTGTTTC TGTCCTGTTC
51 TGGACACATC CAGGATGAGC ACCCCCAATA TCACAGCCCT CCGGATGTGG
 
101 TGATTCCTGT GAGGATAACT GGCACCACCA GAGGCATGAC ACCTCCAGGC
151 TGGCTCTCCT ATATCCTGCC CTTTGGAGGC CAGAAACACA TTATCCACAT
201 AAAGGTCAAG AAGCTTTTGT TTTCCAAACA CCTCCCTGTG TTCACCTACA
251 CAGACCAGGG TGCTATCCTT GAGGACCAGC CATTTGTCCA GAATAACTGC
301 TACTATCATG GTTATGTGGA AGGGGACCCA GAATCCCTGG TTTCCCTCAG
351 TACCTGTTTT GGGGGTTTTC AAGGAATATT ACAGATAAAT GACTTTGCTT
401 ATGAAATCAA GCCCCTAGCA TTTTCTACCA CGTTTGAACA TCTGGTATAC
451 AAGATGGACA GTGAGGAGAA ACAATTTTCA ACCATGAGAT CCGGATTTAT
501 GCAAAATGAA ATAACATGCC GAATGGAATT TGAAGAAATT GATAATTCCA
551 CTCAGAAGCA AAGTTCTTAT GTGGGCTGGT GGATCCATTT TAGGATTGTT
601 GAAATTGTAG TCGTCATTGA TAATTATCTG TACATTCGTT ATGAAAGGAA
651 CGACTCAAAG TTGCTGGAGG ATCTATATGT TATTGTTAAT ATAGTGGATT
701 CCATTTTGGA TGTCATTGGT GTTAAGGTGT TATTATTTGG TTTGGAGATC
751 TGGACCAATA AAAACCTCAT TGTAGTAGAT GATGTAAGGA AATCTGTGCA
801 CCTGTATTGC AAGTGGAAGT CGGAGAACAT TACGCCCCGG ATGCAACATG
851 ACACCTCACA TCTTTTCACA ACTCTAGGAT TAAGAGGGTT AAGTGGCATA
901 GGAGCTTTTA GAGGAATGTG TACACCACAC CGTAGTTGTG CAATTGTTAC
951 TTTCATGAAC AAAACTTTGG GCACTTTTTC AATTGCAGTG GCTCATCATC
1001 TAGGTCATAA TTTGGGCATG AACCATGATG AGGATACATG TCGTTGTTCA
1051 CAACCTAGAT GCATAATGCA TGAAGGCAAC CCACCAATAA CTAAATTTAG
1101 CAATTGTAGT TATGGTGATT TTTGGGAATA TACTGTAGAG AGGACAAAGT
1151 GTTTGCTTGA AACAGTACAC ACAAAGGACA TCTTTAATGT GAAGCGCTGT
1201 GGGAATGGTG TTGTTGAAGA AGGAGAAGAG TGTGACTGTG GACCTTTAAA
1251 GCATTGTGCA AAAGATCCCT GCTGTCTGTC AAATTGCACT CTGACTGATG
1301 GTTCTACTTG TGCTTTTGGG CTTTGTTGCA AAGACTGCAA GTTCCTACCA
1351 TCAGGGAAAG TGTGTAGAAA GGAGGTCAAT GAATGTGATC TTCCAGAGTG
1401 GTGCAATGGT ACTTCCCATA AGTGCCCAGA TGACTTTTAT GTGGAAGATG
1451 GAATTCCCTG TAAGGAGAGG GGCTACTGCT ATGAAAAGAG CTGTCATGAC
1501 CGCAATGAAC AGTGTAGGAG GATTTTTGGT GCAGGCGCAA ATACTGCAAG
1551 TGAGACTTGC TACAAAGAAT TGAACACCTT AGGTGACCGT GTTGGTCACT
1601 GTGGTATCAA AAATGCTACA TATATAAAGT GTAATATCTC AGATGTCCAG
1651 TGTGGAAGAA TTCAGTGTGA GAATGTGACA GAAATTCCCA ATATGAGTGA
1701 TCATACTACT GTGCATTGGG CTCGCTTCAA TGACATAATG TGCTGGAGTA
1751 CTGATTACCA TTTGGGGATG AAGGGACCTG ATATTGGTGA AGTGAAAGAT
1801 GGAACAGAGT GTGGGATAGA TCATATATGC ATCCACAGGC ACTGTGTCCA
1851 TATAACCATC TTGAATAGTA ATTGCTCACC TGCATTTTGT AACAAGAGGG
1901 GCATCTGCAA CAATAAACAT CACTGCCATT GCAATTATCT GTGGGACCCT
1951 CCCAACTGCC TGATAAAAGG CTATGGAGGT AGTGTTGACA GTGGTCCACC
2001 CCCTAAGAGA AAGAAGAAAA AGAAGTTCTG TTATCTGTGT ATATTGTTGC
2051 TTATTGTTTT GTTTATTTTA TTATGTTGTC TTTATCGACT TTGTAAAAAA
2101 AGTAAACCAA TAAAAAAGCA GCAAGATGTT CAAACTCCAT CTGCAAAAGA
2151 AGAGGAAAAA ATTCAGCGTC GACCTCATGA GTTACCTCCC CAGAGTCAAC
2201 CTTGGGTGAT GCCTTCCCAG AGTCAACCTC CTGTGACGCC TTCCCAGAGT
2251 CATCCTCAGG TGATGCCTTC CCAGAGTCAA CCTCCTCAAA ATTTATTCCT
2301 GTTCAGCTTC TCAATCAGTG ACTGTGTGCT AAATTTTAGG CTACTGTATC
2351 TTCAGGCCAC CTGA (SEQ ID NO:8)
**Name: SVPH-1c** 
1 ATGAAGATGT TACTCCTGCT GCATTGCCTT GGGGTGTTTC TGTCCTGTTC
51 TGGACACATC CAGGATGAGC ACCCCCAATA TCACAGCCCT CCGGATGTGG
101 TGATTCCTGT GAGGATAACT GGCACCACCA GAGGCATGAC ACCTCCAGGC
151 TGGCTCTCCT ATATCCTGCC CTTTGGAGGC CAGAAACACA TTATCCACAT
201 AAAGGTCAAG AAGCTTTTGT TTTCCAAACA CCTCCCTGTG TTCACCTACA
 
251 CAGACCAGGG TGCTATCCTT GAGGACCAGC CATTTGTCCA GAATAACTGC
301 TACTATCATG GTTATGTGGA AGGGGACCCA GAATCCCTGG TTTCCCTCAG
351 TACCTGTTTT GGGGGTTTTC AAGGAATATT ACAGATAAAT GACTTTGCTT
401 ATGAAATCAA GCCCCTAGCA TTTTCTACCA CGTTTGAACA TCTGGTATAC
451 AAGATGGACA GTGAGGAGAA ACAATTTTCA ACCATGAGAT CCGGATTTAT
501 GCAAAATGAA ATAACATGCC GAATGGAATT TGAAGAAATT GATAATTCCA
551 CTCAGAAGCA AAGTTCTTAT GTGGGCTGGT GGATCCATTT TAGGATTGTT
601 GAAATTGTAG TCGTCATTGA TAATTATCTG TACATTCGTT ATGAAAGGAA
651 CGACTCAAAG TTGCTGGAGG ATCTATATGT TATTGTTAAT ATAGTGGATT
701 CCATTTTGGA TGTCATTGGT GTTAAGGTGT TATTATTTGG TTTGGAGATC
751 TGGACCAATA AAAACCTCAT TGTAGTAGAT GATGTAAGGA AATCTGTGCA
801 CCTGTATTGC AAGTGGAAGT CGGAGAACAT TACGCCCCGG ATGCAACATG
851 ACACCTCACA TCTTTTCACA ACTCTAGGAT TAAGAGGGTT AAGTGGCATA
901 GGAGCTTTTA GAGGAATGTG TACACCACAC CGTAGTTGTG CAATTGTTAC
951 TTTCATGAAC AAAACTTTGG GCACTTTTTC AATTGCAGTG GCTCATCATC
1001 TAGGTCATAA TTTGGGCATG AACCATGATG AGGATACATG TCGTTGTTCA
1051 CAACCTAGAT GCATAATGCA TGAAGGCAAC CCACCAATAA CTAAATTTAG
1101 CAATTGTAGT TATGGTGATT TTTGGGAATA TACTGTAGAG AGGACAAAGT
1151 GTTTGCTTGA AACAGTACAC ACAAAGGACA TCTTTAATGT GAAGCGCTGT
1201 GGGAATGGTG TTGTTGAAGA AGGAGAAGAG TGTGACTGTG GACCTTTAAA
1251 GCATTGTGCA AAAGATCCCT GCTGTCTGTC AAATTGCACT CTGACTGATG
1301 GTTCTACTTG TGCTTTTGGG CTTTGTTGCA AAGACTGCAA GTTCCTACCA
1351 TCAGGGAAAG TGTGTAGAAA GGAGGTCAAT GAATGTGATC TTCCAGAGTG
1401 GTGCAATGGT ACTTCCCATA AGTGCCCAGA TGACTTTTAT GTGGAAGATG
1451 GAATTCCCTG TAAGGAGAGG GGCTACTGCT ATGAAAAGAG CTGTCATGAC
1501 CGCAATGAAC AGTGTAGGAG GATTTTTGGT GCAGGCGCAA ATACTGCAAG
1551 TGAGACTTGC TACAAAGAAT TGAACACCTT AGGTGACCGT GTTGGTCACT
1601 GTGGTATCAA AAATGCTACA TATATAAAGT GTAATATCTC AGATGTCCAG
1651 TGTGGAAGAA TTCAGTGTGA GAATGTGACA GAAATTCCCA ATATGAGTGA
1701 TCATACTACT GTGCATTGGG CTCGCTTCAA TGACATAATG TGCTGGAGTA
1751 CTGATTACCA TTTGGGGATG AAGGGACCTG ATATTGGTGA AGTGAAAGAT
1801 GGAACAGAGT GTGGGATAGA TCATATATGC ATCCACAGGC ACTGTGTCCA
1851 TATAACCATC TTGAATAGTA ATTGCTCACC TGCATTTTGT AACAAGAGGG
1901 GCATCTGCAA CAATAAACAT CACTGCCATT GCAATTATCT GTGGGACCCT
1951 CCCAACTGCC TGATAAAAGG CTATGGAGGT AGTGTTGACA GTGGCCCACC
2001 CCCTAAGAGA AAGAAGAAAA AGAAGTTCTG TTATCTGTGT ATATTGTTGC
2051 TTATTGTTTT GTTTATTTTA TTATGTTGTC TTTATCGACT TTGTAAAAAA
2101 AGTAAACCAA TAAAAAAGCA GCAAGATGTT CAAACTCCAT CTGCAAAAGA
2151 AGAGGAAAAA ATTCAGCGTC GACCTCATGA GTTACCTCCC CAGAGTCAAC
2201 CTTGGGTGAT GCCTTCCCAG AGTCAACCTC CTGTGACGCC TTCCCAGAGT
2251 CATCCTCGGG TGATGCCTTC TCAGAGTCAA CCTCCTGTGA TGCCTTCCCA
2301 GAGTCATCCT CAGTTGACGC CTTCCCAGAG TCAACCTCCT GTGATGCCTT
2351 CCCAGAGTCA TCCTCAGTTG ACGCCTTCCC AGAGTCAACC TCCTGTGACA
2401 CCCTCCCAGA GGCAACCTCA GTTGATGCCT TCCCAGAGTC AACCTCCTGT
2451 GACGCCCTCC TAG (SEQ ID NO:9)
**Name: SVPH-4a** 
1 ATGAGGTCAG TGCAGATCTT CCTCTCCCAA TGCCGTTTGC TCCTTCTACT
51 AGTTCCGACA ATGCTCCTTA AGTCTCTTGG CGAAGATGTA ATTTTTCACC
101 CTGAAGGGGA GTTTGACTCG TATGAAGTCA CCATTCCTGA GAAGCTGAGC
151 TTCCGGGGAG AGGTGCAGGG TGTGGTCAGT CCCGTGTCCT ACCTACTGCA
201 GTTAAAAGGC AAGAAGCACG TCCTCCATTT GTGGCCCAAG AGACTTCTGT
251 TGCCCCGACA TCTGCGCGTT TTCTCCTTCA CAGAACATGG GGAACTGCTG
 
301 GAGGATCATC CTTACATACC AAAGGACTGC AACTACATGG GCTCCGTGAA
351 AGAGTCTCTG GACTCTAAAG CTACTATAAG CACATGCATG GGGGGTCTCC
401 GAGGTGTATT TAACATTGAT GCCAAACATT ACCAAATTGA GCCCCTCAAG
451 GCCTCTCCCA GTTTTGAACA TGTCGTCTAT CTCCTGAAGA AAGAGCAGTT
501 TGGGAATCAG GTTTGTGGCT TAAGTGATGA TGAAATAGAA TGGCAGATGG
551 CCCCTTATGA GAATAAGGCG AGGCTAAGGG ACTTTCCTGG ATCCTATAAA
601 CACCCAAAGT ACTTGGAATT GATCCTACTC TTTGATCAAA GTAGGTATAG
651 GTTTGTGAAC AACAATCTTT CTCAAGTCAT ACATGATGCC ATTCTTTTGA
701 CTGGGATTAT GGACACCTAC TTTCAAGATG TTCGTATGAG GATACACTTA
751 AAGGCTCTTG AAGTATGGAC AGATTTTAAC AAAATACGCG TTGGATATCC
801 AGAGTTAGCT GAAGTTTTAG GCAGATTTGT AATATATAAA AAAAGTGTAT
851 TAAATGCTCG CCTGTCATCA GATTGGGCAC ATTTATATCT TCAAAGAAAA
901 TATAATGATG CTCTTGCATG GTCGTTTGGA AAAGTGTGTT CTCTAGAATA
951 TGCTGGATCA GTGAGTACTT TACTAGATAC AAATATCCTT GCCCCTGCTA
1001 CCTGGTCTGC TCATGAGCTG GGTCATGCTG TAGGAATGTC ACATGATGAA
1051 CAATACTGCC AATGTAGGGG TAGGCCTAAT TGCATCATGG GCTCAGGACG
1101 CACTGGGTTT AGCAATTGCA GTTATATCTC TTTTTTTAAA CATATCTCTT
1151 CGGGAGCAAC ATGTCTAAAT AATATCCCAG GACTAGGTTA TGTGCTTAAG
1201 AGATGTGGAA ACAAAATTGT GGAGGACAAT GAGGAATGTG ATTGTGGTTC
1251 CACAGAGGAG TGTCAGAAAG ATCGGTGTTG CCAATCAAAT TGTAAGTTGC
1301 AACCAGGTGC CAACTGTAGC ATTGGACTTT GCTGTCATGA TTGTCGGTTT
1351 CGTCCATCTG GATACGTGTG TAGGCAGGAA GGAAATGAAT GTGACCTTGC
1401 AGAGTACTGC GACGGGAATT CAAGTTCCTG CCCAAATGAC GTTTATAAGC
1451 AGGATGGAAC CCCTTGCAAG TATGAAGGCC GTTGTTTCAG GAAGGGGTGC
1501 AGATCCAGAT ATATGCAGTG CCAAAGCATT TTTGGACCTG ATGCCATGGA
1551 GGCTCCTAGT GAGTGCTATG ATGCAGTTAA CTTAATAGGT GATCAATTTG
1601 GTAACTGTGA GATTACAGGA ATTCGAAATT TTAAAAAGTG TGAAAGTGCA
1651 AATTCAATAT GTGGCAGGCT ACAGTGTATA AATGTTGAAA CCATCCCTGA
1701 TTTGCCAGAG CATACGACTA TAATTTCTAC TCATTTACAG GCAGAAAATC
1751 TCATGTGCTG GGGCACAGGC TATCATCTAT CCATGAAACC CATGGGAATA
1801 CCTGACCTAG GTATGATAAA TGATGGCACC TCCTGTGGAG AAGGCCGGGT
1851 ATGTTTTAAA AAAAATTGCG TCAATAGCTC AGTCCTGCAG TTTGACTGTT
1901 TGCCTGAGAA ATGCAATACC CGGGGTGTTT GCAACAACAG AAAAAACTGC
1951 CACTGCATGT ATGGGTGGGC ACCTCCATTC TGTGAGGAAG TGGGGTATGG
2001 AGGAAGCATT GACAGTGGGC CTCCAGGACT GCTCAGAGGG GCGATTCCCT
2051 CGTCAATTTG GGTTGTGTCC ATCATAATGT TTCGCCTTAT TTTATTAATC
2101 CTTTCAGTGG TTTTTGTGTT TTTCCGGCAA GTGATAGGAA ACCACTTAAA
2151 ACCCAAACAG GAAAAAATGC CACTATCCAA AGCAAAAACT GAACAGGAAG
2201 AATCTAAAAC AAAAACTGTA CAGGAAGAAT CTAAAACAAA AACTGGACAG
2251 GAAGAATCTG AAGCAAAAAC TGGACAGGAA GAATCTAAAG CAAAAACTGG
2301 ACAGGAAGAA TCTAAAGCAA ACATTGAAAG TAAACGACCC AAAGCAAAGA
2351 GTGTCAAGAA ACAAAAAAAG TAA (SEQ ID NO:10)
**Name: SVPH-4b**

1 ATGAGGTCAG TGCAGATCTT CCTCTCCCAA TGCCGTTTGC TCCTTCTACT
51 AGTTCCGACA ATGCTCCTTA AGTCTCTTGG CGAAGATGTA ATTTTCACC
101 CTGAAGGGGA GTTTGACTCG TATGAAGTCA CCATTCCTGA GAAGCTGAGC
151 TTCCGGGGAG AGGTGCAGGG TGTGGTCAGT CCCGTGTCCT ACCTACTGCA
201 GTTAAAAGGC AAGAAGCACG TCCTCCATTT GTGGCCCAAG AGACTTCTGT
251 TGCCCCGACA TCTGCGCGTT TTCTCCTTCA CAGAACATGG GGAACTGCTG
301 GAGGATCATC CTTACATACC AAAGGACTGC AACTACATGG GCTCCGTGAA
351 AGAGTCTCTG GACTCTAAAG CTACTATAAG CACATGCATG GGGGGTCTCC
401 GAGGTGTATT TAACATTGAT GCCAAACATT ACCAAATTGA GCCCCTCAAG

451 GCCTCTCCCA GTTTTGAACA TGTCGTCTAT CTCCTGAAGA AAGAGCAGTT
501 TGGGAATCAG GTTTGTGGCT TAAGTGATGA TGAAATAGAA TGGCAGATGG
551 CCCCTTATGA GAATAAGGCG AGGCTAAGGG ACTTTCCTGG ATCCTATAAA
601 CACCCAAAGT ACTTGGAATT GATCCTACTC TTTGATCAAA GTAGGTATAG
651 GTTTGTGAAC AACAATCTTT CTCAAGTCAT ACATGATGCC ATTCTTTTGA
701 CTGGGATTAT GGACACCTAC TTTCAAGATG TTCGTATGAG GATACACTTA
751 AAGGCTCTTG AAGTATGGAC AGATTTTAAC AAAATACGCG TTGGATATCC
801 AGAGTTAGCT GAAGTTTTAG GCAGATTTGT AATATATAAA AAAAGTGTAT
851 TAAATGCTCG CCTGTCATCA GATTGGGCAC ATTTATATCT TCAAAGAAAA
901 TATAATGATG CTCTTGCATG GTCGTTTGGA AAAGTGTGTT CTCTAGAATA
951 TGCTGGATCA GTGAGTACTT TACTAGATAC AAATATCCTT GCCCCTGCTA
1001 CCTGGCCTGC TCATGAGCTG GGTCATGCTG TAGGAATGTC ACATGATGAA
1051 CAATACTGCC AATGTAGGGG TAGGCTTAAT TGCATCATGG GCTCAGGACG
1101 CACTGGGTTT AGCAATTGCA GTTATATCTC TTTTTTTAAA CATATCTCTT
1151 CGGGAGCAAC ATGTCTAAAT AATATCCCAG GACTAGGTTA TGTGCTTAAG
1201 AGATGTGGAA ACAAAATTGT GGAGGACAAT GAGGAATGTG ACTGTGGTTC
1251 CACAGAGGAG TGTCAGAAAG ATCGGTGTTG CCAATCAAAT TGTAAGTTGC
1301 AACCAGGTGC CAACTGTAGC ATTGGACTTT GCTGTCATGA TTGTCGGTTT
1351 CGTCCATCTG GATACGTGTG TAGGCAGGAA GGAAATGAAT GTGACCTTGC
1401 AGAGTACTGC GACGGGAATT CAAGTTCCTG CCCAAATGAC GTTTATAAGC
1451 AGGATGGAAC CCCTTGCAAG TATGAAGGCC GTTGTTTCAG GAAGGGGTGC
1501 AGATCCAGAT ATATGCAGTG CCAAAGCATT TTTGGACCTG ATGCCATGGA
1551 GGCTCCTAGT GAGTGCTATG ATGCAGTTAA CTTAATAGGT GATCAATTTG
1601 GTAACTGTGA GATTACAGGA ATTCGAAATT TTAAAAAGTG TGAAAGTGCA
1651 AATTCAATAT GTGGCAGGCT ACAGTGTATA AATGTTGAAA CCATCCCTGA
1701 TTTGCCAGAG CATACGACTA TAATTTCTAC TCATTTACAG GCAGAAAATC
1751 TCATGTGCTG GGGCACAGGC TATCATCTAT CCATGAAACC CATGGGAATA
1801 CCTGACCTAG GTATGATAAA TGATGGCACC TCCTGTGGAG AAGGCCGGGT
1851 ATGTTTTAAA AAAAATTGCG TCAATAGCTC AGTCCTGCAG TTTGACTGTT
1901 TGCCTGAGAA ATGCAATACC CGGGGTGTTT GCAACAACAG AAAAAACTGC
1951 CACTGCATGT ATGGGTGGGC ACCTCCATTC TGTGAGGAAG TGGGGTATGG
2001 AGGAAGCATT GACAGTGGGC CTCCAGGACT GCTCAGAGGG GCGATTCCCT
2051 CGTCAATTTG GGTTGTGTCC ATCATAATGT TTCGCCTTAT TTTATTAATC
2101 CTTTCAGTGG TTTTTGTGTT TTTCCGGCAA GTGATAGGAA ACCACTTAAA
2151 ACCCAAACAG GAAAAAATGC CACTATCCAA AGCAAAAACT GAACAGGAAG
2201 AATCTAAAAC AAAAACTGTA CAGGAAGAAT CTAAAACAAA AACTGGACAG
2251 GAAGAATCTG AAGCAAAAAC TGGACAGGAA GAATCTAAAG CAAACATTGA
2301 AAGTAAACGA CCCAAAGCAA AGAGTGTCAA GAAACAAAAA AAGTAA
(SEQ ID NO:11)

The amino acid sequences of the polypeptides encoded by the nucleotide sequence of the invention includes:
**Name: SVPH-1 (polypeptide)**
   1 MTAXEQCRRI FGAGANTASE TCYKELNTLG DRVGHCGIKN (SEQ ID NO:4)
**Name: SVPH-3 (polypeptide)** 
1 EDWVYYRISH EEKDLFFNLT VNEGFLSNSY IMEKRYGNLS HVKMMASSAP

51 LCHLSGTVLQ QGTRVGTAAL SACHGLTGFF QLPHGDFFIE PVKKHPLVEG

101 GYHPHIVYRR QKVPETKEPT CGL (SEQ ID NO:5)
**Name: SVPH-4 (polypeptide)** 
1 HEDLYLQRKY NDALAWSFGK VCSLEYAGSV STLLDTNILA PATWSAHELG
51 HAVGMSHDEQ YCQCRGRPNC IMGSGRTGFS NCSYISFFKH ISSGATCLNN
101 IPGLGYVLKR CGNKIVEDNE ECDCGSTEEC QKDRCCQSNC KLQPGANCSI
151 GLCCHDCRFR PSGYVCRQEG NECDLAEYCD GNSSSCPNDV YKQDGTPCKY
201 EGRCFRKGCR SRYMQCQSIF GPDAMEAPSE CYDAVNLIGD QFGNCEITGI
251 RNFKKCESAN SICGRLQCIN VETIPDLPEH TTIISTHLQA ENLMCWGTGY
301 HLSMKPMGIP DLGMINDGTS CGEGRVCFKK NCVNSSVLQF DCLPEKCNTR
351 GVCNNRKNCH CMYGWAPPFC EEVGYGGSID SGPPGLLRGA IPSSIWVVSI
401 IMFRLILLIL SVVFVFFRQV IGNHLKPKQE KMPLSKAKTE QEESKTKTVQ
451 EESKTKTGQE ESEAKTGQEE SKAKTGQEES KANIESKRPK AKSVKKQKK*
(SEQ ID NO:6)
**Name: SVPH-1a (polypeptide)** 
1 MKMLLLLHCL GVFLSCSGHI QDEHPQYHSP PDVVIPVRIT GTTRGMTPPG
51 WLSYILPFGG QKHIIHIKVK KLLFSKHLPV FTYTDQGAIL EDQPFVQNNC
101 YYHGYVEGDP ESLVSLSTCF GGFQGILQIN DFAYEIKPLA FSTTFEHLVY
151 KMDSEEKQFS TMRSGFMQNE ITCRMEFEEI DNSTQKQSSY VGWWIHFRIV
201 EIVVVIDNYL YIRYERNDSK LLEDLYVIVN IVDSILDVIG VKVLLFGLEI
251 WTNKNLIVVD DVRKSVHLYC KWKSENITPR MQHDTSHLFT TLGLRGLSGI
301 GAFRGMCTPH RSCAIVTFMN KTLGTFSIAV AHHLGHNLGM NHDEDTCRCS
351 QPRCIMHEGN PPITKFSNCS YGDFWEYTVE RTKCLLETVH TKDIFNVKRC
401 GNGVVEEGEE CDCGPLKHCA KDPCCLSNCT LTDGSTCAFG LCCKDCKFLP
451 SGKVCRKEVN ECDLPEWCNG TSHKCPDDFY VEDGIPCKER GYCYEKSCHD
501 RNEQCRRIFG AGANTASETC YKELNTLGDR VGHCGIKNAT YIKCNISDVQ
551 CGRIQCENVT EIPNMSDHTT VHWARFNDIM CWSTDYHLGM KGPDIGEVKD
601 GTECGIDHIC IHRHCVHITI LNSNCSPAFC NKRGICNNKH HCHCNYLWDP
651 PNCLIKGYGG SVDSGPPPKR KKKKKFCYLC ILLLIVLFIL LCCLYRLCKK
701 SKPIKKQQDV QTPSAKEEEK IQRRPHELPP QSQPWVMPSQ SQPPVTPSQR
751 QPQLMPSQSQ PPVTPS* (SEQ ID NO:12)
**Name: SVPH-1b (polypeptide)** 
1 MKMLLLLHCL GVFLSCSGHI QDEHPQYHSP PDVVIPVRIT GTTRGMTPPG
51 WLSYILPFGG QKHIIHIKVK KLLFSKHLPV FTYTDQGAIL EDQPFVQNNC
101 YYHGYVEGDP ESLVSLSTCF GGFQGILQIN DFAYEIKPLA FSTTFEHLVY
151 KMDSEEKQFS TMRSGFMQNE ITCRMEFEEI DNSTQKQSSY VGWWIHFRIV
201 EIVVVIDNYL YIRYERNDSK LLEDLYVIVN IVDSILDVIG VKVLLFGLEI
251 WTNKNLIVVD DVRKSVHLYC KWKSENITPR MQHDTSHLFT TLGLRGLSGI
301 GAFRGMCTPH RSCAIVTFMN KTLGTFSIAV AHHLGHNLGM NHDEDTCRCS
351 QPRCIMHEGN PPITKFSNCS YGDFWEYTVE RTKCLLETVH TKDIFNVKRC
401 GNGVVEEGEE CDCGPLKHCA KDPCCLSNCT LTDGSTCAFG LCCKDCKFLP
451 SGKVCRKEVN ECDLPEWCNG TSHKCPDDFY VEDGIPCKER GYCYEKSCHD
501 RNEQCRRIFG AGANTASETC YKELNTLGDR VGHCGIKNAT YIKCNISDVQ
 
551 CGRIQCENVT EIPNMSDHTT VHWARFNDIM CWSTDYHLGM KGPDIGEVKD
601 GTECGIDHIC IHRHCVHITI LNSNCSPAFC NKRGICNNKH HCHCNYLWDP
651 PNCLIKGYGG SVDSGPPPKR KKKKKFCYLC ILLLIVLFIL LCCLYRLCKK
701 SKPIKKQQDV QTPSAKEEEK IQRRPHELPP QSQPWVMPSQ SQPPVTPSQS
751 HPQVMPSQSQ PPQNLFLFSF SISDCVLNFR LLYLQAT* (SEQ ID NO:13)
**Name: SVPH-1c (polypeptide)** 
1 MKMLLLLHCL GVFLSCSGHI QDEHPQYHSP PDVVIPVRIT GTTRGMTPPG
51 WLSYILPFGG QKHIIHIKVK KLLFSKHLPV FTYTDQGAIL EDQPFVQNNC
101 YYHGYVEGDP ESLVSLSTCF GGFQGILQIN DFAYEIKPLA FSTTFEHLVY
151 KMDSEEKQFS TMRSGFMQNE ITCRMEFEEI DNSTQKQSSY VGWWIHFRIV
201 EIVVVIDNYL YIRYERNDSK LLEDLYVIVN IVDSILDVIG VKVLLFGLEI
251 WTNKNLIVVD DVRKSVHLYC KWKSENITPR MQHDTSHLFT TLGLRGLSGI
301 GAFRGMCTPH RSCAIVTFMN KTLGTFSIAV AHHLGHNLGM NHDEDTCRCS
351 QPRCIMHEGN PPITKFSNCS YGDFWEYTVE RTKCLLETVH TKDIFNVKRC
401 GNGVVEEGEE CDCGPLKHCA KDPCCLSNCT LTDGSTCAFG LCCKDCKFLP
451 SGKVCRKEVN ECDLPEWCNG TSHKCPDDFY VEDGIPCKER GYCYEKSCHD
501 RNEQCRRIFG AGANTASETC YKELNTLGDR VGHCGIKNAT YIKCNISDVQ
551 CGRIQCENVT EIPNMSDHTT VHWARFNDIM CWSTDYHLGM KGPDIGEVKD
601 GTECGIDHIC IHRHCVHITI LNSNCSPAFC NKRGICNNKH HCHCNYLWDP
651 PNCLIKGYGG SVDSGPPPKR KKKKKFCYLC ILLLIVLFIL LCCLYRLCKK
701 SKPIKKQQDV QTPSAKEEEK IQRRPHELPP QSQPWVMPSQ SQPPVTPSQS
751 HPRVMPSQSQ PPVMPSQSHP QLTPSQSQPP VMPSQSHPQL TPSQSQPPVT
801 PSQRQPQLMP SQSQPPVTPS * (SEQ ID NO:14)
**Name: SVPH-4a (polypeptide)** 
1 
51 
101 
151 
201 
251 
301 
351 
401 
451 
501 
551 
601 
651 
701 
751  *
(SEQ ID NO:15)
**Name: SVPH-4b (polypeptide)**

1 MRSVQIFLSQ CRLLLLLVPT MLLKSLGEDV IFHPEGEFDS YEVTIPEKLS
51 FRGEVQGVVS PVSYLLQLKG KKHVLHLWPK RLLLPRHLRV FSFTEHGELL
101 EDHPYIPKDC NYMGSVKESL DSKATISTCM GGLRGVFNID AKHYQIEPLK
151 ASPSFEHVVY LLKKEQFGNQ VCGLSDDEIE WQMAPYENKA RLRDFPGSYK
201 HPKYLELILL FDQSRYRFVN NNLSQVIHDA ILLTGIMDTY FQDVRMRIHL

251 KALEVWTDFN KIRVGYPELA EVLGRFVIYK KSVLNARLSS DWAHLYLQRK
301 YNDALAWSFG KVCSLEYAGS VSTLLDTNIL APATWPAHEL GHAVGMSHDE
351 QYCQCRGRLN CIMGSGRTGF SNCSYISFFK HISSGATCLN NIPGLGYVLK
401 RCGNKIVEDN EECDCGSTEE CQKDRCCQSN CKLQPGANCS IGLCCHDCRF
451 RPSGYVCRQE GNECDLAEYC DGNSSSCPND VYKQDGTPCK YEGRCFRKGC
501 RSRYMQCQSI FGPDAMEAPS ECYDAVNLIG DQFGNCEITG IRNFKKCESA
551 NSICGRLQCI NVETIPDLPE HTTIISTHLQ AENLMCWGTG YHLSMKPMGI
601 PDLGMINDGT SCGEGRVCFK KNCVNSSVLQ FDCLPEKCNT RGVCNNRKNC
651 HCMYGWAPPF CEEVGYGGSI DSGPPGLLRG AIPSSIWVVS IIMFRLILLI
701 LSVVFVFFRQ VIGNHLKPKQ EKMPLSKAKT EQEESKTKTV QEESKTKTGQ
751 EESEAKTGQE ESKANIESKR PKAKSVKKQK K* (SEQ ID NO:16)

The discovery of the nucleic acids of the invention enables the construction of expression vectors comprising nucleic acid sequences encoding polypeptides; host cells transfected or transformed with the expression vectors; isolated and purified biologically active polypeptides and fragments thereof; the use of the nucleic acids or oligonucleotides thereof as probes to identify nucleic acid encoding proteins having metalloproteinase-disintegrin activity; the use of the nucleic acids or oligonucleotides thereof to identify human chromosome number 1 or 4; the use of the nucleic acids or oligonucleotides thereof to map genes on human chromosome number 1 or 4; the use of the nucleic acid or oligonucleotides thereof to identify genes associated with certain diseases, syndromes or other human conditions associated with human chromosome number 1 or 4, including fetal hydantoin syndrome, diphenylhydantoin toxicity, and pheochromocytoma; the use of single-stranded sense or antisense oligonucleotides from the nucleic acids to inhibit expression of polynucleotide encoded by the SVPH-1, SVPH-3, or SVPH-4 gene; the use of such polypeptides and soluble fragments to function as a proteinase; the use of such polypeptides and fragmented peptides as molecular weight markers; the use of such polypeptides and fragmented peptides as controls for peptide fragmentation, and kits comprising these reagents; the use of such polypeptides and fragments thereof to generate antibodies; and the use of antibodies to purify SVPH polypeptides.

### NUCLEIC ACID MOLECULES

In a particular embodiment, the invention relates to certain isolated nucleotide sequences that are free from contaminating endogenous material. A "nucleotide sequence" refers to a polynucleotide molecule in the form of a separate fragment or as a component of a larger nucleic acid construct. The nucleic acid molecule has been derived from DNA or RNA isolated at least once in substantially pure form and in a quantity or concentration enabling identification, manipulation, and recovery of its component nucleotide sequences by standard biochemical methods, such as those outlined in Sambrook et al., Molecular Cloning: A Laboratory Manual, 2nd ed., Cold Spring Harbor Laboratory, Cold Spring Harbor, NY (1989). Such sequences are preferably provided and/or constructed in the form of an open reading frame uninterrupted by internal non-translated sequences, or introns, that are typically present in eukaryotic genes. Sequences of non-translated DNA can be present 5' or 3' from an open reading frame, where the same do not interfere with manipulation or expression of the coding region.

Nucleic acid molecules of the invention include DNA in both single-stranded and double-stranded form, as well as the RNA complement thereof. DNA includes, for example, cDNA, genomic DNA, chemically synthesized DNA, DNA amplified by PCR, and combinations thereof. Genomic DNA may be isolated by conventional techniques, *e.g.*, using the cDNA of SEQ ID NOs:1-3 and 7-11, or a suitable fragment thereof, as a probe.

The DNA molecules of the invention include full length genes as well as polynucleotides and fragments thereof. The full length gene may include the N-terminal signal peptide. Other embodiments include DNA encoding a soluble form, *e.g*., encoding the extracellular domain of the protein, either with or without the signal peptide.

The nucleic acids of the invention are preferentially derived from human sources, but the invention includes those derived from non-human species, as well.

### Preferred Sequences

Particularly preferred nucleotide sequences of the invention are SEQ ID NOs:1-3 and 7-11, as set forth above. The sequences of amino acids encoded by the DNA of SEQ ID NOs:1-3 and 7-11 are shown in SEQ ID NOs:4-6 and 12-16, respectively. In SEQ ID NO:1 "N" can represent any nucleotide. These sequences identify the SVPH polynucleotides as members of the metalloproteinase-disintegrin family. As noted above, proteins of this family are characterized by a pro-domain, a disintegrin domain, a metalloproteinase domain, a cysteine rich region, a transmembrane domain, and a cytoplasmic tail.

In particular, SVPH- (originally isolated from human testis) and SVPH-4 (originally isolated from human testis, fetal lung, and B-cells) both share homology to the cysteine rich region of the metalloproteinase-disintegrin family, and SVPH-3 (originally isolated from human fetus tissue) shares homology to the pro-domain of these family members. In addition, SVPH-4 polypeptide (SEQ ID NO:3) encodes a zinc binding motif (His 47 to Asp 58), a disintegrin domain (Leu 104 to Cys 179), and a cysteine rich region (Asp 180 to Arg 388).

SVPH-1a, SVPH-1b, and SVPH-1c represent the nucleotide sequences (SEQ ID NOs:7-9) of three alternatively spliced SVPH-1 clones with divergent cytoplasmic domains. These clones were isolated by screening a human testis library (Clonetech cat no. HL3024a) at 42°C and washing at 42°C in 2x SSC using four different oligonucleotides:
CACCTAAGGTGTTCAATTCTTTG (SEQ ID NO:17),
CAAATACTGCAAGTGAGACTTGC (SEQ ID NO:18),
TGCACAACTACGTGTGGTGTACCC (SEQ ID NO:19), and
GAGCCACTGCAATTGAAAAAGTGCCC (SEQ ID NO:20).

SVPH-4a and SVPH-4b represent the nucleotide sequences (SEQ ID NOs:10-11) of two alternatively spliced SVPH-4 clones with divergent cytoplasmic domains. These clones were isolated by screening a human testis library (Clonetech cat no. HL3024a) at 42°C and washing at 42°C in 2x SSC using three different oligonucleotides:
AATGATGCTCTTGCATGGTCG (SEQ ID NO:21),
CTTTCACGGAGCCCATGTAGTTGCAG (SEQ ID NO:22), and
TGAAGGAGAAAACGCGCAGATGTCGG (SEQ ID NO:23).

### Additional Sequences

Due to the known degeneracy of the genetic code, wherein more than one codon can encode the same amino acid, a DNA sequence can vary from that shown in SEQ ID NOs:1-3 and 7-11, and still encode a polypeptide having the amino acid sequence of SEQ ID NOs:4-6 and 12-16, respectively. Such variant DNA sequences can result from silent mutations (*e.g.*, occurring during PCR amplification), or can be the product of deliberate mutagenesis of a native sequence.

The invention thus provides isolated DNA sequences encoding polypeptides of the invention, selected from: (a) DNA comprising the nucleotide sequence of SEQ ID NOs:1-3 and 7-11; (b) DNA encoding the polypeptides of SEQ ID NOs:4-6 and 12-16; (c) DNA capable of hybridization to a DNA of (a) or (b) under conditions of moderate stringency and which encodes polypeptides of the invention; (d) DNA capable of hybridization to a DNA of (a) or (b) under conditions of high stringency and which encodes polypeptides of the invention, and (e) DNA which is degenerate as a result of the genetic code to a DNA defined in (a), (b), (c), or (d) and which encode polypeptides of the invention. Of course, polypeptides encoded by such DNA sequences are encompassed by the invention.

As used herein, conditions of moderate stringency can be readily determined by those having ordinary skill in the art based on, for example, the length of the DNA. The basic conditions are set forth by Sambrook et al. Molecular- Cloning: A Laboratory Manual, 2nd ed. Vol. 1, pp. 1.101-104, Cold Spring Harbor Laboratory Press, (1989), and include use of a prewashing solution for the nitrocellulose filters 5X SSC, 0.5% SDS, 1.0 mM EDTA (pH 8.0), hybridization conditions of about 50% formamide, 6X SSC at about 42°C (or other similar hybridization solution, such as Stark's solution, in about 50% formamide at about 42°C), and washing conditions of about 60°C, 0.5X SSC, 0.1% SDS. Conditions of high stringency can also be readily determined by the skilled artisan based on, for example, the length of the DNA. Generally, such conditions are defined as hybridization conditions as above, and with washing at approximately 68°C, 0.2X SSC, 0.1% SDS. The skilled artisan will recognize that the temperature and wash solution salt concentration can be adjusted as necessary according to factors such as the length of the probe.

Also included as an embodiment of the invention is DNA encoding polypeptide fragments and polypeptides comprising inactivated N-glycosylation site(s), inactivated protease processing site(s), or conservative amino acid substitution(s), as described below.

In another embodiment, the nucleic acid molecules of the invention also comprise nucleotide sequences that are at least 80% identical to a native sequence. Also contemplated are embodiments in which a nucleic acid molecule comprises a sequence that is at least 90% identical, at least 95% identical, at least 98% identical, at least 99% identical, or at least 99.9% identical to a native sequence.

The percent identity may be determined by visual inspection and mathematical calculation. Alternatively, the percent identity of two nucleic acid sequences can be determined by comparing sequence information using the GAP computer program, version 6.0 described by Devereux et al., Nucl. Acids Res., 12:387 (1984) and available from the University of Wisconsin Genetics Computer Group (UWGCG). The preferred default parameters for the GAP program include: (1) a unary comparison matrix (containing a value of 1 for identities and 0 for non-identities) for nucleotides, and the weighted comparison matrix of Gribskov and Burgess, Nucl. Acids Res. 14:6745 (1986), as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps. Other programs used by one skilled in the art of sequence comparison may also be used.

The invention also provides isolated nucleic acids useful in the production of polypeptides. Such polypeptides may be prepared by any of a number of conventional techniques. A DNA sequence encoding an SVPH polypeptide, or desired fragment thereof may be subcloned into an expression vector for production of the polypeptide or fragment. The DNA sequence advantageously is fused to a sequence encoding a suitable leader or signal peptide. Alternatively, the desired fragment may be chemically synthesized using known techniques. DNA fragments also may be produced by restriction endonuclease digestion of a full length cloned DNA sequence, and isolated by electrophoresis on agarose gels. If necessary, oligonucleotides that reconstruct the 5' or 3' terminus to a desired point may be ligated to a DNA fragment generated by restriction enzyme digestion. Such oligonucleotides may additionally contain a restriction endonuclease cleavage site upstream of the desired coding sequence, and position an initiation codon (ATG) at the N-terminus of the coding sequence.

The well-known polymerase chain reaction (PCR) procedure also may be employed to isolate and amplify a DNA sequence encoding a desired protein fragment. Oligonucleotides that define the desired termini of the DNA fragment are employed as 5' and 3' primers. The oligonucleotides may additionally contain recognition sites for restriction endonucleases, to facilitate insertion of the amplified DNA fragment into an expression vector. PCR techniques are described in Saiki et al., Science 239:487 (1988); Recombinant DNA Methodology, Wu et al., eds., Academic Press, Inc., San Diego, pp. 189-196 (1989); and PCR Protocols: A Guide to Methods and Applications, Innis et al., eds., Academic Press, Inc. (1990).

### POLYPEPTIDES AND FRAGMENTS THEREOF

The invention encompasses polypeptides and fragments thereof in various forms, including those that are naturally occurring or produced through various techniques such as procedures involving recombinant DNA technology. Such forms include, but are not limited to, derivatives, variants, and oligomers, as well as fusion proteins or fragments thereof.

### Polypeptides and Fragments Thereof

The polypeptides of the invention include the proteins encoded by the nucleic acid sequences set forth above. Particularly preferred polypeptides comprise the amino acid sequence of SEQ ID NOs:4-6 and 12-16.

The polypeptides, as set forth in SEQ ID NOs:4 and 6, include a cysteine rich region homologous to the metalloproteinase-disintegrin family, and the polypeptide of SEQ ID NO:5 includes a pro-domain homologous to the same family of proteins. SVPH-1 (SEQ ID NO:4) has an N-terminal region having amino acids Met 1 to Asn 40. In SEQ ID NO:4 "X" can represent any amino acid. SVPH-3 (SEQ ID NO: 5) has an N-terminal region having amino acids Asn 1 to Leu 23. SVPH-4 (SEQ ID NO:6) also includes an extracellular domain comprising amino acids His 1 to Arg 388, a transmembrane region comprising amino acids Gly 389 through Phe 417, and a C-terminal cytoplasmic domain comprising amino acids Arg 418 to Lys 499 and is believed to overlap with EST designated AA 782936.

The SVPH-1a polypeptide (SEQ ID NO: 12), SVPH-1b polypeptide (SEQ ID NO:13), and SVPH-1c polypeptide (SEQ ID NO: 14) each encodes a signal sequence (Met 1 to Ser 15), a pro-domain (Cys 16 to Ser 188), a catalytic domain (Ser 189 to Thr 388), a disintegrin domain (Val 389 to Gly 491), a cysteine rich region (Tyr 492 to Lys 675), and a transmembrane domain (Phe 676 to Cys 698). In addition, each of the SVPH-1a, SVPH-1b, and SVPH-1c polypeptides (SEQ ID NOs:12-14) encodes a cytoplasmic domain. Due to alternative splicing the cytoplasmic domain of each polypeptide is different. For SVPH-1a, SVPH-1b, and SVPH-1c the cytoplasmic domains are (Lys 699 to Ser 766), (Lys 699 to Thr 787), and (Lys 699 to Ser 820), respectively.

Similarly, the SVPH-4a polypeptide (SEQ ID NO:15) and SVPH-4b polypeptide (SEQ ID NO:16) each encodes a signal sequence (Met 1 to Gly 27), a pro-domain (Glu 28 to Arg 193), a catalytic domain (Asp 194 to Ile 392), a disintegrin domain (Pro 393 to Gly 493), a cysteine rich region (Arg 494 to Ser 685), and a transmembrane domain (Ile 686 to Gly 713). In addition, each of the SVPH-4a and SVPH-4b polypeptides (SEQ ID NOs:15-16) encodes a cytoplasmic domain. Due to alternative splicing the cytoplasmic domain of each polypeptide is different. The cytoplasmic domain of SVPH-4a is (Asn 714 to Lys 790), and the cytoplasmic domain of SVPH-4b is (Asn 714 to Lys 781).

The skilled artisan will recognize that the above-described boundaries of such regions of the polypeptide are approximate and that the boundaries of the transmembrane region (which may be predicted by using computer programs available for that purpose) may differ from those described above.

The polypeptides of the invention may be membrane bound or they may be secreted and thus soluble. Soluble polypeptides are capable of being secreted from the cells in which they are expressed. In general, soluble polypeptides may be identified (and distinguished from non-soluble membrane-bound counterparts) by separating intact cells which express the desired polypeptide from the culture medium, *e.g.,* by centrifugation, and assaying the medium (supernatant) for the presence of the desired polypeptide. The presence of polypeptide in the medium indicates that the polypeptide was secreted from the cells and thus is a soluble form of the protein.

In one embodiment, the soluble polypeptides and fragments thereof comprise all or part of the extracellular domain, but lack the transmembrane region that would cause retention of the polypeptide on a cell membrane. A soluble polypeptide may include the cytoplasmic domain, or a portion thereof, as long as the polypeptide is secreted from the cell in which it is produced.

In general, the use of soluble forms is advantageous for certain applications. Purification of the polypeptides from recombinant host cells is facilitated, since the soluble polypeptides are secreted from the cells. Further, soluble polypeptides are generally more suitable for intravenous administration.

The invention also provides polypeptides and fragments of the extracellular domain that retain a desired biological activity. Particular embodiments are directed to polypeptide fragments that retain the ability to bind the "binding partner" or the native cognates, substrates, or counter-structure. Such a fragment may be a soluble polypeptide, as described above. In another embodiment, the polypeptides and fragments advantageously include regions that are conserved in the SVPH family as described above.

Also provided herein are polypeptide fragments comprising at least 20, or at least 30, contiguous amino acids of the sequences of SEQ ID NOs:4-6 and 12-16. Fragments derived from the cytoplasmic domain find use in studies of signal transduction, and in regulating cellular processes associated with transduction of biological signals. Polypeptide fragments also may be employed as immunogens, in generating antibodies.

### Variants

Naturally occurring variants as well as derived variants of the polypeptides and fragments are provided herein.

Variants may exhibit amino acid sequences that are at least 80% identical. Also contemplated are embodiments in which a polypeptide or fragment comprises an amino acid sequence that is at least 90% identical, at least 95% identical, at least 98% identical, at least 99% identical, or at least 99.9% identical to the preferred polypeptide or fragment thereof. Percent identity may be determined by visual inspection and mathematical calculation. Alternatively, the percent identity of two protein sequences can be determined by comparing sequence information using the GAP computer program, based on the algorithm of Needleman and Wunsch, J. Mol. Biol., 48:443 (1970) and available from the University of Wisconsin Genetics Computer Group (UWGCG). The preferred default parameters for the GAP program include: (1) a scoring matrix, blosum62, as described by Henikoff and Henikoff, Proc. Natl. Acad. Sci. USA, 89:10915 (1992); (2) a gap weight of 12; (3) a gap length weight of 4; and (4) no penalty for end gaps. Other programs used by one skilled in the art of sequence comparison may also be used.

The variants of the invention include, for example, those that result from alternate mRNA splicing events or from proteolytic cleavage. Alternate splicing of mRNA may, for example, yield a truncated but biologically active protein, such as a naturally occurring soluble form of the protein. Variations attributable to proteolysis include, for example, differences in the N- or C-termini upon expression in different types of host cells, due to proteolytic removal of one or more terminal amino acids from the protein (generally from 1-5 terminal amino acids). Proteins in which differences in amino acid sequence are attributable to genetic polymorphism (allelic variation among individuals producing the protein) are also contemplated herein.

Additional variants within the scope of the invention include polypeptides that may be modified to create derivatives thereof by forming covalent or aggregative conjugates with other chemical moieties, such as glycosyl groups, lipids, phosphate, acetyl groups and the like. Covalent derivatives may be prepared by linking the chemical moieties to functional groups on amino acid side chains or at the N-terminus or C-terminus of a polypeptide. Conjugates comprising diagnostic (detectable) or therapeutic agents attached thereto are contemplated herein, as discussed in more detail below.

Other derivatives include covalent or aggregative conjugates of the polypeptides with other proteins or polypeptides, such as by synthesis in recombinant culture as N-terminal or C-terminal fusions. Examples of fusion proteins are discussed below in connection with oligomers. Further, fusion proteins can comprise peptides added to facilitate purification and identification. Such peptides include, for example, poly-His or the antigenic identification peptides described in U.S. Patent No. 5,011,912 and in Hopp et al., Bio/Technology 6:1204 (1988). One such peptide is the FLAG® peptide, Asp-Tyr-Lys-Asp-Asp-Asp-Asp-Lys (SEQ ID NO:28), which is highly antigenic and provides an epitope reversibly bound by a specific monoclonal antibody, enabling rapid assay and facile purification of expressed recombinant protein. A murine hybridoma designated 4E I produces a monoclonal antibody that binds the FLAG® peptide in the presence of certain divalent metal cations, as described in U.S. Patent 5,011,912, hereby incorporated by reference. The 4E11 hybridoma cell line has been deposited with the American Type Culture Collection under accession no. HB 9259. Monoclonal antibodies that bind the FLAG® peptide are available from Eastman Kodak Co., Scientific Imaging Systems Division, New Haven, Connecticut.

Among the variant polypeptides provided herein are variants of native polypeptides that retain the native biological activity or the substantial equivalent thereof. One example is a variant that binds with essentially the same binding affinity as does the native form. Binding affinity can be measured by conventional procedures, e.g., as described in U.S. Patent No. 5,512,457 and as set forth below.

Variants include polypeptides that are substantially homologous to the native form, but which have an amino acid sequence different from that of the native form because of one or more deletions, insertions or substitutions. Particular embodiments include, but are not limited to, polypeptides that comprise from one to ten deletions, insertions or substitutions of amino acid residues, when compared to a native sequence.

A given amino acid may be replaced, for example, by a residue having similar physiochemical characteristics. Examples of such conservative substitutions include substitution of one aliphatic residue for another, such as Ile, Val, Leu, or Ala for one another; substitutions of one polar residue for another, such as between Lys and Arg, Glu and Asp, or Gln and Asn; or substitutions of one aromatic residue for another, such as Phe, Trp, or Tyr for one another. Other conservative substitutions, e.g., involving substitutions of entire regions having similar hydrophobicity characteristics, are well known.

Similarly, the DNAs of the invention include variants that differ from a native DNA sequence because of one or more deletions, insertions or substitutions, but that encode a biologically active polypeptide.

The invention further includes polypeptides of the invention with or without associated native-pattern glycosylation. Polypeptides expressed in yeast or mammalian expression systems (*e.g.,* COS-1 or COS-7 cells) can be similar to or significantly different from a native polypeptide in molecular weight and glycosylation pattern, depending upon the choice of expression system. Expression of polypeptides of the invention in bacterial expression systems, such as *E. coli,* provides non-glycosylated molecules. Further, a given preparation may include multiple differentially glycosylated species of the protein. Glycosyl groups can be removed through conventional methods, in particular those utilizing glycopeptidase. In general, glycosylated polypeptides of the invention can be incubated with a molar excess of glycopeptidase (Boehringer Mannheim).

Correspondingly, similar DNA constructs that encode various additions or substitutions of amino acid residues or sequences, or deletions of terminal or internal residues or sequences are encompassed by the invention. For example, N-glycosylation sites in the polypeptide extracellular domain can be modified to preclude glycosylation, allowing expression of a reduced carbohydrate analog in mammalian and yeast expression systems. N-glycosylation sites in eukaryotic polypeptides are characterized by an amino acid triplet Asn-X-Y, wherein X is any amino acid except Pro and Y is Ser or Thr. Appropriate substitutions, additions, or deletions to the nucleotide sequence encoding these triplets will result in prevention of attachment of carbohydrate residues at the Asn side chain. Alteration of a single nucleotide, chosen so that Asn is replaced by a different amino acid, for example, is sufficient to inactivate an N-glycosylation site. Alternatively, the Ser or Thr can by replaced with another amino acid, such as Ala. Known procedures for inactivating N-glycosylation sites in proteins include those described in U.S. Patent 5,071,972 and EP 276,846, hereby incorporated by reference.

In another example of variants, sequences encoding Cys residues that are not essential for biological activity can be altered to cause the Cys residues to be deleted or replaced with other amino acids, preventing formation of incorrect intramolecular disulfide bridges upon folding or renaturation.

Other variants are prepared by modification of adjacent dibasic amino acid residues, to enhance expression in yeast systems in which KEX2 protease activity is present. EP 212,914 discloses the use of site-specific mutagenesis to inactivate KEX2 protease processing sites in a protein. KEX2 protease processing sites are inactivated by deleting, adding or substituting residues to alter Arg-Arg, Arg-Lys, and Lys-Arg pairs to eliminate the occurrence of these adjacent basic residues. Lys-Lys pairings are considerably less susceptible to KEX2 cleavage, and conversion of Arg-Lys or Lys-Arg to Lys-Lys represents a conservative and preferred approach to inactivating KEX2 sites.

### Oligomers

Encompassed by the invention are oligomers or fusion proteins that contain SVPH polypeptides. When the polypeptide of the invention is a type I membrane protein, such as SVPH, the fusion partner is linked to the C terminus of the type I membrane protein. Such oligomers may be in the form of covalently-linked or non-covalently-linked multimers, including dimers, trimers, or higher oligomers. As noted above, preferred polypeptides are soluble and thus these oligomers may comprise soluble polypeptides. In one aspect of the invention, the oligomers maintain the binding ability of the polypeptide components and provide therefor, bivalent, trivalent, etc., binding sites.

One embodiment of the invention is directed to oligomers comprising multiple polypeptides joined via covalent or non-covalent interactions between peptide moieties fused to the polypeptides. Such peptides may be peptide linkers (spacers), or peptides that have the property of promoting oligomerization. Leucine zippers and certain polypeptides derived from antibodies are among the peptides that can promote oligomerization of the polypeptides attached thereto, as described in more detail below.

### Immunoglobulin-based Oligomers

As one alternative, an oligomer is prepared using polypeptides derived from immunoglobulins. Preparation of fusion proteins comprising certain heterologous polypeptides fused to various portions of antibody-derived polypeptides (including the Fc domain) has been described, *e.g.,* by Ashkenazi et al., PNAS USA, 88:10535 (1991); Bym et al., Nature, 344:677 (1990); and Hollenbaugh and Aruffo, "Construction of Immunoglobulin Fusion Proteins", Current Protocols in Immunology, Suppl. 4, pages 10.19.1 - 10.19.11 (1992).

One embodiment of the present invention is directed to a dimer comprising two fusion proteins created by fusing a polypeptide of the invention to an Fc polypeptide derived from an antibody. A gene fusion encoding the polypeptide/Fc fusion protein is inserted into an appropriate expression vector. Polypeptide/Fc fusion proteins are expressed in host cells transformed with the recombinant expression vector, and allowed to assemble much like antibody molecules, whereupon interchain disulfide bonds form between the Fc moieties to yield divalent molecules.

The term "Fc polypeptide" as used herein includes native and mutein forms of polypeptides made up of the Fc region of an antibody comprising any or all of the CH domains of the Fc region. Truncated forms of such polypeptides containing the hinge region that promotes dimerization are also included. Preferred polypeptides comprise an Fc polypeptide derived from a human IgGI antibody.

One suitable Fc polypeptide, described in PCT application WO 93/10151 (hereby incorporated by reference), is a single chain polypeptide extending from the N-terminal hinge region to the native C-terminus of the Fc region of a human IgG 1 antibody. Another useful Fc polypeptide is the Fc mutein described in U.S. Patent 5,457,035 and in Baum et al., EMBO J. 13:3992-4001 (1994), incorporated herein by reference. The amino acid sequence of this mutein is identical to that of the native Fc sequence presented in WO 93/10151, except that amino acid 19 has been changed from Leu to Ala, amino acid 20 has been changed from Leu to Glu, and amino acid 22 has been changed from Gly to Ala. The mutein exhibits reduced affinity for Fc receptors.

The above-described fusion proteins comprising Fc moieties (and oligomers formed therefrom) offer the advantage of facile purification by affinity chromatography over Protein A or Protein G columns.

In other embodiments, the polypeptides of the invention may be substituted for the variable portion of an antibody heavy or light chain. If fusion proteins are made with both heavy and light chains of an antibody, it is possible to form an oligomer with as many as four SVPH extracellular regions.

### Peptide-linker Based Oligomers

Alternatively, the oligomer is a fusion protein comprising multiple polypeptides, with or without peptide linkers (spacer peptides). Among the suitable peptide linkers are those described in U.S. Patents 4,751,180 and 4,935,233, which are hereby incorporated by reference. A DNA sequence encoding a desired peptide linker may be inserted between, and in the same reading frame as, the DNA sequences of the invention, using any suitable conventional technique. For example, a chemically synthesized oligonucleotide encoding the linker may be ligated between the sequences. In particular embodiments, a fusion protein comprises from two to four soluble SVPH polypeptides, separated by peptide linkers.

### Leucine-Zippers

Another method for preparing the oligomers of the invention involves use of a leucine zipper. Leucine zipper domains are peptides that promote oligomerization of the proteins in which they are found. Leucine zippers were originally identified in several DNA-binding proteins (Landschulz et al., Science, 240:1759 (1988)), and have since been found in a variety of different proteins. Among the known leucine zippers are naturally occurring peptides and derivatives thereof that dimerize or trimerize.

The zipper domain (also referred to herein as an oligomerizing, or oligomer-forming, domain) comprises a repetitive heptad repeat, often with four or five leucine residues interspersed with other amino acids. Examples of zipper domains are those found in the yeast transcription factor GCN4 and a heat-stable DNA-binding protein found in rat liver, C/EBP, (Landschulz et al., Science 243:1681 (1989)). Two nuclear transforming proteins, fos and *jun,* also exhibit zipper domains, as does the gene product of the murine proto-oncogene, c-myc (Landschulz et al., Science 240:1759 (1988)). The zipper domains of *fos* and *jun* preferentially form heterodimer (O'Shea et al., Science 245:646 (1989), Turner and Tjian, Science, 243:1689 (1989)). The zipper domain is necessary for biological activity (DNA binding) in these proteins.

The fusogenic proteins of several different viruses, including paramyxovirus, coronavirus, measles virus and many retroviruses, also possess zipper domains (Buckland and Wild, Nature 338:547 (1989); Britton, Nature, 353:394 (1991); Delwart and Mosialos, AIDS Research and Human Retroviruses 6:703 (1990)). The zipper domains in these fusogenic viral proteins are near the transmembrane region of the proteins; it has been suggested that the zipper domains could contribute to the oligomeric structure of the fusogenic proteins. Oligomerization of fusogenic viral proteins is involved in fusion pore formation (Spruce et al., Proc. Natl. Acad. Sci. U.S.A., 88:3523 (1991)). Zipper domains have also been recently reported to play a role in oligomerization of heat-shock transcription factors (Rabindran et al., Science 259:230 (1993)).

Zipper domains fold as short, parallel coiled coils (O'Shea et al., Science 254:539 (1991)). The general architecture of the parallel coiled coil has been well characterized, with a "knobs-into-holes" packing as proposed by Crick, Acta Crystallogr., 6:689 (1953). The dimer formed by a zipper domain is stabilized by the heptad repeat, designated *(abedefg)*_{*n*} according to the notation of McLachlan and Stewart, J. Mol. Biol., 98:293 (1975), in which residues *a* and *d* are generally hydrophobic residues, with d being a leucine, which line up on the same face of a helix. Oppositely-charged residues commonly occur at positions *g* and *e.* Thus, in a parallel coiled coil formed from two helical zipper domains, the "knobs" formed by the hydrophobic side chains of the first helix are packed into the "holes" formed between the side chains of the second helix.

The residues at position d (often leucine) contribute large hydrophobic stabilization energies, and are important for oligomer formation (Krystek: et al., Int. J. Peptide Res. 38:229 (1991)). Lovejoy et al., Science 259:1288 (1993), recently reported the synthesis of a triple-stranded α-helical bundle in which the helices run up-up-down. Their studies confirmed that hydrophobic stabilization energy provides the main driving force for the formation of coiled coils from helical monomers. These studies also indicate that electrostatic interactions contribute to the stoichiometry and geometry of coiled coils. Further discussion of the structure of leucine zippers is found in Harbury et al., Science, 262:1401 (1993).

Examples of leucine zipper domains suitable for producing soluble oligomeric proteins are described in PCT application WO 94/10308, as well as the leucine zipper derived from lung surfactant protein D (SPD) described in Hoppe et al., FEBS Letters, 344:191 (1994), hereby incorporated by reference. The use of a modified leucine zipper that allows for stable trimerization of a heterologous protein fused thereto is described in Fanslow et al., Semin. Immunol., 6:267-278 (1994). Recombinant fusion proteins comprising a soluble polypeptide fused to a leucine zipper peptide are expressed in suitable host cells, and the soluble oligomer that forms is recovered from the culture supernatant.

Certain leucine zipper moieties preferentially form trimers. One example is a leucine zipper derived from lung surfactant protein D (SPD) noted above, as described in Hoppe et al., FEBS Letters, 344:191 (1994) and in U.S. Patent 5,716,805, hereby incorporated by reference in their entirety. This lung SPD-derived leucine zipper peptide comprises the amino acid sequence Pro Asp Val Ala Ser Leu Arg Gin Gin Val Glu Ala Leu Gin Gly Gin Val Gln His Leu Gin Ala Ala Phe Ser GIn Tyr (SEQ ID NO:29).

Another example of a leucine zipper that promotes trimerization is a peptide comprising the amino acid sequence Arg Met Lys Gln Ile Glu Asp Lys Ile Glu Glu Ile Leu Ser Lys Ile Tyr His Ile Glu Asn Glu Ile Ala Arg Ile Lys Lys Leu Ile Gly Glu Arg (SEQ ID NO:30), as described in U.S. Patent 5,716,805. In one alternative embodiment, an N-terminal Asp residue is added; in another, the peptide lacks the N-terminal Arg residue.

Fragments of the foregoing zipper peptides that retain the property of promoting oligomerization may be employed as well. Examples of such fragments include, but are not limited to, peptides lacking one or two of the N-terminal or C-terminal residues presented in the foregoing amino acid sequences. Leucine zippers may be derived from naturally occurring leucine zipper peptides, e.g., *via* conservative substitution(s) in the native amino acid sequence, wherein the peptide's ability to promote oligomerization is retained.

Other peptides derived from naturally occurring trimeric proteins may be employed in preparing trimeric SVPH. Alternatively, synthetic peptides that promote oligomerization may be employed. In particular embodiments, leucine residues in a leucine zipper moiety are replaced by isoleucine residues. Such peptides comprising isoleucine may be referred to as isoleucine zippers, but are encompassed by the term "leucine zippers" as employed herein.

### PRODUCTION OF POLYPEPTIDES AND FRAGMENTS THEREOF

Expression, isolation and purification of the polypeptides and fragments of the invention may be accomplished by any suitable technique, including but not limited to the following:

### Expression Systems

The present invention also provides recombinant cloning and expression vectors containing DNA, as well as host cell containing the recombinant vectors. Expression vectors comprising DNA may be used to prepare the polypeptides or fragments of the invention encoded by the DNA. A method for producing polypeptides comprises culturing host cells transformed with a recombinant expression vector encoding the polypeptide, under conditions that promote expression of the polypeptide, then recovering the expressed polypeptides from the culture. The skilled artisan will recognize that the procedure for purifying the expressed polypeptides will vary according to such factors as the type of host cells employed, and whether the polypeptide is membrane-bound or a soluble form that is secreted from the host cell.

Any suitable expression system may be employed. The vectors include a DNA encoding a polypeptide or fragment of the invention, operably linked to suitable transcriptional or translational regulatory nucleotide sequences, such as those derived from a mammalian, microbial, viral, or insect gene. Examples of regulatory sequences include transcriptional promoters, operators, or enhancers, an mRNA ribosomal binding site, and appropriate sequences which control transcription and translation initiation and termination. Nucleotide sequences are operably linked when the regulatory sequence functionally relates to the DNA sequence. Thus, a promoter nucleotide sequence is operably linked to a DNA sequence if the promoter nucleotide sequence controls the transcription of the DNA sequence. An origin of replication that confers the ability to replicate in the desired host cells, and a selection gene by which transformants are identified, are generally incorporated into the expression vector.

In addition, a sequence encoding an appropriate signal peptide (native or heterologous) can be incorporated into expression vectors. A DNA sequence for a signal peptide (secretory leader) may be fused in frame to the nucleic acid sequence of the invention so that the DNA is initially transcribed, and the mRNA translated, into a fusion protein comprising the signal peptide. A signal peptide that is functional in the intended host cells promotes extracellular secretion of the polypeptide. The signal peptide is cleaved from the polypeptide upon secretion of polypeptide from the cell.

The skilled artisan will also recognize that the position(s) at which the signal peptide is cleaved may differ from that predicted by computer program, and may vary according to such factors as the type of host cells employed in expressing a recombinant polypeptide. A protein preparation may include a mixture of protein molecules having different N-terminal amino acids, resulting from cleavage of the signal peptide at more than one site.

Suitable host cells for expression of polypeptides include prokaryotes, yeast or higher eukaryotic cells. Mammalian or insect cells are generally preferred for use as host cells. Appropriate cloning and expression vectors for use with bacterial, fungal, yeast, and mammalian cellular hosts are described, for example, in Pouwels et al. Cloning Vectors: A Laboratory Manual, Elsevier, New York, (1985). Cell-free translation systems could also be employed to produce polypeptides using RNAs derived from DNA constructs disclosed herein.

### Prokaryotic Systems

Prokaryotes include gram-negative or gram-positive organisms. Suitable prokaryotic host cells for transformation include, for example, *E. coli, Bacillus subtilis, Salmonella typhimurium,* and various other species within the genera *Pseudomonas, Streptomyces,* and *Staphylococcus.* In a prokaryotic host cell, such as *E. coli,* a polypeptide may include an N-terminal methionine residue to facilitate expression of the recombinant polypeptide in the prokaryotic host cell. The N-terminal Met may be cleaved from the expressed recombinant polypeptide.

Expression vectors for use in prokaryotic host cells generally comprise one or more phenotypic selectable marker genes. A phenotypic selectable marker gene is, for example, a gene encoding a protein that confers antibiotic resistance or that supplies an autotrophic requirement. Examples of useful expression vectors for prokaryotic host cells include those derived from commercially available plasmids such as the cloning vector pBR322 (ATCC 37017). pBR322 contains genes for ampicillin and tetracycline resistance and thus provides simple means for identifying transformed cells. An appropriate promoter and a DNA sequence are inserted into the pBR322 vector. Other commercially available vectors include, for example, pKK223-3 (Pharmacia Fine Chemicals, Uppsala, Sweden) and pGEM1 (Promega Biotec, Madison, WI, USA).

Promoter sequences commonly used for recombinant prokaryotic host cell expression vectors include β-lactamase (penicillinase), lactose promoter system (Chang et al., Nature, 275:615 (1978); and Goeddel et al., Nature, 281:544 (1979)), tryptophan (trp) promoter system (Goeddel et al., Nucl. Acids Res., 8:4057 (1980)); and EP-A-36776 and tac promoter (Maniatis, Molecular Cloning: A Laboratory Manual, Cold Spring Harbor Laboratory, p. 412 (1982)). A particularly useful prokaryotic host cell expression system employs a phage λP_{L} promoter and a cI857ts thermolabile repressor sequence. Plasmid vectors available from the American Type Culture Collection which incorporate derivatives of the λP_{L} promoter include plasmid pHUB2 (resident in *E. coli* strain JMB9, ATCC 37092) and pPLc28 (resident in *E. coli* RR1, ATCC 53082).

### Yeast Systems

Alternatively, the polypeptides may be expressed in yeast host cells, preferably from the *Saccharomyces* genus (*e.g., S. cerevisiae*). Other genera of yeast, such as *Pichia* or *Kluyveromyces,* may also be employed. Yeast vectors will often contain an origin of replication sequence from a 2µ yeast plasmid, an autonomously replicating sequence (ARS), a promoter region, sequences for polyadenylation, sequences for transcription termination, and a selectable marker gene. Suitable promoter sequences for yeast vectors include, among others, promoters for metallothionein, 3-phosphoglycerate kinase (Hitzeman et al., J. Biol. Chem. 255:2073 (1980)) or other glycolytic enzymes (Hess et al., J. Adv. Enzyme Reg., 7:149 (1968); and Holland et al., Biochem., 17:4900 (1978)), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phospho-glucose isomerase, and glucokinase. Other suitable vectors and promoters for use in yeast expression are further described in Hitzeman, EPA-73,657. Another alternative is the glucose-repressible ADH2 promoter described by Russell et al., J. Biol. Chem., 258:2674 (1982) and Beier et al., Nature, 300:724 (1982). Shuttle vectors replicable in both yeast and *E. coli* may be constructed by inserting DNA sequences from pBR322 for selection and replication in *E. coli* (Amp^{r} gene and origin of replication) into the above-described yeast vectors.

The yeast α-factor leader sequence may be employed to direct secretion of the polypeptide. The α-factor leader sequence is often inserted between the promoter sequence and the structural gene sequence. *See,* Kurjan et al., Cell, 30:933 (1982) and Bitter et al., Proc. Natl. Acad. Sci. USA, 81:5330 (1984). Other leader sequences suitable for facilitating secretion of recombinant polypeptides from yeast hosts are known to those of skill in the art. A leader sequence may be modified near its 3' end to contain one or more restriction sites. This will facilitate fusion of the leader sequence to the structural gene.

Yeast transformation protocols are known to those of skill in the art. One such protocol is described by Hinnen et al., Proc. Natl. Acad. Sci. USA, 75:1929 (1978). The Hinnen et al. protocol selects for Trp⁺ transformants in a selective medium, wherein the selective medium consists of 0.67% yeast nitrogen base, 0.5% casamino acids, 2% glucose, 10 mg/ml adenine and 20 mg/ml uracil.

Yeast host cells transformed by vectors containing an ADH2 promoter sequence may be grown for inducing expression in a "rich" medium. An example of a rich medium is one consisting of 1% yeast extract, 2% peptone, and 1% glucose supplemented with 80 mg/ml adenine and 80 mg/ml uracil. Derepression of the ADH2 promoter occurs when glucose is exhausted from the medium.

### Mammalian or Insect Systems

Mammalian or insect host cell culture systems also may be employed to express recombinant polypeptides. Bacculovirus systems for production of heterologous proteins in insect cells are reviewed by Luckow and Summers, *Bio*/*Technology* 6:47 (1988). Established cell lines of mammalian origin also may be employed. Examples of suitable mammalian host cell lines include the COS-7 line of monkey kidney cells (ATCC CRL 1651) (Gluzman et al., Cell 23:175 (1981)), L cells, C127 cells, 3T3 cells (ATCC CCL 163), Chinese hamster ovary (CHO) cells, HeLa cells, and BHK (ATCC CRL 10) cell lines, and the CV1/EBNA cell line derived from the African green monkey kidney cell line CV1 (ATCC CCL 70) as described by McMahan et al., EMBO J. 10: 2821 (1991).

Established methods for introducing DNA into mammalian cells have been described (Kaufman, R.J., Large Scale Mammalian Cell Culture, pp. 15-69 (1990)). Additional protocols using commercially available reagents, such as Lipofectamine lipid reagent (Gibco/BRL) or Lipofectamine-Plus lipid reagent, can be used to transfect cells (Feigner et al., Proc. Natl. Acad. Sci. USA 84:7413-7417 (1987)). In addition, electroporation can be used to transfect mammalian cells using conventional procedures, such as those in Sambrook et al., Molecular Cloning: A Laboratory Manual, 2nd ed. Vol. 1-3, Cold Spring Harbor Laboratory Press (1989). Selection of stable transformants can be performed using methods known in the art, such as, for example, resistance to cytotoxic drugs. Kaufman et al., Meth. in Enzymology 185:487-511 (1990), describes several selection schemes, such as dihydrofolate reductase (DHFR) resistance. A suitable host strain for DHFR selection can be CHO strain DX-B11, which is deficient in DHFR (Urlaub and Chasin, Proc. Natl. Acad. Sci. USA 77:4216-4220 (1980)). A plasmid expressing the DHFR cDNA can be introduced into strain DX-B11, and only cells that contain the plasmid can grow in the appropriate selective media. Other examples of selectable markers that can be incorporated into an expression vector include cDNAs conferring resistance to antibiotics, such as G418 and hygromycin B. Cells harboring the vector can be selected on the basis of resistance to these compounds.

Transcriptional and translational control sequences for mammalian host cell expression vectors can be excised from viral genomes. Commonly used promoter sequences and enhancer sequences are derived from polyoma virus, adenovirus 2, simian virus 40 (SV40), and human cytomegalovirus. DNA sequences derived from the SV40 viral genome, for example, SV40 origin, early and late promoter, enhancer, splice, and polyadenylation sites can be used to provide other genetic elements for expression of a structural gene sequence in a mammalian host cell. Viral early and late promoters are particularly useful because both are easily obtained from a viral genome as a fragment, which can also contain a viral origin of replication (Fiers et a]., Nature 273:113 (1978); Kaufman et al., Meth. in Enzymology 185:487-511 (1990)). Smaller or larger SV40 fragments can also be used, provided the approximately 250 bp sequence extending from the *Hind* III site toward the *Bgl* I site located in the SV40 viral origin of replication site is included.

Additional control sequences shown to improve expression of heterologous genes from mammalian expression vectors include such elements as the expression augmenting sequence element (EASE) derived from CHO cells (Morris et al., Animal Cell Technology, pp. 529-534 (1997)) and PCT Application WO 97/25420 and the tripartite leader (TPL) and VA gene RNAs from Adenovirus 2 (Gingeras et al., *J. Biol. Chem. 257*:13475-13491 (1982)). The internal ribosome entry site (IRES) sequences of viral origin allows dicistronic mRNAs to be translated efficiently (Oh and Sarnow, Current Opinion in Genetics and Development 3:295-300 (1993); Ramesh et al., Nucleic Acids Research 24:2697-2700 (1996)). Expression of a heterologous cDNA as part of a dicistronic mRNA followed by the gene for a selectable marker (e.g. DHFR) has been shown to improve transfectability of the host and expression of the heterologous cDNA (Kaufman et al., Meth. in Enzymology 185:487-511 (1990)). Exemplary expression vectors that employ dicistronic mRNAs are pTR-DC/GFP described by Mosser et al., Biotechniques 22:150-161 (1997), and p2A5I described by Morris et al., Animal Cell Technology, pp. 529-534 (1997).

A useful high expression vector, pCAVNOT, has been described by Mosley et al., Cell 59:335-348 (1989). Other expression vectors for use in mammalian host cells can be constructed as disclosed by Okayama and Berg, Mol. Cell. Biol. 3:280 (1983). A useful system for stable high level expression of mammalian cDNAs in C127 murine mammary epithelial cells can be constructed substantially as described by Cosman et al., Mol. Immunol. 23:935 (1986). A useful high expression vector, PMLSV N1/N4, described by Cosman et al., Nature 312:768 (1984), has been deposited as ATCC 39890. Additional useful mammalian expression vectors are described in EP-A-0367566, and in WO 91/18982, incorporated by reference herein. In yet another alternative, the vectors can be derived from retroviruses.

Additional useful expression vectors, pFLAG® and pDC311, can also be used. FLAG® technology is centered on the fusion of a low molecular weight (1kD), hydrophilic, FLAG® marker peptide to the N-terminus of a recombinant protein expressed by pFLAG® expression vectors. pDC311 is another specialized vector used for expressing proteins in CHO cells. pDC311 is characterized by a bicistronic sequence containing the gene of interest and a dihydrofolate reductase (DHFR) gene with an internal ribosome binding site for DHFR translation, an expression augmenting sequence element (EASE), the human CMV promoter, a tripartite leader sequence, and a polyadenylation site.

Regarding signal peptides that may be employed, the native signal peptide may be replaced by a heterologous signal peptide or leader sequence, if desired. The choice of signal peptide or leader may depend on factors such as the type of host cells in which the recombinant polypeptide is to be produced. To illustrate, examples of heterologous signal peptides that are functional in mammalian host cells include the signal sequence for interleukin-7 (IL-7) described in United States Patent 4,965,195; the signal sequence for interleukin-2 receptor described in Cosman et al., Nature, 312:768 (1984); the interleukin-4 receptor signal peptide described in EP 367,566; the type I interleukin-1 receptor signal peptide described in U.S. Patent 4,968,607; and the type II interleukin-1 receptor signal peptide described in EP 460,846.

### Purification

The invention also includes methods of isolating and purifying the polypeptides and fragments thereof.

### Isolation and Purification

The "isolated" polypeptides or fragments thereof encompassed by this invention are polypeptides or fragments that are not in an environment identical to an environment in which it or they can be found in nature. The "purified" polypeptides or fragments thereof encompassed by this invention are essentially free of association with other proteins or polypeptides, for example, as a purification product of recombinant expression systems such as those described above or as a purified product from a non-recombinant source such as naturally occurring cells and/or tissues.

In one preferred embodiment, the purification of recombinant polypeptides or fragments can be accomplished using fusions of polypeptides or fragments of the invention to another polypeptide to aid in the purification of polypeptides or fragments of the invention. Such fusion partners can include the poly-His or other antigenic identification peptides described above as well as the Fc moieties described previously.

With respect to any type of host cell, as is known to the skilled artisan, procedures for purifying a recombinant polypeptide or fragment will vary according to such factors as the type of host cells employed and whether or not the recombinant polypeptide or fragment is secreted into the culture medium.

In general, the recombinant polypeptide or fragment can be isolated from the host cells if not secreted, or from the medium or supernatant if soluble and secreted, followed by one or more concentration, salting-out, ion exchange, hydrophobic interaction, affinity purification or size exclusion chromatography steps. As to specific ways to accomplish these steps, the culture medium first can be concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. In addition, a chromatofocusing step can be employed. Alternatively, a hydrophobic interaction chromatography step can be employed. Suitable matrices can be phenyl or octyl moieties bound to resins. In addition, affinity chromatography with a matrix which selectively binds the recombinant protein can be employed. Examples of such resins employed are lectin columns, dye columns, and metal-chelating columns. Finally, one or more reversed-phase high performance liquid chromatography (RP-HPLC) steps employing hydrophobic RP-HPLC media, (e.g., silica gel or polymer resin having pendant methyl, octyl, octyldecyl or other aliphatic groups) can be employed to further purify the polypeptides. Some or all of the foregoing purification steps, in various combinations, are well known and can be employed to provide an isolated and purified recombinant protein.

It is also possible to utilize an affinity column comprising a polypeptide-binding protein of the invention, such as a monoclonal antibody generated against polypeptides of the invention, to affinity-purify expressed polypeptides. These polypeptides can be removed from an affinity column using conventional techniques, e.g., in a high salt elution buffer and then dialyzed into a lower salt buffer for use or by changing pH or other components depending on the affinity matrix utilized, or be competitively removed using the naturally occurring substrate of the affinity moiety, such as a polypeptide derived from the invention.

In this aspect of the invention, polypeptide-binding proteins, such as the anti-polypeptide antibodies of the invention or other proteins that may interact with the polypeptide of the invention, can be bound to a solid phase support such as a column chromatography matrix or a similar substrate suitable for identifying, separating, or purifying cells that express polypeptides of the invention on their surface. Adherence of polypeptide-binding proteins of the invention to a solid phase contacting surface can be accomplished by any means, for example, magnetic microspheres can be coated with these polypeptide-binding proteins and held in the incubation vessel through a magnetic field. Suspensions of cell mixtures are contacted with the solid phase that has such polypeptide-binding proteins thereon. Cells having polypeptides of the invention on their surface bind to the fixed polypeptide-binding protein and unbound cells then are washed away. This affinity-binding method is useful for purifying, screening, or separating such polypeptide-expressing cells from solution. Methods of releasing positively selected cells from the solid phase are known in the art and encompass, for example, the use of enzymes. Such enzymes are preferably non-toxic and non-injurious to the cells and are preferably directed to cleaving the cell-surface binding partner.

Alternatively, mixtures of cells suspected of containing polypeptide-expressing cells of the invention first can be incubated with a biotinylated polypeptide-binding protein of the invention. Incubation periods are typically at least one hour in duration to ensure sufficient binding to polypeptides of the invention. The resulting mixture then is passed through a column packed with avidin-coated beads, whereby the high affinity of biotin for avidin provides the binding of the polypeptide-binding cells to the beads. Use of avidin-coated beads is known in the art. *See,* Berenson, et al., J Cell. Biochem., 10D:239 (1986). Wash of unbound material and the release of the bound cells is performed using conventional methods.

The desired degree of purity depends on the intended use of the protein. A relatively high degree of purity is desired when the polypeptide is to be administered *in vivo,* for example. In such a case, the polypeptides are purified such that no protein bands corresponding to other proteins are detectable upon analysis by SDS-polyacrylamide gel electrophoresis (SDS-PAGE). It will be recognized by one skilled in the pertinent field that multiple bands corresponding to the polypeptide may be visualized by SDS-PAGE, due to differential glycosylation, differential post-translational processing, and the like. Most preferably, the polypeptide of the invention is purified to substantial homogeneity, as indicated by a single protein band upon analysis by SDS-PAGE. The protein band may be visualized by silver staining, Coomassie blue staining, or (if the protein is radiolabeled) by autoradiography.

### Assays

The purified polypeptides of the invention (including proteins, polypeptides, fragments, variants, oligomers, and other forms) may be tested for the ability to bind the binding partner in any suitable assay, such as a conventional binding assay. To illustrate, the polypeptide may be labeled with a detectable reagent (e.g., a radionuclide, chromophore, enzyme that catalyzes a colorimetric or fluorometric reaction, and the like). The labeled polypeptide is contacted with cells expressing the binding partner. The cells then are washed to remove unbound labeled polypeptide, and the presence of cell-bound label is determined by a suitable technique, chosen according to the nature of the label.

One example of a binding assay procedure is as follows. A recombinant expression vector containing binding partner cDNA is constructed using methods well known in the art. The binding partner comprises an N-terminal cytoplasmic domain, a transmembrane region, and a C-terminal extracellular domain. CV 1-EBNA-1 cells in 10 cm² dishes are transfected with the recombinant expression vector. CV-1/EBNA-1 cells (ATCC CRL 10478) constitutively express EBV nuclear antigen-1 driven from the CMV immediate-early enhancer/promoter. CV1-EBNA-1 was derived from the African Green Monkey kidney cell line CV-1 (ATCC CCL 70), as described by McMahan et al., EMBO J., 10:2821 (1991).

The transfected cells are cultured for 24 hours, and the cells in each dish then are split into a 24-well plate. After culturing an additional 48 hours, the transfected cells (about 4 x 10⁴ cells/well) are washed with BM-NFDM, which is binding medium (RPMI 1640 containing 25 mg/ml bovine serum albumin, 2 mg/ml sodium azide, 20 mM Hepes pH 7.2) to which 50 mg/ml nonfat dry milk has been added. The cells then are incubated for 1 hour at 37°C with various concentrations of, for example, a soluble polypeptide/Fc fusion protein made as set forth above. Cells then are washed and incubated with a constant saturating concentration of a ¹²⁵I-mouse anti-human IgG in binding medium, with gentle agitation for 1 hour at 37°C. After extensive washing, cells are released *via* trypsinization.

The mouse anti-human IgG employed above is directed against the Fc region of human IgG and can be obtained from Jackson Immunoresearch Laboratories, Inc., West Grove, PA. The antibody is radioiodinated using the standard chloramine-T method. The antibody will bind to the Fc portion of any polypeptide/Fc protein that has bound to the cells. In all assays, non-specific binding of ¹²⁵I-antibody is assayed in the absence of the Fc fusion protein/Fc, as well as in the presence of the Fc fusion protein and a 200-fold molar excess of unlabeled mouse anti-human IgG antibody.

Cell-bound ¹²⁵I-antibody is quantified on a Packard Autogamma counter. Affinity calculations (Scatchard, Ann. N. Y. Acad. Sci., 51:660 (1949)) are generated on RS/1 (BBN Software, Boston, MA) run on a Microvax computer.

Another type of suitable binding assay is a competitive binding assay. To illustrate, biological activity of a variant may be determined by assaying for the variant's ability to compete with the native protein for binding to the binding partner.

Competitive binding assays can be performed by conventional methodology. Reagents that may be employed in competitive binding assays include radiolabeled SVPH and intact cells expressing the binding partner (endogenous or recombinant) on the cell surface. For example, a radiolabeled soluble SVPH fragment can be used to compete with a soluble SVPH variant for binding to cells expressing the binding partner on the surface. Instead of intact cells, one could substitute a soluble binding partner/Fc fusion protein bound to a solid phase through the interaction of Protein A or Protein G (on the solid phase) with the Fc moiety. Chromatography columns that contain Protein A and Protein G include those available from Pharmacia Biotech, Inc., Piscataway, NJ.

Another type of competitive binding assay utilizes radiolabeled soluble binding partner, such as a soluble binding partner/Fc fusion protein, and intact cells expressing SVPH. Qualitative results can be obtained by competitive autoradiographic plate binding assays, while (Scatchard plots Scatchard, Ann. N. Y. Acad. Sci. 51:660 (1949)) may be utilized to generate quantitative results.

### USE OF SVPH NUCLEIC ACID OR OLIGONUCLEOTIDES

In addition to being used to express polypeptides as described above, the nucleic acids of the invention, including DNA, RNA, mRNA, and oligonucleotides thereof can be used:
- as probes to identify nucleic acid encoding proteins having proteinase activity;
- to identify human chromosome number 1 or 4;
- to map genes on human chromosome number I or 4;
- to identify genes associated with certain diseases, syndromes, or other conditions associated with human chromosome number 1 or 4;
- as single-stranded sense or antisense oligonucleotides, to inhibit expression of polypeptides encoded by the SVPH-1, SVPH-3, or SVPH-4 gene;
- to detect defective genes in an individual; and
- for gene therapy.

### Probes

The nucleotides of the invention can be used as probes to identify nucleic acid encoding proteins having similar activity or structure. Such uses include the use of fragments. Such fragments may comprise any length of contiguous nucleotides. In one embodiment, the fragment comprises at least about 17 contiguous nucleotides of a DNA sequence. In other embodiments, a DNA fragment comprises at least 30, or at least 60, contiguous nucleotides of a DNA sequence.

Because homologs of SEQ ID NOs:l-3 and 7-11, from other mammalian species are contemplated herein, probes based on the human DNA sequence of SEQ ID NOs:1-3 and 7-11 may be used to screen cDNA libraries derived from other mammalian species, using conventional cross-species hybridization techniques.

Using knowledge of the genetic code in combination with the amino acid sequences set forth above, sets of degenerate oligonucleotides can be prepared. Such oligonucleotides are useful as primers, e.g., in polymerase chain reactions (PCR), whereby DNA fragments are isolated and amplified.

### Chromosome Mapping

All or a portion of the nucleic acids of SEQ ID NOs:1-3 and 7-11, including oligonucleotides, can be used by those skilled in the art using well-known techniques to identify human chromosomes and the specific locus thereof, that contains the DNA of SVPH family members. For example, all or a portion of SEQ ID NO:3, SEQ ID NO:10, and SEQ ID NO:11 can be used to identify human chromosome 1. In addition, all or a portion of SEQ ID NO:1, SEQ ID NO:7, SEQ ID NO:8, and SEQ ID NO:9 can be used to identify human chromosome 4. Useful techniques include, but are not limited to, using the sequence or portions, including oligonucleotides, as a probe in various well-known techniques such as radiation hybrid mapping (high resolution), in situ hybridization to chromosome spreads (moderate resolution), and Southern blot hybridization to hybrid cell lines containing individual human chromosomes (low resolution).

For example, chromosomes can be mapped by radiation hybrid mapping. First, PCR is performed using the Whitehead Institute/MIT Center for Genome Research Genebridge4 panel of 93 radiation hybrids
(http://www-genome.wi.mit.edu/ftp/distribution/
human-STS-releases/july97/rhmap/genebridge4.html). Primers are used which lie within a putative exon of the gene of interest and which amplify a product from human genomic DNA, but do not amplify hamster genomic DNA. The results of the PCRs are converted into a data vector that is submitted to the Whitehead/MIT Radiation Mapping site on the internet (http://www-seq.wi.mit.edu). The data is scored and the chromosomal assignment and placement relative to known Sequence Tag Site (STS) markers on the radiation hybrid map is provided. The following web site provides additional information about radiation hybrid mapping:
http://www-genome.wi.mit.edu/ftp/distribution/human_STS_releases/july97/ 07-97.INTRO.html).

### Identifying Associated Diseases

As set forth below, sequences encoding SVPH-4a and SVPH-4b have been mapped by radiation hybrid mapping to the 1p11-13 region of chromosome 1. That region is associated with specific diseases which include but are not limited to fetal hydantoin syndrome, diphenylhydantoin toxicity, and pheochromocytoma. Thus, the nucleic acid of SEQ ID Nos:3, 10 and 11, or a fragment thereof, can be used by one skilled in the art using well-known techniques to analyze abnormalities associated with SVPH-4 genes. In addition, sequences encoding SVPH-1a, SVPH-1b, and SVPH-1c have been mapped by radiation hybrid mapping to the 4q34 region of chromosome 4. Thus, the nucleic acid of SEQ ID Nos:1, 7, 8, and 9, or a fragment thereof, can be used by one skilled in the art using well-known techniques to analyze abnormalities associated with SVPH-1 genes. Similarly, a skilled artisan can use the nucleic acid of SEQ ID No.:2, or a fragment thereof, to analyze abnormalities associated with SVPH-3 genes. This enables one to distinguish conditions in which this marker is rearranged or deleted. In addition, nucleic acid of SEQ ID NOs:1-3 and 7-11 or a fragment thereof can be used as a positional marker to map other genes of unknown location.

The DNA may be used in developing treatments for any disorder mediated (directly or indirectly) by defective, or insufficient amounts of, the genes corresponding to the nucleic acids of the invention. Disclosure herein of native nucleotide sequences permits the detection of defective genes, and the replacement thereof with normal genes. Defective genes may be detected in *in vitro* diagnostic assays, and by comparison of a native nucleotide sequence disclosed herein with that of a gene derived from a person suspected of harboring a defect in this gene.

### Sense-Antisense

Other useful fragments of the nucleic acids include antisense or sense oligonucleotides comprising a single-stranded nucleic acid sequence (either RNA or DNA) capable of binding to target mRNA (sense) or DNA (antisense) sequences. Antisense or sense oligonucleotides, according to the present invention, comprise a fragment of SEQ ID NOs:1-3 or 7-11. Such a fragment generally comprises at least about 14 nucleotides, preferably from about 14 to about 30 nucleotides. The ability to derive an antisense or a sense oligonucleotide, based upon a cDNA sequence encoding a given protein is described in, for example, Stein and Cohen, Cancer Res., 48:2659 (1988) and van der Krol et al., BioTechniques, 6:958 (1988).

Binding of antisense or sense oligonucleotides to target nucleic acid sequences results in the formation of duplexes that block or inhibit protein expression by one of several means, including enhanced degradation of the mRNA by RNAseH, inhibition of splicing, premature termination of transcription or translation, or by other means. The antisense oligonucleotides thus may be used to block expression of proteins. Antisense or sense oligonucleotides further comprise oligonucleotides having modified sugar-phosphodiester backbones (or other sugar linkages, such as those described in WO91/06629) and wherein such sugar linkages are resistant to endogenous nucleases. Such oligonucleotides with resistant sugar linkages are stable *in vivo* (*i.e.,* capable of resisting enzymatic degradation) but retain sequence specificity to be able to bind to target nucleotide sequences.

Other examples of sense or antisense oligonucleotides include those oligonucleotides which are covalently linked to organic moieties, such as those described in WO 90/10448, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, lipofection, CaPO₄-Mediated DNA transfection, electroporation, or by using gene transfer vectors such as Epstein-Barr virus.

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

### USE OF SVPH POLYPEPTIDES AND FRAGMENTED POLYPEPTIDES

Uses include, but are not limited to, the following:
- Purifying proteins and measuring activity thereof
- Delivery Agents
- Therapeutic and Research Reagents
- Molecular weight and Isoelectric focusing markers
- Controls for peptide fragmentation
- Identification of unknown proteins
- Preparation of Antibodies

### Purification Reagents

Each of the polypeptides of the invention finds use as a protein purification reagent. For example, the polypeptides may be used to purify binding partner proteins. In particular embodiments, a polypeptide (in any form described herein that is capable of binding the binding partner) is attached to a solid support by conventional procedures. As one example, affinity chromatography columns containing functional groups that will react with functional groups on amino acid side chains of proteins are available (Pharmacia Biotech, Inc., Piscataway, NJ). In an alternative, a polypeptide/Fc protein (as discussed above) is attached to Protein A- or Protein G-containing chromatography columns through interaction with the Fc moiety.

The polypeptide also finds use in purifying or identifying cells that express the binding partner on the cell surface. Polypeptides are bound to a solid phase such as a column chromatography matrix or a similar suitable substrate. For example, magnetic microspheres can be coated with the polypeptides and held in an incubation vessel through a magnetic field. Suspensions of cell mixtures containing the binding partner expressing cells are contacted with the solid phase having the polypeptides thereon. Cells expressing the binding partner on the cell surface bind to the fixed polypeptides, and unbound cells then are washed away.

Alternatively, the polypeptides can be conjugated to a detectable moiety, then incubated with cells to be tested for binding partner expression. After incubation, unbound labeled matter is removed and the presence or absence of the detectable moiety on the cells is determined.

In a further alternative, mixtures of cells suspected ofcontaining cells expressing the binding partner are incubated with biotinylated polypeptides. Incubation periods are typically at least one hour in duration to ensure sufficient binding. The resulting mixture then is passed through a column packed with avidin-coated beads, whereby the high affinity of biotin for avidin provides binding of the desired cells to the beads. Procedures for using avidin-coated beads are known. *See,* Berenson, et al., J. Cell. Biochem., 10D:239 (1986). Washing to remove unbound material, and the release of the bound cells, are performed using conventional methods.

### Measuring Activity

Polypeptides also find use in measuring the biological activity of the binding partner protein in terms of their binding affinity. The polypeptides thus may be employed by those conducting "quality assurance" studies, e.g., to monitor shelf life and stability of protein under different conditions. For example, the polypeptides may be employed in a binding affinity study to measure the biological activity of a binding partner protein that has been stored at different temperatures, or produced in different cell types. The proteins also may be used to determine whether biological activity is retained after modification of a binding partner protein (e.g., chemical modification, truncation, mutation, etc.). The binding affinity of the modified binding partner protein is compared to that of an unmodified binding partner protein to detect any adverse impact of the modifications on biological activity of the binding partner. The biological activity of a binding partner protein thus can be ascertained before it is used in a research study, for example.

### Delivery Agents

The polypeptides also find use as carriers for delivering agents attached thereto to cells bearing the binding partner (or to other cell types found to express the binding partner on the cell surface) in *in vitro* or *in vivo* procedures.

Detectable (diagnostic) and therapeutic agents that may be attached to a polypeptide include, but are not limited to, toxins, other cytotoxic agents, drugs, radionuclides, chromophores, enzymes that catalyze a colorimetric or fluorometric reaction, and the like, with the particular agent being chosen according to the intended application. Among the toxins are ricin, abrin, diphtheria toxin, *Pseudomonas aeruginosa* exotoxin A, ribosomal inactivating proteins, mycotoxins such as trichothecenes, and derivatives and fragments (e.g., single chains) thereof. Radionuclides suitable for diagnostic use include, but are not limited to, ¹²³I, ¹³¹I, ^{99m}Tc, ¹¹¹In, and ⁷⁶Br. Examples ofradionuclides suitable for therapeutic use are¹³¹I, ²¹¹At, ⁷⁷ Br, ¹⁸⁶Re, ¹⁸⁸Re, ²¹²Pb, ²¹²Bi, ¹⁰⁹Pd, ⁶⁴Cu, and ⁶⁷Cu.

Such agents may be attached to the polypeptide by any suitable conventional procedure. The polypeptide comprises functional groups on amino acid side chains that can be reacted with functional groups on a desired agent to form covalent bonds, for example. Alternatively, the protein or agent may be derivatized to generate or attach a desired reactive functional group. The derivatization may involve attachment of one of the bifunctional coupling reagents available for attaching various molecules to proteins (Pierce Chemical Company, Rockford, Illinois). A number of techniques for radiolabeling proteins are known. Radionuclide metals may be attached to polypeptides by using a suitable bifunctional chelating agent, for example.

Conjugates comprising polypeptides and a suitable diagnostic or therapeutic agent (preferably covalently linked) are thus prepared. The conjugates are administered or otherwise employed in an amount appropriate for the particular application.

### Therapeutic Agents

Polypeptides of the invention may be used in developing treatments for any disorder mediated (directly or indirectly) by defective, or insufficient amounts of the polypeptides. These polypeptides may be administered to a mammal afflicted with such a disorder.

The polypeptides may also be employed in inhibiting a biological activity of the binding partner, in *in vitro* or *in vivo* procedures. For example, a purified polypeptide may be used to inhibit binding of the binding partner to an endogenous cell surface binding partner. Biological effects that result from the binding of SVPH to endogenous binding partner thus are inhibited.

In addition, an SVPH binding partner may be administered to a mammal to treat a binding partner-mediated disorder. Such binding partner-mediated disorders include conditions caused (directly or indirectly) or exacerbated by the binding partner.

Compositions of the present invention may contain a polypeptide in any form described herein, such as native proteins, variants, derivatives, oligomers, and biologically active fragments. In particular embodiments, the composition comprises a soluble polypeptide or an oligomer comprising soluble SVPH polypeptides or SVPH binding partner polypeptides.

Compositions comprising an effective amount of a polypeptide of the present invention, in combination with other components such as a physiologically acceptable diluent, carrier, or excipient, are provided herein. The polypeptides can be formulated according to known methods used to prepare pharmaceutically useful compositions. They can be combined in admixture, either as the sole active material or with other known active materials suitable for a given indication, with pharmaceutically acceptable diluents (e.g., saline, Tris-HCl, acetate, and phosphate buffered solutions), preservatives (e.g., thimerosal, benzyl alcohol, parabens), emulsifiers, solubilizers, adjuvants and/or carriers. Suitable formulations for pharmaceutical compositions include those described in Remington's Pharmaceutical Sciences, 16th ed. 1980, Mack Publishing Company, Easton, PA.

In addition, such compositions can be complexed with polyethylene glycol (PEG), metal ions, or incorporated into polymeric compounds such as polyacetic acid, polyglycolic acid, hydrogels, dextran, etc., or incorporated into liposomes, microemulsions, micelles, unilamellar or multilamellar vesicles, erythrocyte ghosts or spheroblasts. Such compositions will influence the physical state, solubility, stability, rate of *in vivo* release, and rate of *in* vivo clearance, and are thus chosen according to the intended application.

The compositions of the invention can be administered in any suitable manner, e.g., topically, parenterally, or by inhalation. The term "parenteral" includes injection, e.g., by subcutaneous, intravenous, or intramuscular routes, also including localized administration, e.g., at a site of disease or injury. Sustained release from implants is also contemplated. One skilled in the pertinent art will recognize that suitable dosages will vary, depending upon such factors as the nature of the disorder to be treated, the patient's body weight, age, and general condition, and the route of administration. Preliminary doses can be determined according to animal tests, and the scaling of dosages for human administration is performed according to art-accepted practices.

Compositions comprising nucleic acids in physiologically acceptable formulations are also contemplated. DNA may be formulated for injection, for example.

### Research Agents

Another use of the polypeptide of the present invention is as a research tool for studying the biological effects that result from inhibiting binding partner/SVPH interactions on different cell types. Polypeptides also may be employed in *in vitro* assays for detecting the binding partner or SVPH or the interactions thereof.

### Molecular Weight, Isoelectric Point Markers

The polypeptides of the present invention can be subjected to fragmentation into smaller peptides by chemical and enzymatic means, and the peptide fragments so produced can be used in the analysis of other proteins or polypeptides. For example, such peptide fragments can be used as peptide molecular weight markers, peptide isoelectric point markers, or in the analysis of the degree of peptide fragmentation. Thus, the invention also includes these polypeptides and peptide fragments, as well as kits to aid in the determination of the apparent molecular weight and isoelectric point of an unknown protein and kits to assess the degree of fragmentation of an unknown protein.

Although all methods of fragmentation are encompassed by the invention, chemical fragmentation is a preferred embodiment, and includes the use of cyanogen bromide to cleave under neutral or acidic conditions such that specific cleavage occurs at methionine residues (E. Gross, Methods in Enz., 11:238-255 (1967)). This can further include additional steps, such as a carboxymethylation step to convert cysteine residues to an unreactive species. Table 1 summarizes the fragmentation pattern of SEQ ID NOs:12-16 following chemical cleavage with cyanogen bromide.

Enzymatic fragmentation is another preferred embodiment, and includes the use of a protease such as Asparaginylendo-peptidase, Arginylendo-peptidasc, *Achrontobacter* protease I, Trypsin, *Staphlococcus aureus* V8 protease, Endoproteinase Asp-N, or Endoproteinase Lys-C under conventional conditions to result in cleavage at specific amino acid residues. Asparaginylendo-peptidase can cleave specifically on the carboxyl side of the asparagine residues present within the polypeptides of the invention. Arginylendo-peptidase can cleave specifically on the carboxyl side of the arginine residues present within these polypeptides. *Achromobacter* protease I can cleave specifically on the carboxyl side of the lysine residues present within the polypeptides (Sakiyama and Nakat, U.S. Patent No. 5,248,599; T. Masaki et al., Biochim. Biophys. Acta, 660:44-50 (1981); T. Masaki et al., Biochim. Biophys. Acta, 660:51-55 (1981)). Trypsin can cleave specifically on the carboxyl side of the arginine and lysine residues present within polypeptides of the invention. Enzymatic fragmentation may also occur with a protease that cleaves at multiple amino acid residues. For example, *Staphlococcus aureus* V8 protease can cleave specifically on the carboxyl side of the aspartic and glutamic acid residues present within polypeptides (D. W. Cleveland, J. Biol. Chem., 3:1102-1106 (1977)). Endoproteinase Asp-N can cleave specifically on the amino side of the asparagine residues present within polypeptides. Endoproteinase Lys-C can cleave specifically on the carboxyl side of the lysine residues present within polypeptides of the invention. Other enzymatic and chemical treatments can likewise be used to specifically fragment these polypeptides into a unique set of specific peptides.

Of course, the peptides and fragments of the polypeptides of the invention can also be produced by conventional recombinant processes and synthetic processes well known in the art. With regard to recombinant processes, the polypeptides and peptide fragments encompassed by invention can have variable molecular weights, depending upon the host cell in which they are expressed. Glycosylation of polypeptides and peptide fragments of the invention in various cell types can result in variations of the molecular weight of these pieces, depending upon the extent of modification. The size of these pieces can be most heterogeneous with fragments of polypeptide derived from the extracellular portion of the polypeptide. Consistent polypeptides and peptide fragments can be obtained by using polypeptides derived entirely from the transmembrane and cytoplasmic regions, pretreating with N-glycanase to remove glycosylation, or expressing the polypeptides in bacterial hosts.

The molecular weight of these polypeptides can also be varied by fusing additional peptide sequences to both the amino and carboxyl terminal ends of polypeptides of the invention. Fusions of additional peptide sequences at the amino and carboxyl terminal ends of polypeptides of the invention can be used to enhance expression of these polypeptides or aid in the purification of the protein. In addition, fusions of additional peptide sequences at the amino and carboxyl terminal ends of polypeptides of the invention will alter some, but usually not all, of the fragmented peptides of the polypeptides generated by enzymatic or chemical treatment. Of course, mutations can be introduced into polypeptides of the invention using routine and known techniques of molecular biology. For example, a mutation can be designed so as to eliminate a site of proteolytic cleavage by a specific enzyme or a site of cleavage by a specific chemically induced fragmentation procedure. The elimination of the site will alter the peptide fingerprint of polypeptides of the invention upon fragmentation with the specific enzyme or chemical procedure.

When the invention relates to the use of fragmented peptide molecular weight markers, those markers are preferably at least 10 amino acids in size. More preferably, these fragmented peptide molecular weight markers are between 10 and 100 amino acids in size. Even more preferable are fragmented peptide molecular weight markers between 10 and 50 amino acids in size and especially between 10 and 35 amino acids in size. Most preferable are fragmented peptide molecular weight markers between 10 and 20 amino acids in size.

Because the unique amino acid sequence of each fragment specifies a molecular weight, these fragments can thereafter serve as molecular weight markers using such analysis techniques to assist in the determination of the molecular weight of an unknown protein, polypeptides or fragments thereof. The molecular weight markers of the invention serve particularly well as molecular weight markers for the estimation of the apparent molecular weight of proteins that have similar apparent molecular weights and, consequently, allow increased accuracy in the determination of apparent molecular weight of proteins.

Among the methods for determining molecular weight are sedimentation, gel electrophoresis, chromatography, and mass spectrometry. A particularly preferred embodiment is denaturing polyacrylamide gel electrophoresis (U. K. Laemmli, Nature, 227:680-685 (1970)). Conventionally, the method uses two separate lanes of a gel containing sodium dodecyl sulfate and a concentration of acrylamide between 6-20%. The ability to simultaneously resolve the marker and the sample under identical conditions allows for increased accuracy. It is understood, of course, that many different techniques can be used for the determination of the molecular weight of an unknown protein using polypeptides of the invention, and that this embodiment in no way limits the scope of the invention.

' In addition, each unglycosylated polypeptide or fragment thereof has a pI that is intrinsically determined by its unique amino acid sequence (which pI can be estimated by the skilled artisan using any of the computer programs designed to predict pI values currently available, calculated using any well-known amino acid pKa table, or measured empirically). Therefore these polypeptides and fragments thereof can serve as specific markers to assist in the determination of the isoelectric point of an unknown protein, polypeptide, or fragmented peptide using techniques such as isoelectric focusing. These polypeptide or fragmented peptide markers serve particularly well for the estimation of apparent isoelectric points of unknown proteins that have apparent isoelectric points close to that of the polypeptide or fragmented peptide markers of the invention.

The technique of isoelectric focusing can be further combined with other techniques such as gel electrophoresis to simultaneously separate a protein on the basis of molecular weight and charge. The ability to simultaneously resolve these polypeptide or fragmented peptide markers and the unknown protein under identical conditions allows for increased accuracy in the determination of the apparent isoelectric point of the unknown protein. This is of particular interest in techniques, such as two dimensional electrophoresis (T.D. Brock and M.T. Madigan, Biology of Microorganisms 76-77, Prentice Hall, 6th ed. (1991)), where the nature of the procedure dictates that any markers should be resolved simultaneously with the unknown protein. In addition, with such methods, these polypeptides and fragmented peptides thereof can assist in the determination of both the isoelectric point and molecular weight of an unknown protein or fragmented peptide.

Polypeptides and fragmented peptides can be visualized using two different methods that allow a discrimination between the unknown protein and the molecular weight markers. In one embodiment, the polypeptide and fragmented peptide molecular weight markers of the invention can be visualized using antibodies generated against these markers and conventional immunoblotting techniques. This detection is performed under conventional conditions that do not result in the detection of the unknown protein. It is understood that it may not be possible to generate antibodies against all polypeptide fragments of the invention, since small peptides may not contain immunogenic epitopes. It is further understood that not all antibodies will work in this assay; however, those antibodies which are able to bind polypeptides and fragments of the invention can be readily determined using conventional techniques.

The unknown protein is also visualized by using a conventional staining procedure. The molar excess of unknown protein to polypeptide or fragmented peptide molecular weight markers of the invention is such that the conventional staining procedure predominantly detects the unknown protein. The level of these polypeptide or fragmented peptide molecular weight markers is such as to allow little or no detection of these markers by the conventional staining method. The preferred molar excess of unknown protein to polypeptide molecular weight markers of the invention is between 2 and 100,000 fold. More preferably, the preferred molar excess of unknown protein to these polypeptide molecular weight markers is between 10 and 10,000 fold and especially between 100 and 1,000 fold.

It is understood of course that many techniques can be used for the determination and detection of molecular weight and isoelectric point of an unknown protein, polypeptides, and fragmented peptides thereof using these polypeptide molecular weight markers and peptide fragments thereof and that these embodiments in no way limit the scope of the invention.

In another embodiment, the analysis of the progressive fragmentation of the polypeptides of the invention into specific peptides (D. W. Cleveland et al., J. Biol. Chem. 252:1102-1106 (1977)), such as by altering the time or temperature of the fragmentation reaction, can be used as a control for the extent of cleavage of an unknown protein. For example, cleavage of the same amount of polypeptide and unknown protein under identical conditions can allow for a direct comparison of the extent of fragmentation. Conditions that result in the complete fragmentation of the polypeptide can also result in complete fragmentation of the unknown protein.

As to the specific use of the polypeptides and fragmented peptides of the invention as molecular weight markers, the fragmentation of the polypeptide of SEQ ID NOs:4-6 and 12-16 with cyanogen bromide in the absence of glycosylation generates a unique set of fragmented peptide molecular weight markers with molecular weights as set forth in Table 1 on the following page.

**Table 1. Molecular Weights of Peptide Fragments Generated by Cyanogen Bromide Digest**

| SEQ ID NO:4 | SEQ ID NO:5 | SEQ ID NO:6 | SEQ ID NO:12 | SEQ ID NO:13 | SEQ ID NO:14 | SEQ ID NO:15 | SEQ ID NO:16 |
|---|---|---|---|---|---|---|---|
| | | | | | | | |
| 149.2 | 149.2 | 374.3 | 149.2 | 149.2 | 149.2 | 149.2 | 149.2 |
| 4,067.5 | 1,461.7 | 701.8 | 277.4 | 277.4 | 277.4 | 374.5 | 374.5 |
| | 3,960.4 | 1,154.0 | 596.7 | 596.7 | 596.7 | 701.8 | 701.8 |
| | 8,420.6 | 1,196.0 | 994.1 | 994.1 | 970.1 | 1,154.3 | 1,154.3 |
| | | 1,724.0 | 1,106.2 | 1,201.3 | 994.1 | 1,174.3 | 1,174,3 |
| | | 2,040.0 | 1,201.3 | 1,212.4 | 1,106.2 | 1,196.3 | 1,196.3 |
| | | 3,614.0 | 1,212.4 | 1,465.8 | 1,201.3 | 1,757.0 | 1,757.0 |
| | | 4,180.0 | 1,465.8 | 1,830.0 | 1,212.4 | 2,040.3 | 2,056.3 |
| | | 5,327.0 | 1,830.0 | 1,908.1 | 1,465.8 | 2,330.9 | 2,330.9 |
| | | 7,446.0 | 1,908.1 | 1,932.1 | 1,830.0 | 3,614.5 | 3,614.5 |
| | | 7,603.0 | 2,006.3 | 2,218.6 | 1,908.1 | 4,179.8 | 4,179.8 |
| | | 7,611.0 | 2,218.6 | 2,673.0 | 1,946.2 | 5,327.1 1 | 5,327.1 |
| | | 15,692.0 | 2,673.0 | 3,657.1 | 1,960.2 | 6,065.8 | 6,065.8 |
| | | | 4,738.5 | 4,738.5 | 2,218.6 | 6,380.3 | 6,380.3 |
| | | | 12,088.8 | 12,088.8 | 2,G73.0 | 7,446.3 | 6,487.2 |
| | | | 12,649.5 | 12,649.5 | 2,982.3 | 7,610.5 | 7,610.5 |
| | | | 1G,801.8 | 16,801.8 | 4,738.5 | 10.741.4 | 10,741.4 |
| | | | 23,353.2 | 23,353.2 | 12,088.8 | 11,292.9 | i 1,302.9 |
| | | | | | 12,649.5 | 15,692.4 | 15,692.4 |
| | | | | | 16,801.8 | | |
| | | | | | 23.353.2 | | |

The distribution of methionine residues determines the number of amino acids in each peptide and the unique amino acid composition of each peptide determines its molecular weight. Where fragments are used, there is increased accuracy in determining molecular weight over the range of the molecular weights of the fragment.

In addition, the preferred purified polypeptides of the invention (SEQ ID NOs:4-6 and 12-16) have calculated molecular weights of approximately 4,199; 13,938; 55,209; 86,983; 89459; 92,781; 88,923; and 87,990 Daltons, respectively. Thus, where an intact protein is used, the use of these polypeptide molecular weight markers allows increased accuracy in the determination of apparent molecular weight of proteins that have apparent molecular weights close to these weights.

Finally, as to the kits that are encompassed by the invention, the constituents of such kits can be varied, but typically contain the polypeptide and fragmented peptide molecular weight markers. Also, such kits can contain the polypeptides wherein a site necessary for fragmentation has been removed. Furthermore, the kits can contain reagents for the specific cleavage of the polypeptide and the unknown protein by chemical or enzymatic cleavage. Kits can further contain antibodies directed against polypeptides or fragments thereof of the invention.

### Identification of Unknown Proteins

As set forth above, a polypeptide or peptide fingerprint can be entered into or compared to a database of known proteins to assist in the identification of the unknown protein using mass spectrometry (W.J. Henzel et al., Proc. Natl. Acad. Sci. USA, 90:501 I-5015 (1993); D. Fenyo et al., Electrophoresis, 19:998-1005 (1998)). A variety of computer software programs to facilitate these comparisons are accessible via the Internet, such as Protein Prospector (Internet site: prospector.uscf.edu), MultiIdent (Internet site: www.expasy.ch/sprot/multiident.html), PeptideSearch (Internet site:www.mann.embl-heiedelberg.de...deSearch/FR PeptideSearch Form.html), and ProFound (Internet site:www.chait-sgi.rockefeller.edu/cgi-bin/ prot-id-frag.html). These programs allow the user to specify the cleavage agent and the molecular weights of the fragmented peptides within a designated tolerance. The programs compare observed molecular weights to predicted peptide molecular weights derived from sequence databases to assist in determining the identity of the unknown protein.

In addition, a polypeptide or peptide digest can be sequenced using tandem mass spectrometry (MS/MS) and the resulting sequence searched against databases (J.K. Eng, et al., J. Am. Soc. Mass Spec., 5:976-989 (1994); M. Mann and M. Wilm, Anal. Chem., 66:4390-4399 (1994); J.A. Taylor and R.S. Johnson, Rapid Comm. Mass Spec., 11:1067-1075 (1997)). Searching programs that can be used in this process exist on the Internet, such as Lutefisk 97 (Internet site:
www.lsbc.com:70/Lutefisk97.html), and the Protein Prospector, Peptide Search and ProFound programs described above.

Therefore, adding the sequence of a gene and its predicted protein sequence and peptide fragments to a sequence database can aid in the identification of unknown proteins using mass spectrometry.

### Antibodies

Antibodies that are immunoreactive with the polypeptides of the invention are provided herein. Such antibodies specifically bind to the polypeptides via the antigen-binding sites of the antibody (as opposed to non-specific binding). Thus, the polypeptides, fragments, variants, fusion proteins, etc., as set forth above may be employed as "immunogens" in producing antibodies immunoreactive therewith. More specifically, the polypeptides, fragment, variants, fusion proteins, etc. contain antigenic determinants or epitopes that elicit the formation of antibodies.

These antigenic determinants or epitopes can be either linear or conformational (discontinuous). Linear epitopes are composed of a single section of amino acids of the polypeptide, while conformational or discontinuous epitopes are composed of amino acids sections from different regions of the polypeptide chain that are brought into close proximity upon protein folding (C. A. Janeway, Jr. and P. Travers, *Immuno Biology* 3:9, Garland Publishing Inc., 2nd ed. (1996)). Because folded proteins have complex surfaces, the number of epitopes available is quite numerous; however, due to the conformation of the protein and steric hinderances, the number of antibodies that actually bind to the epitopes is less than the number of available epitopes (C. A. Janeway, Jr. and P. Travers, *Immuno Biology,* 2:14, Garland Publishing Inc., 2nd ed. (1996)). Epitopes may be identified by any of the methods known in the art.

Thus, one aspect of the present invention relates to the antigenic epitopes of the polypeptides of the invention. Such epitopes are useful for raising antibodies, in particular monoclonal antibodies, as described in more detail below. Additionally, epitopes from the polypeptides of the invention can be used as research reagents, in assays, and to purify specific binding antibodies from substances such as polyclonal sera or supernatants from cultured hybridomas. Such epitopes or variants thereof can be produced using techniques well known in the art such as solid-phase synthesis, chemical or enzymatic cleavage of a polypeptide, or using recombinant DNA technology.

As to the antibodies that can be elicited by the epitopes of the polypeptides of the invention, whether the epitopes have been isolated or remain part of the polypeptides, both polyclonal and monoclonal antibodies may be prepared by conventional techniques. See, for example, Monoclonal Antibodies, Hybridomas: A New Dimension in Biological Analyses, Kennet et al. (eds.), Plenum Press, New York (1980); and Antibodies: A Laboratory Manual, Harlow and Land (eds.), Cold Spring Harbor Laboratory Press, Cold Spring Harbor, NY, (1988).

Hybridoma cell lines that produce monoclonal antibodies specific for the polypeptides of the invention are also contemplated herein. Such hybridomas may be produced and identified by conventional techniques. One method for producing such a hybridoma cell line comprises immunizing an animal with a polypeptide; harvesting spleen cells from the immunized animal; fusing said spleen cells to a myeloma cell line, thereby generating hybridoma cells; and identifying a hybridoma cell line that produces a monoclonal antibody that binds the polypeptide. The monoclonal antibodies may be recovered by conventional techniques.

The monoclonal antibodies of the present invention include chimeric antibodies, e.g., humanized versions of murine monoclonal antibodies. Such humanized antibodies may be prepared by known techniques and offer the advantage of reduced immunogenicity when the antibodies are administered to humans. In one embodiment, a humanized monoclonal antibody comprises the variable region of a murine antibody (or just the antigen binding site thereof) and a constant region derived from a human antibody. Alternatively, a humanized antibody fragment may comprise the antigen binding site of a murine monoclonal antibody and a variable region fragment (lacking the antigen-binding site) derived from a human antibody. Procedures for the production of chimeric and further engineered monoclonal antibodies include those described in Riechmann et al., Nature, 332:323 (1988); Liu et al., PNAS, 84:3439 (1987); Larrick et al., Bio/Technology, 7:934 (1989), and Winter and Harris, TIPS, 14:139 (May 1993). Procedures to generate antibodies transgenically can be found in GB 2,272,440, US Patent Nos. 5,569,825 and 5,545,806 and related patents claiming priority therefrom, all of which are incorporated by reference herein.

Antigen-binding fragments of the antibodies, which may be produced by conventional techniques, are also encompassed by the present invention. Examples of such fragments include, but are not limited to, Fab and F(ab')₂ fragments. Antibody fragments and derivatives produced by genetic engineering techniques are also provided.

In one embodiment, the antibodies are specific for the polypeptides of the present invention and do not cross-react with other proteins. Screening procedures by which such antibodies may be identified are well known, and may involve immunoaffinity chromatography, for example.

### Uses Thereof

The antibodies of the invention can be used in assays to detect the presence of the polypeptides or fragments of the invention, either *in vitro* or *in vivo.* The antibodies also may be employed in purifying polypeptides or fragments of the invention by immunoaffinity chromatography.

Those antibodies that additionally can block binding of the polypeptides of the invention to the binding partner may be used to inhibit a biological activity that results from such binding. Such blocking antibodies may be identified using any suitable assay procedure, such as by testing antibodies for the ability to inhibit binding of SVPH to certain cells expressing the binding partner. Alternatively, blocking antibodies may be identified in assays for the ability to inhibit a biological effect that results from binding of SVPH to target cells. Antibodies may be assayed for the ability to inhibit SVPH-mediated cell lysis, for example.

Such an antibody may be employed in an *in vitro* procedure, or administered *in vivo* to inhibit a biological activity mediated by the entity that generated the antibody. Disorders caused or exacerbated (directly or indirectly) by the interaction of SVPH with cell surface binding partner thus may be treated. A therapeutic method involves *in vivo* administration of a blocking antibody to a mammal in an amount effective in inhibiting an SVPH-binding partner-mediated biological activity. Monoclonal antibodies are generally preferred for use in such therapeutic methods. In one embodiment, an antigen-binding antibody fragment is employed.

Antibodies may be screened for agonistic (i.e., ligand-mimicking) properties. Such antibodies, upon binding to cell surface binging partner, induce biological effects (e.g., transduction of biological signals) similar to the biological effects induced when SVPH binds to cell surface binding partner.

Compositions comprising an antibody that is directed against SVPH or SVPH binding partner, and a physiologically acceptable diluent, excipient, or carrier, are provided herein. Suitable components of such compositions are as described above for compositions containing SVPH or SVPH binding partner proteins.

Also provided herein are conjugates comprising a detectable (e.g., diagnostic) or therapeutic agent, attached to the antibody.

The following examples are provided to further illustrate particular embodiments of the invention, and are not to be construed as limiting the scope of the present invention.

### EXAMPLE 1: Isolation of SVPH Nucleic Acids

A search of the GenBank DNA sequence database revealed two ESTs that share homology with ADAM20 and ADAM21. X85598 showed similarity to the Cys-rich region of ADAM20, while AI214466 showed similarity to the same region in ADAM21. Both ESTs were derived from testis mRNA.

X85598 and AI214466 were used to design primers, which were used to screen a human testis library. SVPH-1 clones were isolated by screening a human testis library (Clonetech cat no. HL3024a), (Cerretti et al., Proc. Natl. Acad. Sci. USA, 83:3223-3227 (1986)), at 42°C and washing at 42°C in 2x SSC/0.1% SDS using ³²P-labeled deoxyoligonucleotides (5'-CACCTAAGGTGTTCAATTCTTTG-3' (SEQ ID NO:17), 5'-CAAATACTGCAAGTGAGACTTGC-3' (SEQ ID NO:18), S'-TGCACAACTACGTGTGGTGTACCC-3' (SEQ ID N0:19), and 5'-GAGCCACTGCAATTGAA.AAAGTGCCC-3' (SEQ ID NO:20). SVPH-4 clones were isolated under the same conditions using ³²P-labeled deoxyoligonucleotides (AATGATGCTCTTGCATGGTCG (SEQ ID N0:21), CTTTCACGGAGCCCATGTAGTTGCAG (SEQ ID NO:22), and TGAAGGAGAAAACGCGCAGATGTCGG (SEQ ID NO:23). DNAs from positively hybridizing phages were purified and characterized by restriction endonuclease mapping, Southern blot analysis, and DNA sequencing.

### EXAMPLE 2: DNA Sequence Analysis of SVPH

SVPH-1c has an open reading frame of 820 amino acids (GenBank accession number AF171929) that encodes all of the ADAMs domains, inluding a signal sequence, pro-domain with a Cys switch, catalytic domain with a zinc-binding motif and a Met-turn, disintegrin domain, cysteine-rich domain, a transmembrane domain, and a cytoplasmic domain. However, SVPH-1c (as well as SVPH-1a and SVPH-1b) has a His residue (His 333) instead of a Glu residue in the zinc-binding motif that may affect catalytic activity. The Glu residue binds a water molecule via hydrogen binding and is required for enzymatic activity (Stocker, W. et al., Protein Sci., 4:823-840 (1995)). SVPH-1a and SVPH-1b represent alternative forms of SVPH-1c with differences in the cytoplasmic domain. SVPH-1a has a deletion of 54 amino acids resulting in a protein of 766 amino acids (GenBank accession number AF 171930), while SVPH-1b has a divergent 38 amino acid C-terminus resulting in a protein with 787 amino acids (GenBank accession number AF171931). These three forms of SVPH-1 encode cytoplasmic domains of 121, 67, and 88 amino acids, respectively. An unusual feature of the cytoplasmic domain of SVPH-1c is the sequence SQSQPPLMP (SEQ ID NO:32), which is repeated nine times. A search of GenBank did not find this repeat sequence in the database.

SVPH-4a has an open reading frame of 790 amino acids (GenBank accession number AF171932) with all of the domains found in ADAMs. Unlike the SVPH-1 clones, SVPH-4a and SVPH-4b each has a consensus zinc-binding motif in the catalytic domain. One cDNA, presumably from an alternative RNA splicing event, deletes nine amino acids in the cytoplasmic domain and has been designated SVPH-4b (GenBank accession number AF171933). Interestingly, SVPH-4a and SVPH-4b contain a repeat sequence, QEESK(T/A)KTG (SEQ ID NO:33), in the cytoplasmic domain, which was not found in GenBank.

As noted above, SVPH-1a, SVPH-1b, and SVPH-1c diverge from the consensus zinc-binding cluster (**HE**XX**H**XX**G**XX**HD**) (SEQ ID NO:31) in the catalytic domain with a Glu to His change at position 333. To analyze these proteins further, DNA and protein sequence multiple alignments of all known mammalian ADAMs (http://www.med.virginia.edu/~jag6n/adams.html) were produced using the PILEUP program from the Wisconsin Package (Wisconsin Package 10.1, Genetics Computer Group, Madison, WI). Protein multiple alignments were generated using the modified PAM scoring matrix of Gribskov and Burgess (Gribskov, M. et al., Nucleic Acids Res., 14:6745-6763 (1986)) provided in the Wisconsin Package, with gap-open and gap-extend penalties of 30 and 1, respectively. Nucleic acid multiple alignments were generated using a scoring matrix with A, C, G, T matches scoring unity, mismatches scoring zero, and gap-open and gap-extend penalties of 5 and I respectively. Unrooted maximum parsimony trees were estimated by the Wisconsin Package implementation of PAUP (version 4.0), starting from multiple alignments produced by PILEUP. PAUP parameters were set to use accelerated transformation character-state optimization with unordered, equally weighted characters.

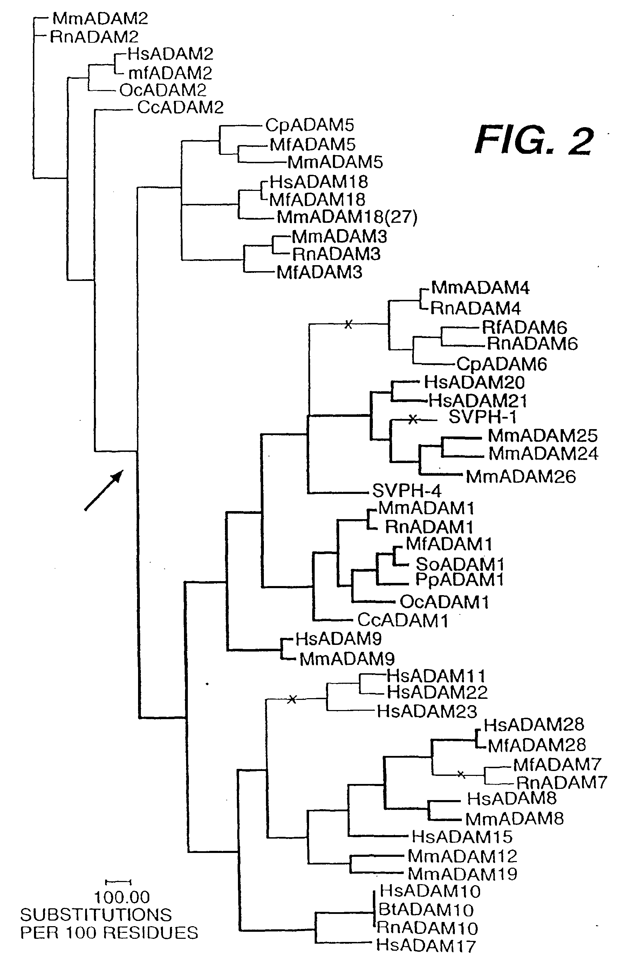

This alignment was used to infer a maximum parsimony phylogeny (Fig. 2). Due to the large number of taxa involved, the phylogeny was inferred using a heuristic tree search, which does not perform an exhaustive search of all possible tree topologies. Examination of the phylogenetic tree revealed an interesting pattern with respect to the presence of a zinc-binding motif. The ADAM sequences can be divided into two well-separated regions of the phylogeny, as marked by the arrow in Fig. 2. ADAM family members that contain a consensus zinc-binding site (**HE**XX**H**XX**G**XX**HD**) (SEQ ID NO:31) are clustered in one large group (heavy lines). Closely related members that do not have a consensus zinc binding motif (ADAMs 4, 6, 7, 11, 22, 23, and SVPH-1) presumably arose from a catalytically active ancestor, as many of them encode remnants of the zinc-binding motif. For example, ADAM4, ADAM7, and SVPH-1 all possess the three His residues and the Asp after the third conserved His. Finally, the corresponding region in ADAMs 2, 3, 5, 18 and 27 is quite distinct. As these sequences form clusters quite divergent from the zinc-binding site-containing ADAMs, it is most likely that the zinc-binding site arose once in the common ancestor to the ADAMs and was lost in those lineages which do not possess a zinc-binding site (denoted by an 'X' in Fig. 2).

### EXAMPLE 3: Chromosome Mapping of SVPH

Radiation hybrid mapping (Walter, et al., Nat. Genet., 7: 22-28 (1994)) was done using the GeneBridge 4 radiation-hybrid mapping panel (Research Genetics, Huntsville, AL). The panel was screened with specific primer pairs for SVPH-1 (sense: 5'-TCGATAATGCATGAAGGCAACCCACC-3' (SEQ ID NO:24) and antisense: 5'-CAAGTCTCACTTGCAGTATTTGCGCC-3' (SEQ ID N0:25), and SVPH-4 (sense; 5'-GCCACTGCATGTATGGGTG-3' (SEQ ID NO:26) and antisense: 5'-GACACTCTTTGCTTTGGGTCG-3' (SEQ ID NO:27) which generated products of 298 and 263 bp, respectively. PCR products were subjected to Southern blot analysis using an internal oligonucleotide probe specific for each gene. Data from two independent PCR screenings for each primer pair were scored against STS markers from the Whitehead Institute/MIT Center for Genome Research database using the statistical program RHMAPPER. LOD scores were >3.0 in all cases.

SVPH-1a, SVPH-1b, and SVPH-1c were mapped to chromosome 4q34, 1.51 cR distal from AFM312WG1. The sequential order of known markers relative to SVPH-1 on the Whitehead framework map was D4S1545, PDGH (Hydroxyprostaglandin Dehyrogenase 15)/SVPH-1/WI-21773/GPM6A (Glycoprotein M6A). This region is syntenic to mouse chromosome 8. SVPH-4a and SVPH-4b were mapped to chromosome 1p11-13, 1.65 cR distal to D1S453. The sequential order of markers relative to SVPH-4 on the Whitehead framework map was CD2 (thymocyte surface antigen)/SVPH-4/3bHSD2 (3 b Hydroxy-5-ene Steroid Dehydrogenase Type 11). This region is syntenic to mouse chromosome 3.

### EXAMPLE 4: Tissue Distribution of SVPH

Northern blot analysis was used to determine the tissue distribution of SVPH- 1 and SVPH-4. Northern blots were purchased from Clontech (catalog number 7760-1, 7759-1, 7755-1, 7750-1). Each lane contained approximately 2 µg of the indicated poly A⁺ RNA. The blots were treated with Stark's buffer (50% formamide, 50mM KPO₄, 5 x SSC, 1% SDS, 5X Denhardt's, 0.05% sarcosyl, 300mg/ml salmon sperm DNA) at 63°C for at least 1h and then probed with ³²P-labeled riboprobes in Stark's buffer at 63°C, overnight (Cosman et al., Nature, 312:768-771 (1984)). Blots were then sequentially washed to high stringency (0.1 x SSC, 0.1% SDS, 63°C) and exposed to film. Films were developed in an automated x-ray film processor. SVPH-1 (nt 1068 to 1786 of SEQ ID NOs:7-9) and SVPH-4 (nt 1343 to 1779 of SEQ ID NOs:10-11) anti-sense riboprobes were prepared by *in vitro,* transcription from a T7 RNA promoter with a commercially available kit (MAXIscript, Ambion, Inc., Austin, TX) using [α-³²P]-UTP as the labeled nucleotide.

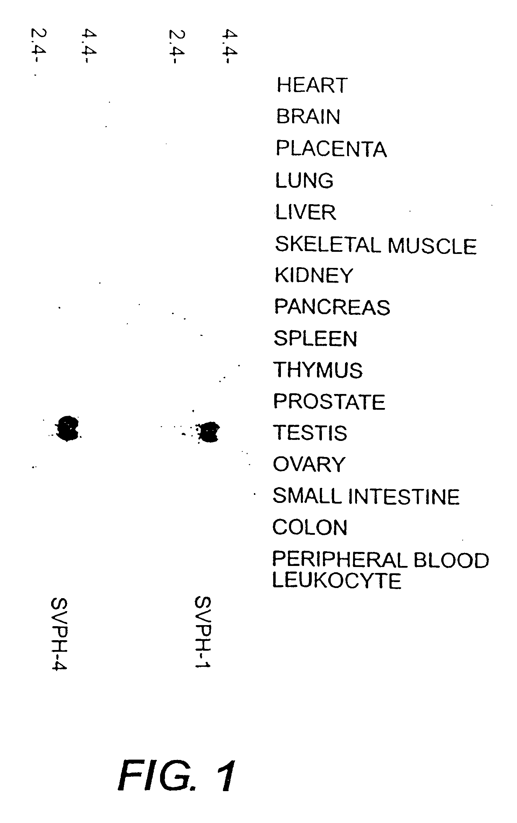

As indicated in Figure 1, both SVPH-1 and SVPH-4 were specifically expressed in testes with a single mRNA species of approximately 3.0 kb. No signals were detected in the other RNA samples.

### EXAMPLE 5: Monoclonal Antibodies

This example illustrates a method for preparing monoclonal antibodies that bind an SVPH-1, SVPH-1a, SVPH-1b, SVPH-1c, SVPH-4, SVPH-4a or SVPH-4b polypeptide. Suitable immunogens that may be employed in generating such antibodies include, but are not limited to, purified SVPH-1, SVPH-1a, SVPH-1b, SVPH-1c, SVPH-4, SVPH-4a or SVPH-4b polypeptide or an immunogenic fragment thereof such as the extracellular domain, or fusion proteins containing SVPH-1, SVPH-1a, SVPH-1b, SVPH-1c, SVPH-4, SVPH-4a or SVPH-4b (e.g., a soluble SVPH-1/Fc fusion protein).

Purified SVPH-1, SVPH-1a, SVPH-1b, SVPH-1c, SVPH-4, SVPH-4a or SVPH-4b can be used to generate monoclonal antibodies immunoreactive therewith, using conventional techniques such as those described in U.S. Patent 4,411,993. Briefly, mice are immunized with SVPH-1, SVPH-1a, SVPH-1b, SVPH-1c, SVPH-4, SVPH-4a or SVPH-4b immunogen emulsified in complete Freund's adjuvant, and injected in amounts ranging from 10-100 µg subcutaneously or intraperitoneally. Ten to twelve days later, the immunized animals are boosted with additional immunogen emulsified in incomplete Freund's adjuvant. Mice are periodically boosted thereafter on a weekly to bi-weekly immunization schedule. Serum samples are periodically taken by retro-orbital bleeding or tail-tip excision to test for SVPH-1, SVPH-1a, SVPH-1b, SVPH-1c, SVPH-4, SVPH-4a or SVPH-4b antibodies by dot blot assay, ELISA (Enzyme-Linked Immunosorbent Assay) or inhibition of binding partner binding.

Following detection of an appropriate antibody titer, positive animals are provided one last intravenous injection of SVPH-1, SVPH-1a, SVPH-1b, SVPH-1c, SVPH-4, SVPH-4a or SVPH-4b in saline. Three to four days later, the animals are sacrificed, spleen cells harvested, and spleen cells are fused to a murine myeloma cell line, e.g., NS1 or preferably P3x63Ag8.653 (ATCC CRL 1580). Fusions generate hybridoma cells, which are plated in multiple microtiter plates in a HAT (hypoxanthine, aminopterin and thymidine) selective medium to inhibit proliferation of non-fused cells, myeloma hybrids, and spleen cell hybrids.

The hybridoma cells are screened by ELISA for reactivity against purified SVPH-1, SVPH-1a, SVPH-1b, SVPH-1c, SVPH-4, SVPH-4a or SVPH-4b by adaptations of the techniques disclosed in Engvall et al., Immunochem. 8:871, 1971 and in U.S. Patent 4,703,004. A preferred screening technique is the antibody capture technique described in Beckmann et al., J. Immunol. 144:4212, (1990). Positive hybridoma cells can be injected intraperitoneally into syngeneic BALB/c mice to produce ascites containing high concentrations of anti-SVPH-1, SVPH-1a, SVPH-1b, SVPH-1c, SVPH-4, SVPH-4a or SVPH-4b monoclonal antibodies. Alternatively, hybridoma cells can be grown *in vitro* in flasks or roller bottles by various techniques. Monoclonal antibodies produced in mouse ascites can be purified by ammonium sulfate precipitation, followed by gel exclusion chromatography. Alternatively, affinity chromatography based upon binding of antibody to Protein A or Protein G can also be used, as can affinity chromatography based upon binding to SVPH-1, SVPH-1a, SVPH-16, SVPH-1c, SVPH-4, SVPH-4a or SVPH-4b.

### EXAMPLE 6: Binding Assay

Full length SVPH-1, SVPH-1a, SVPH-1b, SVPH-1c, SVPH-4, SVPH-4a or SVPH-4b is expressed and tested for the ability to bind its binding partner. The binding assay is conducted as follows.

A fusion protein comprising a leucine zipper peptide fused to the N-terminus of a soluble binding partner polypeptide (LZ-binding partner) is employed in the assay. An expression construct is prepared, essentially as described for preparation of the FLAG®-binding partner expression construct in Wiley et al., Immunity, 3:673-682, (1995), which is hereby incorporated by reference, except that DNA encoding the FLAG® peptide is replaced with a sequence encoding a modified leucine zipper that allows for trimerization. The construct, in expression vector pDC409, encodes a leader sequence derived from human cytomegalovirus, followed by the leucine zipper moiety fused to the N-terminus of a soluble binding partner polypeptide. The LZ-binding partner is expressed in CHO cells, and can be purified from the culture supernatant.

The expression vector designated pDC409 is a mammalian expression vector derived from the pDC406 vector described in McMahan et al., EMBO J 10:2821-2832, (1991), which is hereby incorporated by reference. Features added to pDC409 (compared to pDC406) include additional unique restriction sites in the multiple cloning site (mcs); three stop codons (one in each reading frame) positioned downstream of the mcs; and a T7 polymerase promoter, downstream of the mcs, that facilitates sequencing of DNA inserted into the mcs.

For expression of full length human SVPH-1, SVPH-1a, SVPH-1b, SVPH-1c, SVPH-4, SVPH-4a or SVPH-4b protein, the entire coding region (i.e., the DNA sequence presented in SEQ ID NO:1, SEQ ID NO:3, SEQ ID NO:7, SEQ ID NO:8, SEQ ID NO:9, SEQ ID NO:10, or SEQ ID NO: 11) is amplified by polymerase chain reaction (PCR). The isolated and amplified DNA is inserted into the expression vector pDC409.

LZ-binding partner polypeptide is employed to test its ability to bind host cells expressing recombinant SVPH-1, SVPH-1a, SVPH-1b, SVPH-1c, SVPH-4, SVPH-4a or SVPH-4b polypeptide, as discussed above. Cells are cultured in DMEM supplemented with 10% fetal bovine serum, penicillin, streptomycin, and glutamine. 48 hours after transfection, cells are detached non-enzymatically and incubated with LZ-binding partner (5 mg/ml), a biotinylated anti-LZ monoclonal antibody (5 mg/ml), and phycoerythrin-conjugated streptavidin (1:400), before analysis by fluorescence-activated cell scanning (FACS). The cytometric analysis is conducted on a FACscan (Beckton Dickinson, San Jose, CA).

The cells expressing LZ-binding partner will show significantly enhanced binding of SVPH-1, SVPH-1a, SVPH-1b, SVPH-1c, SVPH-4, SVPH-4a or SVPH-4b, compared to the control cells not expressing LZ-binding partner.

The specification is most thoroughly understood in light of the teachings of the references cited within the specification which are hereby incorporated by reference. The embodiments within the specification provide an illustration of embodiments of the invention and should not be construed to limit the scope of the invention. The skilled artisan readily recognizes that many other embodiments are encompassed by the invention.

## Claims

1. An isolated SVPH nucleic acid molecule selected from the group consisting of:
(a) the DNA sequence selected from the group consisting of SEQ ID NO:1, SEQ ID NO:7, SEQ ID NO:8, and SEQ ID NO:9;
(b) an isolated nucleic acid molecule encoding an amino acid sequence comprising the sequence selected from the group consisting of SEQ ID NO:4, SEQ ID NO:12, SEQ ID NO:13, and SEQ ID NO:14;
(c) an isolated nucleic acid molecule that hybridizes to either strand of a denatured, double-stranded DNA comprising the nucleic acid sequence of (a) or (b) under conditions of moderate stringency in 50% formamide and 6XSSC, at 42 °C with washing conditions of 60°C, 0.5XSSC, 0.1% SDS;
(d) an isolated nucleic acid molecule derived by *in vitro* mutagenesis from SEQ ID NO:1, SEQ ID NO:7, SEQ ID NO:8, and SEQ ID NO:9;
(e) an isolated nucleic acid molecule degenerate from SEQ ID NO:1, SEQ ID NO:7, SEQ ID NO:8, and SEQ ID NO:9 as a result of the genetic code; and
(f) an isolated nucleic acid molecule selected from the group consisting of human SVPH 1 DNA; an allelic variant of human SVPH 1 DNA; and a species homolog of SVPH I DNA.

2. The nucleic acid molecule of claim 1 selected from the group consisting of SEQ ID NO:1, SEQ ID NO: 7, SEQ ID NO: 8, and SEQ ID NO:9.

3. A recombinant vector that directs the expression of the nucleic acid molecule of claim 1.

4. An isolated polypeptide encoded by the nucleic acid molecule of claim 1.

5. An isolated polypeptide according to claim 4 having a molecular weight selected from the group consisting of approximately 4,199; 86,983; 89,459; and 92,781 Daltons as determined by SDS-PAGE.

6. An isolated polypeptide according to claim 4 in non-glycosylated form.

7. Isolated antibodies that bind to a polypeptide of claim 4.

8. Isolated antibodies according to claim 7, wherein the antibodies are monoclonal antibodies.

9. A host cell transfected or transduced with the vector of claim 3.

10. A method for the production of SVPH 1 polypeptide comprising culturing a host cell of claim 9 under conditions promoting expression, and recovering the polypeptide from the culture medium.

11. The method of claim 10, wherein the host cell is selected from the group consisting of bacterial cells, yeast cells, plant cells, and animal cells.

12. The method of claim 10, wherein the host cell is a mammalian cell.

13. An isolated polypeptide comprising an amino acid sequence selected from the group consisting of SEQ ID NO:4, SEQ ID NO:12, SEQ ID NO:13, and SEQ ID NO:14.

14. An oligomer comprising a polypeptide of claim 4.

15. An isolated SVPH nucleic acid molecule selected from the group consisting of:
(a) the DNA sequence selected from the group consisting of SEQ ID NO:3, SEQ ID NO:10, and SEQ ID NO:11;
(b) an isolated nucleic acid molecule encoding an amino acid sequence comprising the sequence selected from the group consisting of SEQ ID NO:6, SEQ ID NO:15, and SEQ ID NO: 16;
(c) an isolated nucleic acid molecule that hybridizes to either strand of a denatured, double-stranded DNA comprising the nucleic acid sequence of (a) or (b) under conditions of moderate stringency in 50% formamide and 6XSSC, at 42°C with washing conditions of 60°C, 0.5XSSC, 0.1% SDS;
(d) an isolated nucleic acid molecule derived by *in vitro* mutagenesis from SEQ ID NO:3, SEQ ID NO:10, and SEQ ID NO: 11;
(e) an isolated nucleic acid molecule degenerate from SEQ ID NO:3, SEQ ID NO:10, and SEQ ID NO:11 as a result of the genetic code; and
(f) an isolated nucleic acid molecule selected from the group consisting of human SVPH 4 DNA; an allelic variant of human SVPH 4 DNA; and a species homolog of SVPH 4 DNA.

16. The nucleic acid molecule of claim 1 selected from the group consisting of SEQ ID NO:3, SEQ ID NO:10, and SEQ ID NO:11.

17. A recombinant vector that directs the expression of the nucleic acid molecule of claim 15.

18. An isolated polypeptide encoded by the nucleic acid molecule of claim 15.

19. An isolated polypeptide according to claim 18 having a molecular weight selected from the group consisting of approximately 55,209; 88,923; and 87,990 Daltons as determined by SDS-PAGE.

20. An isolated polypeptide according to claim 18 in non-glycosylated form.

21. Isolated antibodies that bind to a polypeptide of claim 18.

22. Isolated antibodies according to claim 21, wherein the antibodies are monoclonal antibodies.

23. A host cell transfected or transduced with the vector of claim 17.

24. A method for the production of SVPH 4 polypeptide comprising culturing a host cell of claim 23 under conditions promoting expression, and recovering the polypeptide from the culture medium.

25. The method of claim 24, wherein the host cell is selected from the group consisting of bacterial cells, yeast cells, plant cells, and animal cells.

26. The method of claim 24, wherein the host cell is a mammalian cell.

27. An isolated polypeptide comprising an amino acid sequence selected from the group consisting of SEQ ID NO:6, SEQ ID NO:15, and SEQ ID NO:16.

28. An oligomer comprising a polypeptide of claim 18.

29. An isolated SVPH nucleic acid molecule selected from the group consisting of:
(a) the DNA sequence of SEQ ID NO:2;
(b) an isolated nucleic acid molecule encoding an amino acid sequence comprising the sequence of SEQ ID NO:5;
(c) an isolated nucleic acid molecule that hybridizes to either strand of a denatured, double-stranded DNA comprising the nucleic acid sequence of (a) or (b) under conditions of moderate stringency in 50% formamide and 6XSSC, at 42°C with washing conditions of 60°C, 0.5XSSC, 0.1% SDS;
(d) an isolated nucleic acid molecule derived by *in vitro* mutagenesis from SEQ ID NO:2;
(e) an isolated nucleic acid molecule degenerate from SEQ ID NO:2 as a result of the genetic code; and
(f) an isolated nucleic acid molecule selected from the group consisting of human SVPH 3 DNA; an allelic variant of human SVPH 3 DNA; and a species homolog of SVPH 3 DNA.

30. The nucleic acid molecule of claim 29, wherein the DNA sequence comprises SEQ ID NO:2.

31. A recombinant vector that directs the expression of the nucleic acid molecule of claim 29.

32. An isolated polypeptide encoded by the nucleic acid molecule of claim 29.

33. An isolated polypeptide according to claim 32 having a molecular weight of approximately 13,938 Daltons as determined by SDS-PAGE.

34. An isolated polypeptide according to claim 32 in non-glycosylated form.

35. Isolated antibodies that bind to a polypeptide of claim 32.

36. Isolated antibodies according to claim 35, wherein the antibodies are monoclonal antibodies.

37. A host cell transfected or transduced with the vector of claim 31.

38. A method for the production of SVPH 3 polypeptide comprising culturing a host cell of claim 37 under conditions promoting expression, and recovering the polypeptide from the culture medium.

39. The method of claim 38, wherein the host cell is selected from the group consisting of bacterial cells, yeast cells, plant cells, and animal cells.

40. The method of claim 38, wherein the host cell is a mammalian cell.

41. An isolated polypeptide comprising an amino acid sequence of SEQ ID NO:5.

42. An oligomer comprising a polypeptide of claim 32.
